(19) Europäisches Patentamt
European Patent Office
Office européen des brevets

(11) EP 3 903 828 A1

(12) EUROPEAN PATENT APPLICATION
published in accordance with Art. 153(4) EPC

(43) Date of publication:
**03.11.2021 Bulletin 2021/44**

(21) Application number: **19899188.7**

(22) Date of filing: **20.12.2019**

(51) Int Cl.:
***A61K 47/68*** (2017.01) ***A61K 31/422*** (2006.01)
***A61K 31/436*** (2006.01) ***A61K 31/44*** (2006.01)
***A61K 31/4439*** (2006.01) ***A61K 31/47*** (2006.01)
***A61K 31/4709*** (2006.01) ***A61K 31/4745*** (2006.01)
***A61K 31/506*** (2006.01) ***A61K 31/517*** (2006.01)
***A61K 31/519*** (2006.01) ***A61K 31/553*** (2006.01)
***A61K 39/395*** (2006.01) ***A61K 45/00*** (2006.01)
***A61P 35/00*** (2006.01) ***A61P 35/02*** (2006.01)
***A61P 43/00*** (2006.01)

(86) International application number:
**PCT/JP2019/050017**

(87) International publication number:
**WO 2020/130125 (25.06.2020 Gazette 2020/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.12.2018 JP 2018240049**
**26.04.2019 JP 2019085338**

(71) Applicant: **Daiichi Sankyo Company, Limited**
**Tokyo 103-8426 (JP)**

(72) Inventors:
- **ISHII Chiaki**
  **Tokyo 103-8426 (JP)**
- **KAMAI Yasuki**
  **Tokyo 103-8426 (JP)**
- **SUGIHARA Kiyoshi**
  **Tokyo 103-8426 (JP)**
- **OKAJIMA Daisuke**
  **Tokyo 103-8426 (JP)**
- **HASHIMOTO Yuuri**
  **Tokyo 103-8426 (JP)**
- **SUZUKI Hirokazu**
  **Tokyo 103-8426 (JP)**

(74) Representative: **Wallace, Sheila Jane**
**Marks & Clerk LLP**
**15 Fetter Lane**
**London EC4A 1BW (GB)**

# (54) COMBINATION OF ANTIBODY-DRUG CONJUGATE AND KINASE INHIBITOR

(57) A pharmaceutical composition wherein an antibody-drug conjugate in which a drug-linker represented by the following formula (wherein A represents a connecting position to an antibody) is conjugated to the antibody via a thioether bond and a kinase inhibitor (at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor) are administered in combination, and/or a method of treatment wherein the antibody-drug conjugate and a kinase inhibitor are administered in combination to a subject.

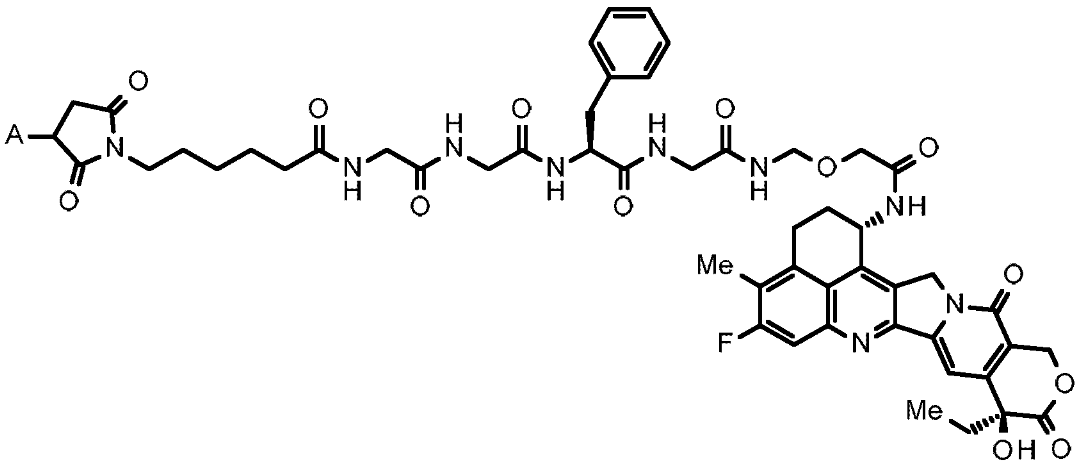

[Figure 11]

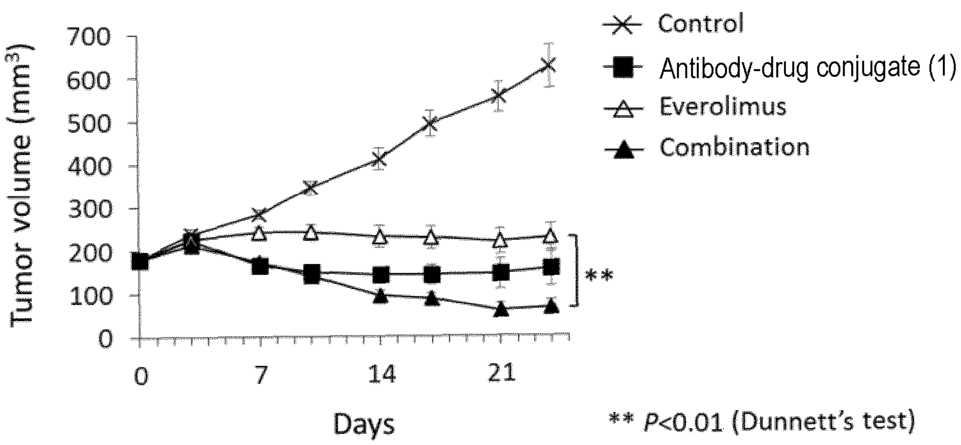
Tumor volume (mm³)
700
600
500
400
300
200
100
0
0
7
14
21
Days
Control
Antibody-drug conjugate (1)
Everolimus
Combination
**
** P<0.01 (Dunnett's test)

SEQUENCE LISTING

**[0250]**

<110> DAIICHI SANKYO COMPANY, LIMITED

<120> COMBINATION OF ANTIBODY-DRUG CONJUGATE AND KINASE INHIBITOR

<130> DSFP1932WO

<150> JP2018-240049
<151> 2018-12-21

<150> JP2019-085338
<151> 2019-04-26

<160> 10

<170> PatentIn version 3.5

<210> 1
<211> 450
<212> PRT
<213> Artificial Sequence

<220>

<223> Heavy chain of anti-HER2 antibody

<400> 1

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140
```

```
Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400
```

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 2
<211> 214
<212> PRT
<213> Artificial Sequence

<220>

<223> Light chain of anti-HER2 antibody

<400> 2

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 3
<211> 447
<212> PRT
<213> Artificial Sequence

<220>

<223> Heavy chain of anti-HER3 antibody

<400> 3

Gln Val Gln Leu Gln Gln Trp Gly Ala Gly Leu Leu Lys Pro Ser Glu
1 5 10 15

Thr Leu Ser Leu Thr Cys Ala Val Tyr Gly Gly Ser Phe Ser Gly Tyr
20 25 30

Tyr Trp Ser Trp Ile Arg Gln Pro Pro Gly Lys Gly Leu Glu Trp Ile
35 40 45

Gly Glu Ile Asn His Ser Gly Ser Thr Asn Tyr Asn Pro Ser Leu Lys
50 55 60

Ser Arg Val Thr Ile Ser Val Glu Thr Ser Lys Asn Gln Phe Ser Leu
65 70 75 80

Lys Leu Ser Ser Val Thr Ala Ala Asp Thr Ala Val Tyr Tyr Cys Ala
85 90 95

Arg Asp Lys Trp Thr Trp Tyr Phe Asp Leu Trp Gly Arg Gly Thr Leu
100 105 110

Val Thr Val Ser Ser Ala Ser Thr Lys Gly Pro Ser Val Phe Pro Leu
115 120 125

```
Ala Pro Ser Ser Lys Ser Thr Ser Gly Gly Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Arg Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380
```

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210> 4
<211> 220
<212> PRT
<213> Artificial Sequence

<220>

<223> Light chain of anti-HER3 antibody

<400> 4

Asp Ile Glu Met Thr Gln Ser Pro Asp Ser Leu Ala Val Ser Leu Gly
1               5                   10                  15

Glu Arg Ala Thr Ile Asn Cys Arg Ser Ser Gln Ser Val Leu Tyr Ser
            20                  25                  30

Ser Ser Asn Arg Asn Tyr Leu Ala Trp Tyr Gln Gln Asn Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Thr Pro Arg Thr Phe Gly Gln Gly Thr Lys Val Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145 150 155 160

Gln Ser Gly Asn Ser Gln Glu Ser Val Thr Glu Gln Asp Ser Lys Asp
165 170 175

Ser Thr Tyr Ser Leu Ser Ser Thr Leu Thr Leu Ser Lys Ala Asp Tyr
180 185 190

Glu Lys His Lys Val Tyr Ala Cys Glu Val Thr His Gln Gly Leu Ser
195 200 205

Ser Pro Val Thr Lys Ser Phe Asn Arg Gly Glu Cys
210 215 220

<210> 5
<211> 470
<212> PRT
<213> Artificial Sequence

<220>

<223> Heavy chain of anti-TROP2 antibody

<400> 5

```
Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Thr Ala Gly Met Gln Trp Val Arg Gln Ala Pro Gly Gln Gly Leu
    50                  55                  60

Glu Trp Met Gly Trp Ile Asn Thr His Ser Gly Val Pro Lys Tyr Ala
65                  70                  75                  80

Glu Asp Phe Lys Gly Arg Val Thr Ile Ser Ala Asp Thr Ser Thr Ser
                85                  90                  95

Thr Ala Tyr Leu Gln Leu Ser Ser Leu Lys Ser Glu Asp Thr Ala Val
            100                 105                 110

Tyr Tyr Cys Ala Arg Ser Gly Phe Gly Ser Ser Tyr Trp Tyr Phe Asp
        115                 120                 125

Val Trp Gly Gln Gly Thr Leu Val Thr Val Ser Ser Ala Ser Thr Lys
```

130 135 140

Gly Pro Ser Val Phe Pro Leu Ala Pro Ser Ser Lys Ser Thr Ser Gly
145 150 155 160

Gly Thr Ala Ala Leu Gly Cys Leu Val Lys Asp Tyr Phe Pro Glu Pro
165 170 175

Val Thr Val Ser Trp Asn Ser Gly Ala Leu Thr Ser Gly Val His Thr
180 185 190

Phe Pro Ala Val Leu Gln Ser Ser Gly Leu Tyr Ser Leu Ser Ser Val
195 200 205

Val Thr Val Pro Ser Ser Ser Leu Gly Thr Gln Thr Tyr Ile Cys Asn
210 215 220

Val Asn His Lys Pro Ser Asn Thr Lys Val Asp Lys Arg Val Glu Pro
225 230 235 240

Lys Ser Cys Asp Lys Thr His Thr Cys Pro Pro Cys Pro Ala Pro Glu
245 250 255

Leu Leu Gly Gly Pro Ser Val Phe Leu Phe Pro Pro Lys Pro Lys Asp
260 265 270

Thr Leu Met Ile Ser Arg Thr Pro Glu Val Thr Cys Val Val Val Asp
275 280 285

Val Ser His Glu Asp Pro Glu Val Lys Phe Asn Trp Tyr Val Asp Gly
290 295 300

Val Glu Val His Asn Ala Lys Thr Lys Pro Arg Glu Glu Gln Tyr Asn
305 310 315 320

Ser Thr Tyr Arg Val Val Ser Val Leu Thr Val Leu His Gln Asp Trp
325 330 335

Leu Asn Gly Lys Glu Tyr Lys Cys Lys Val Ser Asn Lys Ala Leu Pro
340 345 350

Ala Pro Ile Glu Lys Thr Ile Ser Lys Ala Lys Gly Gln Pro Arg Glu
355 360 365

Pro Gln Val Tyr Thr Leu Pro Pro Ser Arg Glu Glu Met Thr Lys Asn
370 375 380

Gln Val Ser Leu Thr Cys Leu Val Lys Gly Phe Tyr Pro Ser Asp Ile
385 390 395 400

Ala Val Glu Trp Glu Ser Asn Gly Gln Pro Glu Asn Asn Tyr Lys Thr
405 410 415

Thr Pro Pro Val Leu Asp Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
450 455 460

Ser Leu Ser Pro Gly Lys
465 470

<210> 6
<211> 234
<212> PRT
<213> Artificial Sequence

<220>

<223> Light chain of anti-TROP2 antibody

<400> 6

```
Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
            20                  25                  30

Ala Ser Val Gly Asp Arg Val Thr Ile Thr Cys Lys Ala Ser Gln Asp
        35                  40                  45

Val Ser Thr Ala Val Ala Trp Tyr Gln Gln Lys Pro Gly Lys Ala Pro
    50                  55                  60

Lys Leu Leu Ile Tyr Ser Ala Ser Tyr Arg Tyr Thr Gly Val Pro Ser
65                  70                  75                  80

Arg Phe Ser Gly Ser Gly Ser Gly Thr Asp Phe Thr Leu Thr Ile Ser
                85                  90                  95

Ser Leu Gln Pro Glu Asp Phe Ala Val Tyr Tyr Cys Gln Gln His Tyr
            100                 105                 110

Ile Thr Pro Leu Thr Phe Gly Gln Gly Thr Lys Leu Glu Ile Lys Arg


        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```

<210> 7
<211> 471
<212> PRT
<213> Artificial Sequence

<220>

<223> Heavy chain of anti-B7-H3 antibody

<400> 7

```
Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ser Ser Val Lys Val Ser Cys Lys Ala Ser Gly Tyr Thr Phe
        35                  40                  45

Thr Asn Tyr Val Met His Trp Val Arg Gln Ala Pro Gly Gln Gly Leu
    50                  55                  60

Glu Trp Met Gly Tyr Ile Asn Pro Tyr Asn Asp Asp Val Lys Tyr Asn
65                  70                  75                  80

Glu Lys Phe Lys Gly Arg Val Thr Ile Thr Ala Asp Glu Ser Thr Ser
                85                  90                  95
```

Thr Ala Tyr Met Glu Leu Ser Ser Leu Arg Ser Glu Asp Thr Ala Val
            100                 105                 110

Tyr Tyr Cys Ala Arg Trp Gly Tyr Tyr Gly Ser Pro Leu Tyr Tyr Phe
        115                 120                 125

Asp Tyr Trp Gly Gln Gly Thr Leu Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu

340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 8
<211> 233
<212> PRT
<213> Artificial Sequence

<220>

<223> Light chain of anti-B7-H3 antibody

<400> 8

```
Met Val Leu Gln Thr Gln Val Phe Ile Ser Leu Leu Leu Trp Ile Ser
1               5                   10                  15

Gly Ala Tyr Gly Glu Ile Val Leu Thr Gln Ser Pro Ala Thr Leu Ser
            20                  25                  30

Leu Ser Pro Gly Glu Arg Ala Thr Leu Ser Cys Arg Ala Ser Ser Arg
        35                  40                  45

Leu Ile Tyr Met His Trp Tyr Gln Gln Lys Pro Gly Gln Ala Pro Arg
    50                  55                  60

Pro Leu Ile Tyr Ala Thr Ser Asn Leu Ala Ser Gly Ile Pro Ala Arg
65                  70                  75                  80

Phe Ser Gly Ser Gly Ser Gly Thr Asp Phe Thr Leu Thr Ile Ser Ser
                85                  90                  95

Leu Glu Pro Glu Asp Phe Ala Val Tyr Tyr Cys Gln Gln Trp Asn Ser
            100                 105                 110

Asn Pro Pro Thr Phe Gly Gln Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230
```

<210> 9
<211> 471
<212> PRT

<213> Artificial Sequence

<220>

<223> Heavy chain of anti-CDH6 antibody

<400> 9

```
Met Lys His Leu Trp Phe Phe Leu Leu Leu Val Ala Ala Pro Arg Trp
1               5                   10                  15

Val Leu Ser Glu Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45
```

Thr Arg Asn Phe Met His Trp Val Arg Gln Ala Pro Gly Gln Gly Leu
50 55 60

Glu Trp Met Gly Trp Ile Tyr Pro Gly Asp Gly Glu Thr Glu Tyr Ala
65 70 75 80

Gln Lys Phe Gln Gly Arg Val Thr Ile Thr Ala Asp Thr Ser Thr Ser
85 90 95

Thr Ala Tyr Met Glu Leu Ser Ser Leu Arg Ser Glu Asp Thr Ala Val
100 105 110

Tyr Tyr Cys Ala Arg Gly Val Tyr Gly Gly Phe Ala Gly Gly Tyr Phe
115 120 125

Asp Phe Trp Gly Gln Gly Thr Leu Val Thr Val Ser Ser Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Arg Val Glu
225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 10
<211> 233
<212> PRT
<213> Artificial Sequence

<220>

<223> Light chain of anti-CDH6 antibody

<400> 10

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

```
Ala Ser Val Gly Asp Arg Val Thr Ile Thr Cys Lys Ala Ser Gln Asn
        35                  40                  45

Ile Tyr Lys Asn Leu Ala Trp Tyr Gln Gln Lys Pro Gly Lys Ala Pro
    50                  55                  60

Lys Leu Leu Ile Tyr Asp Ala Asn Thr Leu Gln Thr Gly Val Pro Ser
65                  70                  75                  80

Arg Phe Ser Gly Ser Gly Ser Gly Ser Asp Phe Thr Leu Thr Ile Ser
                85                  90                  95

Ser Leu Gln Pro Glu Asp Phe Ala Thr Tyr Phe Cys Gln Gln Tyr Tyr
            100                 105                 110

Ser Gly Trp Ala Phe Gly Gln Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230
```

**Description**

Technical Field

**[0001]** The present invention relates to a pharmaceutical composition wherein a specific antibody-drug conjugate and a kinase inhibitor are administered in combination, and/or a method of treatment wherein a specific antibody-drug conjugate and a kinase inhibitor are administered in combination to a subject.

Background Art

**[0002]** Abnormal activation of intracellular signaling is associated with proliferation of cancer cells and tumor angiogenesis. Kinase inhibitors are agents that inhibit kinases involved in abnormally activated intracellular signaling to exert an antitumor effect. Examples of such kinase inhibitors include a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, an PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, and an NTRK inhibitor (Non-Patent References 1 to 11).

**[0003]** An antibody-drug conjugate (ADC) having a drug with cytotoxicity conjugated to an antibody, whose antigen is expressed on the surface of cancer cells and which also binds to an antigen capable of cellular internalization, and therefore can deliver the drug selectively to cancer cells, is thus expected to cause accumulation of the drug within cancer cells and to kill the cancer cells (Non-Patent References 12 to 16).

**[0004]** As one such antibody-drug conjugate, an antibody-drug conjugate comprising an antibody and a derivative of exatecan, which is a topoisomerase I inhibitor, as its components is known (Patent References 1 to 8, Non-Patent References 17 to 21).

**[0005]** Patent References 1 to 8 disclose that the antibody-drug conjugate can be administered together with various cancer treating agents.

**[0006]** However, none of the references describes any test result showing an excellent combined effect when the foregoing antibody-drug conjugate and the kinase inhibitor are used in combination, or any scientific basis for suggesting such a test result.

Citation List

Patent Literature

**[0007]**


Patent Reference 1: International Publication No. 2014/057687
Patent Reference 2: International Publication No. 2014/061277
Patent Reference 3: International Publication No. 2015/098099
Patent Reference 4: International Publication No. 2015/115091
Patent Reference 5: International Publication No. 2015/146132
Patent Reference 6: International Publication No. 2015/155976
Patent Reference 7: International Publication No. 2015/155998
Patent Reference 8: International Publication No. 2018/212136


Non-Patent Literature

**[0008]**


Non-Patent Reference 1: Otto T., et al., Nat. Rev. Cancer (2017) 17(2) : 93-115.
Non-Patent Reference 2: Zhang YJ., et al., Drug Discov. Today (2011) 16(7-8) : 325-331.
Non-Patent Reference 3: Zaytseva YY., et al., Cancer Lett. (2012) 319(1) : 1-7.
Non-Patent Reference 4: Janku F., et al., Nat. Rev. Clin. Oncol. (2018) 15(5): 273-291.
Non-Patent Reference 5: Bergholz JS., et al., J. Clin. Oncol. (2018) 36(13): 1339-1342
Non-Patent Reference 6: Zhao Y., et al., Nat. Rev. Clin. Oncol. (2014) 11(7): 385-400.
Non-Patent Reference 7: Caunt CJ., et al., Nat. Rev. Cancer (2015) 15(10): 577-592.
Non-Patent Reference 8: Ryan MB., et al., Nat. Rev. Clin. Oncol. (2018) 15(11): 709-720.

Non-Patent Reference 9: Ferquson FM., et al., Nat. Rev. Drug Discov. (2018) 17(5): 353-377.
Non-Patent Reference 10: Konig H., et al., Current Cancer Drug Targets (2015) 15, 803-821.
Non-Patent Reference 11: Kheder ES., et al., Clinical Cancer Research (2018) 24(23), 5807-5814.
Non-Patent Reference 12: Ducry, L., et al., Bioconjugate Chem. (2010) 21, 5-13.
Non-Patent Reference 13: Alley, S. C., et al., Current Opinion in Chemical Biology (2010) 14, 529-537.
Non-Patent Reference 14: Damle N. K. Expert Opin. Biol. Ther. (2004) 4, 1445-1452.
Non-Patent Reference 15: Senter P. D., et al., Nature Biotechnology (2012) 30, 631-637.
Non-Patent Reference 16: Burris HA., et al., J. Clin. Oncol. (2011) 29(4): 398-405.
Non-Patent Reference 17: Ogitani Y. et al., Clinical Cancer Research (2016) 22(20), 5097-5108.
Non-Patent Reference 18: Ogitani Y. et al., Cancer Science (2016) 107, 1039-1046.
Non-Patent Reference 19: Doi T, et al., Lancet Oncol. (2017) 18, 1512-22.
Non-Patent Reference 20: Takegawa N, et al., Int. J. Cancer (2017) 141, 1682-1689.
Non-Patent Reference 21: Yonesaka K, et al., Int. Oncogene (2018) 141, 1682-1689 (2017).

## Summary of Invention

### Technical Problem

**[0009]** An antibody-drug conjugate used in the present invention (an antibody-drug conjugate including a derivative of exatecan as a component) has been confirmed to exert a superior antitumor effect even as a single agent. However, there has been a need to obtain a method of treatment which can suppress growth of cancer cells in multiple manners and exert a further superior antitumor effect by using the antibody-drug conjugate in combination with another anticancer agent having a different mechanism of action.

**[0010]** An object of the present invention is to provide a pharmaceutical composition wherein a specific antibody-drug conjugate and a kinase inhibitor are administered in combination, and/or a method of treatment wherein a specific antibody-drug conjugate and a kinase inhibitor are administered in combination to a subject.

### Solution to Problem

**[0011]** As a result of diligent studies in order to solve the above problems, the present inventors have found that combined administration of a specific antibody-drug conjugate and a kinase inhibitor exhibits a superior combined effect, and completed the present invention.

**[0012]** Thus, the present invention provides the following [1] to [1072].

[1] A pharmaceutical composition wherein an antibody-drug conjugate and a kinase inhibitor are administered in combination, and the antibody-drug conjugate is an antibody-drug conjugate in which a drug-linker represented by the following formula:

[Formula 1]

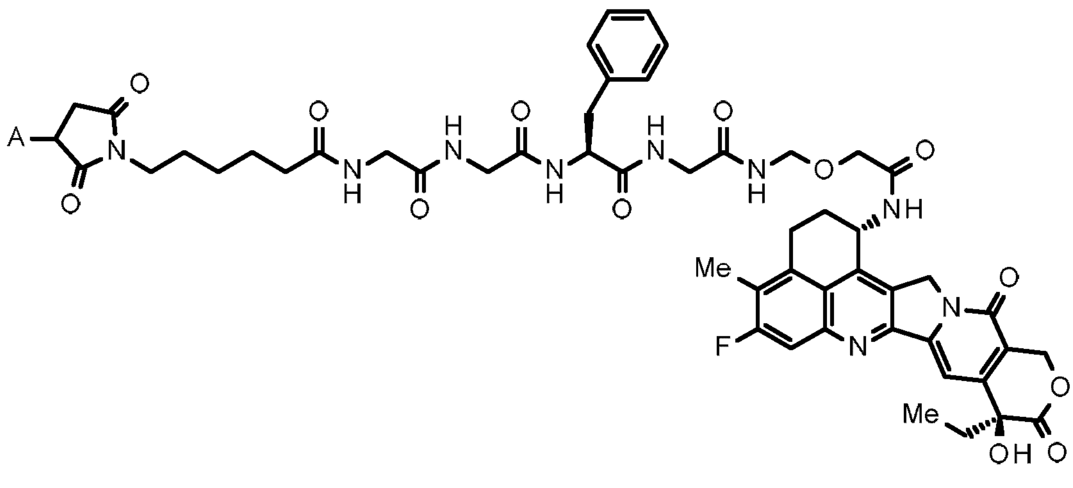

wherein A represents a connecting position to an antibody, is conjugated to the antibody via a thioether bond, and

the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[2] The pharmaceutical composition according to [1], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[3] The pharmaceutical composition according to [2], wherein the kinase inhibitor is a CDK4/6 inhibitor.

[4] The pharmaceutical composition according to [3], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[5] The pharmaceutical composition according to [2], wherein the kinase inhibitor is an mTOR inhibitor.

[6] The pharmaceutical composition according to [5], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[7] The pharmaceutical composition according to [2], wherein the kinase inhibitor is a PI3K inhibitor.

[8] The pharmaceutical composition according to [7], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[9] The pharmaceutical composition according to [2], wherein the kinase inhibitor is an RAF inhibitor.

[10] The pharmaceutical composition according to [9], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[11] The pharmaceutical composition according to [2], wherein the kinase inhibitor is a VEGFR inhibitor.

[12] The pharmaceutical composition according to [11], wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[13] The pharmaceutical composition according to [2], wherein the kinase inhibitor is a KIT inhibitor.

[14] The pharmaceutical composition according to [13], wherein the KIT inhibitor is regorafenib, sorafenib, imatinib, ilorasertib, sunitinib, pazopanib, lenvatinib, or dasatinib, or a pharmacologically acceptable salt thereof.

[15] The pharmaceutical composition according to [2], wherein the kinase inhibitor is an RET inhibitor.

[16] The pharmaceutical composition according to [15], wherein the RET inhibitor is regorafenib, sorafenib, vandetanib, lenvatinib, or sunitinib, or a pharmacologically acceptable salt thereof.

[17] The pharmaceutical composition according to [2], wherein the kinase inhibitor is a PDGFR inhibitor.

[18] The pharmaceutical composition according to [17], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.

[19] The pharmaceutical composition according to [2], wherein the kinase inhibitor is an FGFR inhibitor.

[20] The pharmaceutical composition according to [19], wherein the FGFR inhibitor is regorafenib, sorafenib, lenvatinib, nintedanib, axitinib, or pazopanib, or a pharmacologically acceptable salt thereof.

[21] The pharmaceutical composition according to [2], wherein the kinase inhibitor is an FLT3 inhibitor.

[22] The pharmaceutical composition according to [21], wherein the FLT3 inhibitor is gilteritinib, quizartinib, midostaurin, sorafenib, ilorasertib, ENMD-2076, or sunitinib, or a pharmacologically acceptable salt thereof.

[23] The pharmaceutical composition according to [2], wherein the kinase inhibitor is an ALK inhibitor.

[24] The pharmaceutical composition according to [23], wherein the ALK inhibitor is brigatinib, crizotinib, ceritinib, alectinib, or lorlatinib, or a pharmacologically acceptable salt thereof.

[25] The pharmaceutical composition according to [2], wherein the kinase inhibitor is a CSF-1R inhibitor.

[26] The pharmaceutical composition according to [25], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.

[27] The pharmaceutical composition according to [2], wherein the kinase inhibitor is an EGFR inhibitor.

[28] The pharmaceutical composition according to [27], wherein the EGFR inhibitor is gefitinib, erlotinib, afatinib, osimertinib, dacomitinib, lapatinib, neratinib, pyrotinib, or poziotinib, or a pharmacologically acceptable salt thereof.

[29] The pharmaceutical composition according to [2], wherein the kinase inhibitor is an HER2 inhibitor.

[30] The pharmaceutical composition according to [29], wherein the HER2 inhibitor is tucatinib, neratinib, mubritinib,

lapatinib, pyrotinib, or poziotinib, or a pharmacologically acceptable salt thereof.

[31] The pharmaceutical composition according to any one of [1] to [30], wherein the antibody in the antibody-drug conjugate is an anti-HER2 antibody, an anti-HER3 antibody, an anti-TROP2 antibody, an anti-B7-H3 antibody, or an anti-CDH6 antibody.

[32] The pharmaceutical composition according to [31], wherein the antibody in the antibody-drug conjugate is an anti-HER2 antibody.

[33] The pharmaceutical composition according to [32], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[34] The pharmaceutical composition according to [32], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[35] The pharmaceutical composition according to [32], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[36] The pharmaceutical composition according to [32], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[37] The pharmaceutical composition according to any one of [32] to [36], wherein the average number of units of the drug-linker conjugated per antibody molecule in the antibody-drug conjugate is in the range of from 7 to 8.

[38] The pharmaceutical composition according to [31], wherein the antibody in the antibody-drug conjugate is an anti-HER3 antibody.

[39] The pharmaceutical composition according to [38], wherein the anti-HER3 antibody is an antibody comprising a heavy chain consisting of the amino acid sequence represented by SEQ ID NO: 3 and a light chain consisting of the amino acid sequence represented by SEQ ID NO: 4.

[40] The pharmaceutical composition according to [39], wherein the anti-HER3 antibody lacks a lysine residue at the carboxyl terminus of the heavy chain.

[41] The pharmaceutical composition according to any one of [38] to [40], wherein the average number of units of the drug-linker conjugated per antibody molecule in the antibody-drug conjugate is in the range of from 7 to 8.

[42] The pharmaceutical composition according to [31], wherein the antibody in the antibody-drug conjugate is an anti-TROP2 antibody.

[43] The pharmaceutical composition according to [42], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[44] The pharmaceutical composition according to [43], wherein the anti-TROP2 antibody lacks a lysine residue at the carboxyl terminus of the heavy chain.

[45] The pharmaceutical composition according to any one of [42] to [44], wherein the average number of units of the drug-linker conjugated per antibody molecule in the antibody-drug conjugate is in the range of from 3.5 to 4.5.

[46] The pharmaceutical composition according to [31], wherein the antibody in the antibody-drug conjugate is an anti-B7-H3 antibody.

[47] The pharmaceutical composition according to [46], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[48] The pharmaceutical composition according to [47], wherein the anti-B7-H3 antibody lacks a lysine residue at the carboxyl terminus of the heavy chain.

[49] The pharmaceutical composition according to any one of [46] to [48], wherein the average number of units of the drug-linker conjugated per antibody molecule in the antibody-drug conjugate is in the range of from 3.5 to 4.5.

[50] The pharmaceutical composition according to [31], wherein the antibody in the antibody-drug conjugate is an anti-CDH6 antibody.

[51] The pharmaceutical composition according to [50], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

[52] The pharmaceutical composition according to [51], wherein the anti-CDH6 antibody lacks a lysine residue at the carboxyl terminus of the heavy chain.

[53] The pharmaceutical composition according to any one of [50] to [52], wherein the average number of units of the drug-linker conjugated per antibody molecule in the antibody-drug conjugate is in the range of from 7 to 8.

[54] The pharmaceutical composition according to any one of [1] to [53], wherein the antibody-drug conjugate and the kinase inhibitor are separately contained as active components in different formulations, and are administered simultaneously or at different times.

[55] The pharmaceutical composition according to any one of [1] to [54], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[56] The pharmaceutical composition according to [55], wherein the pharmaceutical composition is for use in treating breast cancer.

[57] The pharmaceutical composition according to [55], wherein the pharmaceutical composition is for use in treating colorectal cancer.

[58] The pharmaceutical composition according to [55], wherein the pharmaceutical composition is for use in treating gastric cancer.

[59] The pharmaceutical composition according to [55], wherein the pharmaceutical composition is for use in treating lung cancer.

[60] The pharmaceutical composition according to [55], wherein the pharmaceutical composition is for use in treating pancreatic cancer.

[61] The pharmaceutical composition according to [55], wherein the pharmaceutical composition is for use in treating kidney cancer.

[62] The pharmaceutical composition according to [55], wherein the pharmaceutical composition is for use in treating ovarian cancer.

[63] A pharmaceutical composition wherein an antibody-drug conjugate and a kinase inhibitor are administered in combination, and the antibody-drug conjugate is an antibody-drug conjugate represented by the following formula:

[Formula 2]

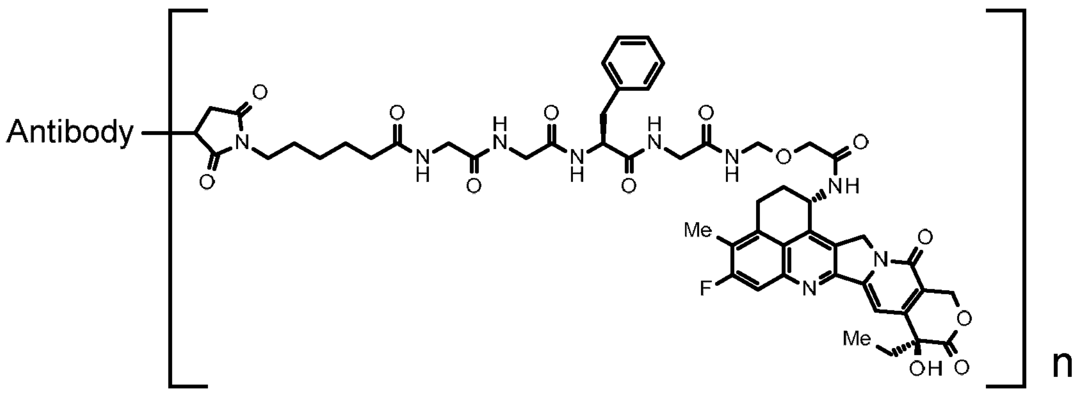

wherein a drug-linker is conjugated to an antibody via a thioether bond, and n is the average number of units of the drug-linker conjugated per antibody molecule, and

the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[64] The pharmaceutical composition according to [63], wherein the kinase inhibitor is at least one selected from the group consisting of a CDK4/6 inhibitor, an mTOR inhibitor, a PI3K inhibitor, an RAF inhibitor, a VEGFR inhibitor,

a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[65] The pharmaceutical composition according to [64], wherein the kinase inhibitor is a CDK4/6 inhibitor.

[66]The pharmaceutical composition according to [65], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[67] The pharmaceutical composition according to [64], wherein the kinase inhibitor is an mTOR inhibitor.

[68] The pharmaceutical composition according to [67], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[69] The pharmaceutical composition according to [64], wherein the kinase inhibitor is a PI3K inhibitor.

[70] The pharmaceutical composition according to [69], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[71] The pharmaceutical composition according to [64], wherein the kinase inhibitor is an RAF inhibitor.

[72] The pharmaceutical composition according to [71], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[73] The pharmaceutical composition according to [64], wherein the kinase inhibitor is a VEGFR inhibitor.

[74] The pharmaceutical composition according to [73], wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[75] The pharmaceutical composition according to [64], wherein the kinase inhibitor is a KIT inhibitor.

[76] The pharmaceutical composition according to [75], wherein the KIT inhibitor is regorafenib, sorafenib, imatinib, ilorasertib, sunitinib, pazopanib, lenvatinib, or dasatinib, or a pharmacologically acceptable salt thereof.

[77] The pharmaceutical composition according to [64], wherein the kinase inhibitor is an RET inhibitor.

[78] The pharmaceutical composition according to [77], wherein the RET inhibitor is regorafenib, sorafenib, vandetanib, lenvatinib, or sunitinib, or a pharmacologically acceptable salt thereof.

[79] The pharmaceutical composition according to [64], wherein the kinase inhibitor is a PDGFR inhibitor.

[80] The pharmaceutical composition according to [79], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.

[81] The pharmaceutical composition according to [64], wherein the kinase inhibitor is an FGFR inhibitor.

[82] The pharmaceutical composition according to [81], wherein the FGFR inhibitor is regorafenib, sorafenib, lenvatinib, nintedanib, axitinib, or pazopanib, or a pharmacologically acceptable salt thereof.

[83] The pharmaceutical composition according to [64], wherein the kinase inhibitor is an FLT3 inhibitor.

[84] The pharmaceutical composition according to [83], wherein the FLT3 inhibitor is gilteritinib, quizartinib, midostaurin, sorafenib, ilorasertib, ENMD-2076, or sunitinib, or a pharmacologically acceptable salt thereof.

[85] The pharmaceutical composition according to [64], wherein the kinase inhibitor is an ALK inhibitor.

[86] The pharmaceutical composition according to [85], wherein the ALK inhibitor is brigatinib, crizotinib, ceritinib, alectinib, or lorlatinib, or a pharmacologically acceptable salt thereof.

[87] The pharmaceutical composition according to [64], wherein the kinase inhibitor is a CSF-1R inhibitor.

[88] The pharmaceutical composition according to [87], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.

[89] The pharmaceutical composition according to [64], wherein the kinase inhibitor is an EGFR inhibitor.

[90] The pharmaceutical composition according to [89], wherein the EGFR inhibitor is gefitinib, erlotinib, afatinib, osimertinib, dacomitinib, lapatinib, neratinib, pyrotinib, or poziotinib, or a pharmacologically acceptable salt thereof.

[91] The pharmaceutical composition according to [64], wherein the kinase inhibitor is an HER2 inhibitor.

[92] The pharmaceutical composition according to [91], wherein the HER2 inhibitor is tucatinib, neratinib, mubritinib, lapatinib, pyrotinib, or poziotinib, or a pharmacologically acceptable salt thereof.

[93] The pharmaceutical composition according to any one of [63] to [92], wherein the antibody in the antibody-drug conjugate is an anti-HER2 antibody, an anti-HER3 antibody, an anti-TROP2 antibody, an anti-B7-H3 antibody, or an anti-CDH6 antibody.

[94] The pharmaceutical composition according to [93], wherein the antibody in the antibody-drug conjugate is an anti-HER2 antibody.

[95] The pharmaceutical composition according to [94], wherein the anti-HER2 antibody is an antibody comprising a heavy chain comprising CDRH1 consisting of an amino acid sequence consisting of amino acid residues 26 to

33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[96] The pharmaceutical composition according to [94], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[97] The pharmaceutical composition according to [94], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[98] The pharmaceutical composition according to [94], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[99] The pharmaceutical composition according to any one of [94] to [98], wherein the average number of units of the drug-linker conjugated per antibody molecule in the antibody-drug conjugate is in the range of from 7 to 8.

[100] The pharmaceutical composition according to [93], wherein the antibody in the antibody-drug conjugate is an anti-HER3 antibody.

[101] The pharmaceutical composition according to [100], wherein the anti-HER3 antibody is an antibody comprising a heavy chain consisting of the amino acid sequence represented by SEQ ID NO: 3 and a light chain consisting of the amino acid sequence represented by SEQ ID NO: 4.

[102] The pharmaceutical composition according to [101], wherein the anti-HER3 antibody lacks a lysine residue at the carboxyl terminus of the heavy chain.

[103] The pharmaceutical composition according to any one of [100] to [102], wherein the average number of units of the drug-linker conjugated per antibody molecule in the antibody-drug conjugate is in the range of from 7 to 8.

[104] The pharmaceutical composition according to [93], wherein the antibody in the antibody-drug conjugate is an anti-TROP2 antibody.

[105] The pharmaceutical composition according to [104], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[106] The pharmaceutical composition according to [105], wherein the anti-TROP2 antibody lacks a lysine residue at the carboxyl terminus of the heavy chain.

[107] The pharmaceutical composition according to any one of [104] to [106], wherein the average number of units of the drug-linker conjugated per antibody molecule in the antibody-drug conjugate is in the range of from 3.5 to 4.5.

[108] The pharmaceutical composition according to [93], wherein the antibody in the antibody-drug conjugate is an anti-B7-H3 antibody.

[109] The pharmaceutical composition according to [108], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[110] The pharmaceutical composition according to [109], wherein the anti-B7-H3 antibody lacks a lysine residue at the carboxyl terminus of the heavy chain.

[111] The pharmaceutical composition according to any one of [108] to [110], wherein the average number of units of the drug-linker conjugated per antibody molecule in the antibody-drug conjugate is in the range of from 3.5 to 4.5.

[112] The pharmaceutical composition according to [93], wherein the antibody in the antibody-drug conjugate is an anti-CDH6 antibody.

[113] The pharmaceutical composition according to [112], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

[114] The pharmaceutical composition according to [113], wherein the anti-CDH6 antibody lacks a lysine residue at the carboxyl terminus of the heavy chain.

[115] The pharmaceutical composition according to any one of [112] to [114], wherein the average number of units of the drug-linker conjugated per antibody molecule in the antibody-drug conjugate is in the range of from 7 to 8.

[116] The pharmaceutical composition according to any one of [63] to [115], wherein the antibody-drug conjugate and the kinase inhibitor are separately contained as active components in different formulations, and are administered simultaneously or at different times.

[117] The pharmaceutical composition according to any one of [63] to [116], wherein the pharmaceutical composition

is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[118] The pharmaceutical composition according to [117], wherein the pharmaceutical composition is for use in treating breast cancer.

[119] The pharmaceutical composition according to [117], wherein the pharmaceutical composition is for use in treating colorectal cancer.

[120] The pharmaceutical composition according to [117], wherein the pharmaceutical composition is for use in treating gastric cancer.

[121] The pharmaceutical composition according to [117], wherein the pharmaceutical composition is for use in treating lung cancer.

[122] The pharmaceutical composition according to [117], wherein the pharmaceutical composition is for use in treating pancreatic cancer.

[123] The pharmaceutical composition according to [117], wherein the pharmaceutical composition is for use in treating kidney cancer.

[124] The pharmaceutical composition according to [117], wherein the pharmaceutical composition is for use in treating ovarian cancer.

[125] A method of treatment, comprising administering an antibody-drug conjugate and a kinase inhibitor in combination to a subject in need of treatment, wherein the antibody-drug conjugate is an antibody-drug conjugate in which a drug-linker represented by the following formula:

[Formula 3]

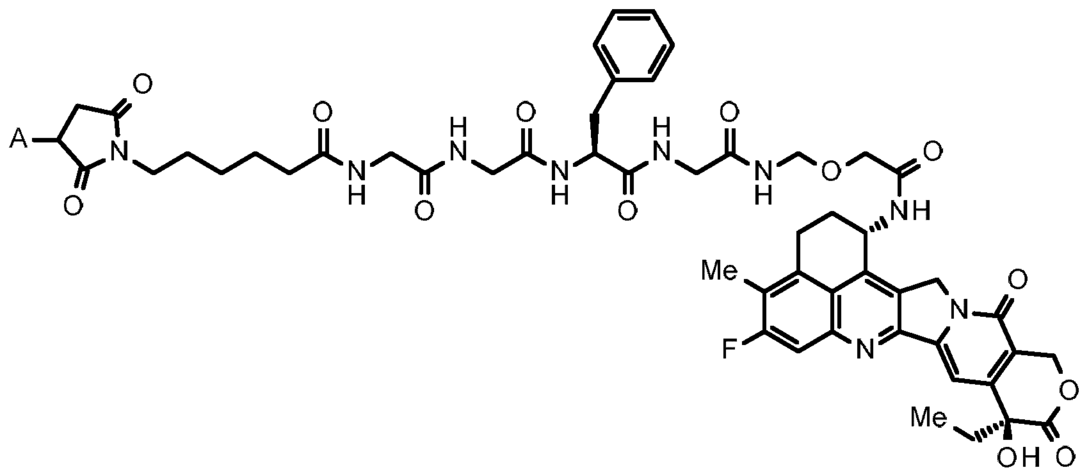

wherein A represents a connecting position to an antibody, is conjugated to the antibody via a thioether bond, and the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[126] The method of treatment according to [125], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[127] The method of treatment according to [126], wherein the kinase inhibitor is a CDK4/6 inhibitor.

[128] The method of treatment according to [127], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[129] The method of treatment according to [126], wherein the kinase inhibitor is an mTOR inhibitor.

[130] The method of treatment according to [129], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[131] The method of treatment according to [126], wherein the kinase inhibitor is a PI3K inhibitor.

[132] The method of treatment according to [131], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[133] The method of treatment according to [126], wherein the kinase inhibitor is an RAF inhibitor.

[134] The method of treatment according to [133], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[135] The method of treatment according to [126], wherein the kinase inhibitor is a VEGFR inhibitor.

[136] The method of treatment according to [135], wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[137] The method of treatment according to [126], wherein the kinase inhibitor is a KIT inhibitor.

[138] The method of treatment according to [137], wherein the KIT inhibitor is regorafenib, sorafenib, imatinib, ilorasertib, sunitinib, pazopanib, lenvatinib, or dasatinib, or a pharmacologically acceptable salt thereof.

[139] The method of treatment according to [126], wherein the kinase inhibitor is an RET inhibitor.

[140] The method of treatment according to [139], wherein the RET inhibitor is regorafenib, sorafenib, vandetanib, lenvatinib, or sunitinib, or a pharmacologically acceptable salt thereof.

[141] The method of treatment according to [126], wherein the kinase inhibitor is a PDGFR inhibitor.

[142] The method of treatment according to [141], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.

[143] The method of treatment according to [126], wherein the kinase inhibitor is a FGFR inhibitor.

[144] The method of treatment according to [143], wherein the FGFR inhibitor is regorafenib, sorafenib, lenvatinib, nintedanib, axitinib, or pazopanib, or a pharmacologically acceptable salt thereof.

[145] The method of treatment according to [126], wherein the kinase inhibitor is a FLT3 inhibitor.

[146] The method of treatment according to [145], wherein the FLT3 inhibitor is gilteritinib, quizartinib, midostaurin, sorafenib, ilorasertib, ENMD-2076, or sunitinib, or a pharmacologically acceptable salt thereof.

[147] The method of treatment according to [126], wherein the kinase inhibitor is an ALK inhibitor.

[148] The method of treatment according to [147], wherein the ALK inhibitor is brigatinib, crizotinib, ceritinib, alectinib, or lorlatinib, or a pharmacologically acceptable salt thereof.

[149] The method of treatment according to [126], wherein the kinase inhibitor is a CSF-1R inhibitor.

[150] The method of treatment according to [149], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.

[151] The method of treatment according to [126], wherein the kinase inhibitor is an EGFR inhibitor.

[152] The method of treatment according to [151], wherein the EGFR inhibitor is gefitinib, erlotinib, afatinib, osimertinib, dacomitinib, lapatinib, neratinib, pyrotinib, or poziotinib, or a pharmacologically acceptable salt thereof.

[153] The method of treatment according to [126], wherein the kinase inhibitor is an HER2 inhibitor.

[154] The method of treatment according to [153], wherein the HER2 inhibitor is tucatinib, neratinib, mubritinib, lapatinib, pyrotinib, or poziotinib, or a pharmacologically acceptable salt thereof.

[155] The method of treatment according to any one of [125] to [154], wherein the antibody in the antibody-drug conjugate is an anti-HER2 antibody, an anti-HER3 antibody, an anti-TROP2 antibody, an anti-B7-H3 antibody, or an anti-CDH6 antibody.

[156] The method of treatment according to [155], wherein the antibody in the antibody-drug conjugate is an anti-HER2 antibody.

[157] The method of treatment according to [156], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[158] The method of treatment according to [156], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[159] The method of treatment according to [156], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[160] The method of treatment according to [156], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[161] The method of treatment according to any one of [156] to [160], wherein the average number of units of the drug-linker conjugated per antibody molecule in the antibody-drug conjugate is in the range of from 7 to 8.
[162] The method of treatment according to [155], wherein the antibody in the antibody-drug conjugate is an anti-HER3 antibody.
[163] The method of treatment according to [162], wherein the anti-HER3 antibody is an antibody comprising a heavy chain consisting of the amino acid sequence represented by SEQ ID NO: 3 and a light chain consisting of the amino acid sequence represented by SEQ ID NO: 4.
[164] The method of treatment according to [163], wherein the anti-HER3 antibody lacks a lysine residue at the carboxyl terminus of the heavy chain.
[165] The method of treatment according to any one of [162] to [164], wherein the average number of units of the drug-linker conjugated per antibody molecule in the antibody-drug conjugate is in the range of from 7 to 8.
[166] The method of treatment according to [155], wherein the antibody in the antibody-drug conjugate is an anti-TROP2 antibody.
[167] The method of treatment according to [166], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[168] The method of treatment according to [167], wherein the anti-TROP2 antibody lacks a lysine residue at the carboxyl terminus of the heavy chain.
[169] The method of treatment according to any one of [166] to [168], wherein the average number of units of the drug-linker conjugated per antibody molecule in the antibody-drug conjugate is in the range of from 3.5 to 4.5.
[170] The method of treatment according to [155], wherein the antibody in the antibody-drug conjugate is an anti-B7-H3 antibody.
[171] The method of treatment according to [170], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[172] The method of treatment according to [171], wherein the anti-B7-H3 antibody lacks a lysine residue at the carboxyl terminus of the heavy chain.
[173] The method of treatment according to any one of [170] to [172], wherein the average number of units of the drug-linker conjugated per antibody molecule in the antibody-drug conjugate is in the range of from 3.5 to 4.5.
[174] The method of treatment according to [155], wherein the antibody in the antibody-drug conjugate is an anti-CDH6 antibody.
[175] The method of treatment according to [174], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.
[176] The method of treatment according to [175], wherein the anti-CDH6 antibody lacks a lysine residue at the carboxyl terminus of the heavy chain.
[177] The method of treatment according to any one of [174] to [176], wherein the average number of units of the drug-linker conjugated per antibody molecule in the antibody-drug conjugate is in the range of from 7 to 8.
[178] The method of treatment according to any one of [125] to [177], wherein the antibody-drug conjugate and the kinase inhibitor are separately contained as active components in different formulations, and are administered simultaneously or at different times.
[179] The method of treatment according to any one of [125] to [178], wherein the method of treatment is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and

melanoma.

[180] The method of treatment according to [179], wherein the method of treatment is for treating breast cancer.

[181] The method of treatment according to [179], wherein the method of treatment is for treating colorectal cancer.

[182] The method of treatment according to [179], wherein the method of treatment is for treating gastric cancer.

[183] The method of treatment according to [179], wherein the method of treatment is for treating lung cancer.

[184] The method of treatment according to [179], wherein the method of treatment is for treating pancreatic cancer.

[185] The method of treatment according to [179], wherein the method of treatment is for treating kidney cancer.

[186] The method of treatment according to [179], wherein the method of treatment is for treating ovarian cancer.

[187] A method of treatment, comprising administering an antibody-drug conjugate and a kinase inhibitor in combination to a subject in need of treatment, wherein the antibody-drug conjugate is an antibody-drug conjugate represented by the following formula:

[Formula 4]

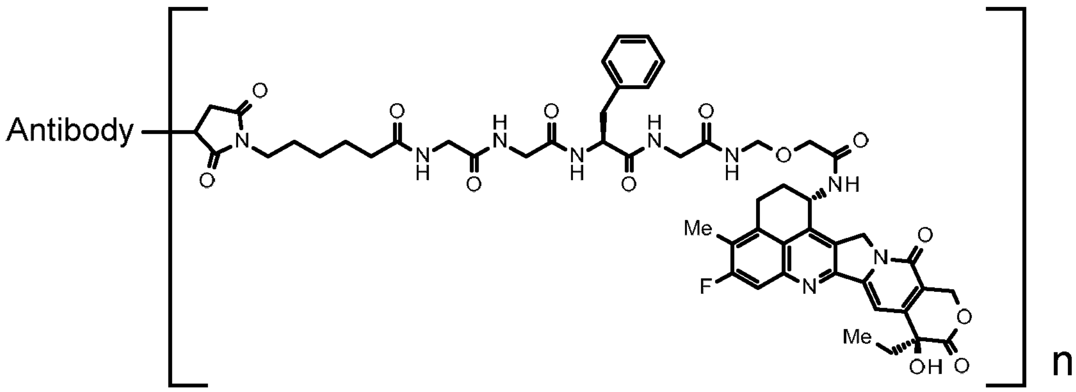

wherein a drug-linker is conjugated to an antibody via a thioether bond, and n is the average number of units of the drug-linker conjugated per antibody molecule, and

the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[188] The method of treatment according to [187], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[189] The method of treatment according to [188], wherein the kinase inhibitor is a CDK4/6 inhibitor.

[190] The method of treatment according to [189], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[191] The method of treatment according to [188], wherein the kinase inhibitor is an mTOR inhibitor.

[192] The method of treatment according to [191], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[193] The method of treatment according to [188], wherein the kinase inhibitor is a PI3K inhibitor.

[194] The method of treatment according to [193], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[195] The method of treatment according to [188], wherein the kinase inhibitor is an RAF inhibitor.

[196] The method of treatment according to [195], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[197] The method of treatment according to [188], wherein the kinase inhibitor is a VEGFR inhibitor.

[198] The method of treatment according to [197], wherein the VEGFR inhibitor is regorafenib, sorafenib, vandetanib,

sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.
[199] The method of treatment according to [188], wherein the kinase inhibitor is a KIT inhibitor.
[200] The method of treatment according to [199], wherein the KIT inhibitor is regorafenib, sorafenib, imatinib, ilorasertib, sunitinib, pazopanib, lenvatinib, or dasatinib, or a pharmacologically acceptable salt thereof.
[201] The method of treatment according to [188], wherein the kinase inhibitor is an RET inhibitor.
[202] The method of treatment according to [201], wherein the RET inhibitor is regorafenib, sorafenib, vandetanib, lenvatinib, or sunitinib, or a pharmacologically acceptable salt thereof.
[203] The method of treatment according to [188], wherein the kinase inhibitor is a PDGFR inhibitor.
[204] The method of treatment according to [203], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.
[205] The method of treatment according to [188], wherein the kinase inhibitor is a FGFR inhibitor.
[206] The method of treatment according to [205], wherein the FGFR inhibitor is regorafenib, sorafenib, lenvatinib, nintedanib, axitinib, or pazopanib, or a pharmacologically acceptable salt thereof.
[207] The method of treatment according to [188], wherein the kinase inhibitor is an FLT3 inhibitor.
[208] The method of treatment according to [207], wherein the FLT3 inhibitor is gilteritinib, quizartinib, midostaurin, sorafenib, ilorasertib, ENMD-2076, or sunitinib, or a pharmacologically acceptable salt thereof.
[209] The method of treatment according to [188], wherein the kinase inhibitor is an ALK inhibitor.
[210] The method of treatment according to [209], wherein the ALK inhibitor is brigatinib, crizotinib, ceritinib, alectinib, or lorlatinib, or a pharmacologically acceptable salt thereof.
[211] The method of treatment according to [188], wherein the kinase inhibitor is a CSF-1R inhibitor.
[212] The method of treatment according to [211], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.
[213] The method of treatment according to [188], wherein the kinase inhibitor is an EGFR inhibitor.
[214] The method of treatment according to [213], wherein the EGFR inhibitor is gefitinib, erlotinib, afatinib, osimertinib, dacomitinib, lapatinib, neratinib, pyrotinib, or poziotinib, or a pharmacologically acceptable salt thereof.
[215] The method of treatment according to [188], wherein the kinase inhibitor is an HER2 inhibitor.
[216] The method of treatment according to [215], wherein the HER2 inhibitor is tucatinib, neratinib, mubritinib, lapatinib, pyrotinib, or poziotinib, or a pharmacologically acceptable salt thereof.
[217] The method of treatment according to any one of [187] to [216], wherein the antibody in the antibody-drug conjugate is an anti-HER2 antibody, an anti-HER3 antibody, an anti-TROP2 antibody, an anti-B7-H3 antibody, or an anti-CDH6 antibody.
[218] The method of treatment according to [217], wherein the antibody in the antibody-drug conjugate is an anti-HER2 antibody.
[219] The method of treatment according to [218], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.
[220] The method of treatment according to [218], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[221] The method of treatment according to [218], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[222] The method of treatment according to [218], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[223] The method of treatment according to any one of [218] to [222], wherein the average number of units of the drug-linker conjugated per antibody molecule in the antibody-drug conjugate is in the range of from 7 to 8.
[224] The method of treatment according to [217], wherein the antibody in the antibody-drug conjugate is an anti-HER3 antibody.
[225] The method of treatment according to [224], wherein the anti-HER3 antibody is an antibody comprising a

heavy chain consisting of the amino acid sequence represented by SEQ ID NO: 3 and a light chain consisting of the amino acid sequence represented by SEQ ID NO: 4.

[226] The method of treatment according to [225], wherein the anti-HER3 antibody lacks a lysine residue at the carboxyl terminus of the heavy chain.

[227] The method of treatment according to any one of [224] to [226], wherein the average number of units of the drug-linker conjugated per antibody molecule in the antibody-drug conjugate is in the range of from 7 to 8.

[228] The method of treatment according to [217], wherein the antibody in the antibody-drug conjugate is an anti-TROP2 antibody.

[229] The method of treatment according to [228], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[230] The method of treatment according to [229], wherein the anti-TROP2 antibody lacks a lysine residue at the carboxyl terminus of the heavy chain.

[231] The method of treatment according to any one of [228] to [230], wherein the average number of units of the drug-linker conjugated per antibody molecule in the antibody-drug conjugate is in the range of from 3.5 to 4.5.

[232] The method of treatment according to [217], wherein the antibody in the antibody-drug conjugate is an anti-B7-H3 antibody.

[233] The method of treatment according to [232], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[234] The method of treatment according to [233], wherein the anti-B7-H3 antibody lacks a lysine residue at the carboxyl terminus of the heavy chain.

[235] The method of treatment according to any one of [232] to [234], wherein the average number of units of the drug-linker conjugated per antibody molecule in the antibody-drug conjugate is in the range of from 3.5 to 4.5.

[236] The method of treatment according to [217], wherein the antibody in the antibody-drug conjugate is an anti-CDH6 antibody.

[237] The method of treatment according to [236], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

[238] The method of treatment according to [237], wherein the anti-CDH6 antibody lacks a lysine residue at the carboxyl terminus of the heavy chain.

[239] The method of treatment according to any one of [236] to [238], wherein the average number of units of the drug-linker conjugated per antibody molecule in the antibody-drug conjugate is in the range of from 7 to 8.

[240] The method of treatment according to any one of [187] to [231], wherein the antibody-drug conjugate and the kinase inhibitor are separately contained as active components in different formulations, and are administered simultaneously or at different times.

[241] The method of treatment according to any one of [187] to [240], wherein the method of treatment is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[242] The method of treatment according to [241], wherein the method of treatment is for treating breast cancer.

[243] The method of treatment according to [241], wherein the method of treatment is for treating colorectal cancer.

[244] The method of treatment according to [241], wherein the method of treatment is for treating gastric cancer.

[245] The method of treatment according to [241], wherein the method of treatment is for treating lung cancer.

[246] The method of treatment according to [241], wherein the method of treatment is for treating pancreatic cancer.

[247] The method of treatment according to [241], wherein the method of treatment is for treating kidney cancer.

[248] The method of treatment according to [241], wherein the method of treatment is for treating ovarian cancer.

[249] An antibody-drug conjugate for use in treating a disease through being administered in combination with a kinase inhibitor, wherein a drug-linker represented by the following formula:

[Formula 5]

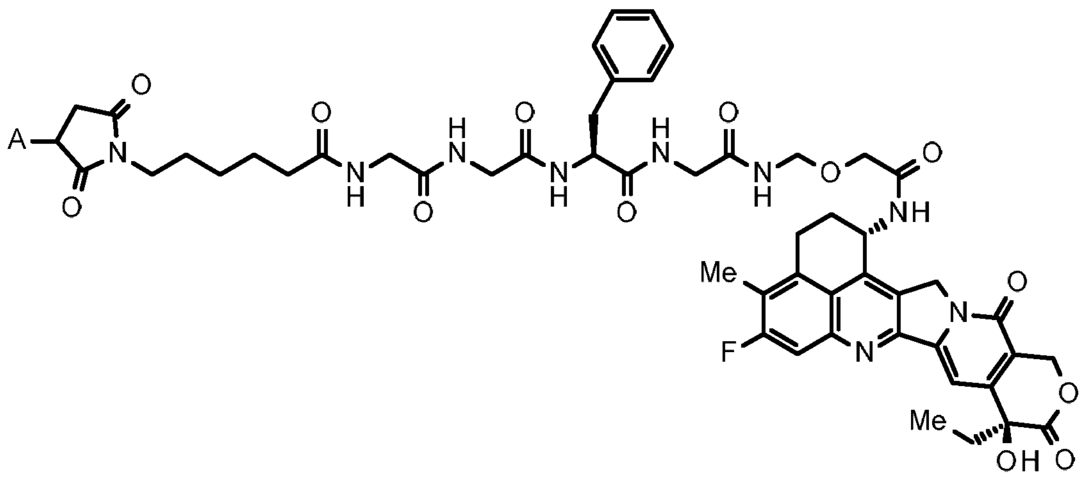

wherein A represents a connecting position to an antibody, is conjugated to the antibody via a thioether bond in the antibody-drug conjugate, and
the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[250] The antibody-drug conjugate according to [249], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[251] The antibody-drug conjugate according to [250], wherein the kinase inhibitor is a CDK4/6 inhibitor.

[252] The antibody-drug conjugate according to [251], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[253] The antibody-drug conjugate according to [250], wherein the kinase inhibitor is an mTOR inhibitor.

[254] The antibody-drug conjugate according to [253], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[255] The antibody-drug conjugate according to [250], wherein the kinase inhibitor is a PI3K inhibitor.

[256] The antibody-drug conjugate according to [255], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[257] The antibody-drug conjugate according to [250], wherein the kinase inhibitor is an RAF inhibitor.

[258] The antibody-drug conjugate according to [257], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[259] The antibody-drug conjugate according to [250], wherein the kinase inhibitor is a VEGFR inhibitor.

[260] The antibody-drug conjugate according to [259], wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[261] The antibody-drug conjugate according to [250], wherein the kinase inhibitor is a KIT inhibitor.

[262] The antibody-drug conjugate according to [261], wherein the KIT inhibitor is regorafenib, sorafenib, imatinib, ilorasertib, sunitinib, pazopanib, lenvatinib, or dasatinib, or a pharmacologically acceptable salt thereof.

[263] The antibody-drug conjugate according to [250], wherein the kinase inhibitor is an RET inhibitor.

[264] The antibody-drug conjugate according to [263], wherein the RET inhibitor is regorafenib, sorafenib, vandetanib, lenvatinib, or sunitinib, or a pharmacologically acceptable salt thereof.

[265] The antibody-drug conjugate according to [250], wherein the kinase inhibitor is a PDGFR inhibitor.

[266] The antibody-drug conjugate according to [265], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.
[267] The antibody-drug conjugate according to [250], wherein the kinase inhibitor is an FGFR inhibitor.
[268] The antibody-drug conjugate according to [267], wherein the FGFR inhibitor is regorafenib, sorafenib, lenvatinib, nintedanib, axitinib, or pazopanib, or a pharmacologically acceptable salt thereof.
[269] The antibody-drug conjugate according to [250], wherein the kinase inhibitor is an FLT3 inhibitor.
[270] The antibody-drug conjugate according to [269], wherein the FLT3 inhibitor is gilteritinib, quizartinib, midostaurin, sorafenib, ilorasertib, ENMD-2076, or sunitinib, or a pharmacologically acceptable salt thereof.
[271] The antibody-drug conjugate according to [250], wherein the kinase inhibitor is an ALK inhibitor.
[272] The antibody-drug conjugate according to [271], wherein the ALK inhibitor is brigatinib, crizotinib, ceritinib, alectinib, or lorlatinib, or a pharmacologically acceptable salt thereof.
[273] The antibody-drug conjugate according to [250], wherein the kinase inhibitor is a CSF-1R inhibitor.
[274] The antibody-drug conjugate according to [273], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.
[275] The antibody-drug conjugate according to [250], wherein the kinase inhibitor is an EGFR inhibitor.
[276] The antibody-drug conjugate according to [275], wherein the EGFR inhibitor is gefitinib, erlotinib, afatinib, osimertinib, dacomitinib, lapatinib, neratinib, pyrotinib, or poziotinib, or a pharmacologically acceptable salt thereof.
[277] The antibody-drug conjugate according to [250], wherein the kinase inhibitor is an HER2 inhibitor.
[278] The antibody-drug conjugate according to [277], wherein the HER2 inhibitor is tucatinib, neratinib, mubritinib, lapatinib, pyrotinib, or poziotinib, or a pharmacologically acceptable salt thereof.
[279] The antibody-drug conjugate according to any one of [249] to [278], wherein the antibody in the antibody-drug conjugate is an anti-HER2 antibody, an anti-HER3 antibody, an anti-TROP2 antibody, an anti-B7-H3 antibody, or an anti-CDH6 antibody.
[280] The antibody-drug conjugate according to [279], wherein the antibody in the antibody-drug conjugate is an anti-HER2 antibody.
[281] The antibody-drug conjugate according to [280], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.
[282] The antibody-drug conjugate according to [280], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[283] The antibody-drug conjugate according to [280], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[284] The antibody-drug conjugate according to [280], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[285] The antibody-drug conjugate according to any one of [280] to [284], wherein the average number of units of the drug-linker conjugated per antibody molecule in the antibody-drug conjugate is in the range of from 7 to 8.
[286] The antibody-drug conjugate according to [279], wherein the antibody in the antibody-drug conjugate is an anti-HER3 antibody.
[287] The antibody-drug conjugate according to [286], wherein the anti-HER3 antibody is an antibody comprising a heavy chain consisting of the amino acid sequence represented by SEQ ID NO: 3 and a light chain consisting of the amino acid sequence represented by SEQ ID NO: 4.
[288] The antibody-drug conjugate according to [287], wherein the anti-HER3 antibody lacks a lysine residue at the carboxyl terminus of the heavy chain.
[289] The antibody-drug conjugate according to any one of [286] to [288], wherein the average number of units of the drug-linker conjugated per antibody molecule in the antibody-drug conjugate is in the range of from 7 to 8.
[290] The antibody-drug conjugate according to [279], wherein the antibody in the antibody-drug conjugate is an anti-TROP2 antibody.
[291] The antibody-drug conjugate according to [290], wherein the anti-TROP2 antibody is an antibody comprising a heavy chain consisting of an amino acid sequence consisting of amino acid residues 20 to 470 of SEQ ID NO: 5

and a light chain consisting of an amino acid sequence consisting of amino acid residues 21 to 234 of SEQ ID NO: 6.

[292] The antibody-drug conjugate according to [291], wherein the anti-TROP2 antibody lacks a lysine residue at the carboxyl terminus of the heavy chain.

[293] The antibody-drug conjugate according to any one of [290] to [292], wherein the average number of units of the drug-linker conjugated per antibody molecule in the antibody-drug conjugate is in the range of from 3.5 to 4.5.

[294] The antibody-drug conjugate according to [279], wherein the antibody in the antibody-drug conjugate is an anti-B7-H3 antibody.

[295] The antibody-drug conjugate according to [294], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[296] The antibody-drug conjugate according to [295], wherein the anti-B7-H3 antibody lacks a lysine residue at the carboxyl terminus of the heavy chain.

[297] The antibody-drug conjugate according to any one of [294] to [296], wherein the average number of units of the drug-linker conjugated per antibody molecule in the antibody-drug conjugate is in the range of from 3.5 to 4.5.

[298] The antibody-drug conjugate according to [279], wherein the antibody in the antibody-drug conjugate is an anti-CDH6 antibody.

[299] The antibody-drug conjugate according to [298], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

[300] The antibody-drug conjugate according to [299], wherein the anti-CDH6 antibody lacks a lysine residue at the carboxyl terminus of the heavy chain.

[301] The antibody-drug conjugate according to any one of [298] to [300], wherein the average number of units of the drug-linker conjugated per antibody molecule in the antibody-drug conjugate is in the range of from 7 to 8.

[302] The antibody-drug conjugate according to any one of [249] to [301], wherein the antibody-drug conjugate and the kinase inhibitor are separately contained as active components in different formulations, and are administered simultaneously or at different times.

[303] The antibody-drug conjugate according to any one of [249] to [302], wherein the antibody-drug conjugate is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[304] The antibody-drug conjugate according to [303], wherein the antibody-drug conjugate is for use in treating breast cancer.

[305] The antibody-drug conjugate according to [303], wherein the antibody-drug conjugate is for use in treating colorectal cancer.

[306] The antibody-drug conjugate according to [303], wherein the antibody-drug conjugate is for use in treating gastric cancer.

[307] The antibody-drug conjugate according to [303], wherein the antibody-drug conjugate is for use in treating lung cancer.

[308] The antibody-drug conjugate according to [303], wherein the antibody-drug conjugate is for use in treating pancreatic cancer.

[309] The antibody-drug conjugate according to [303], wherein the antibody-drug conjugate is for use in treating kidney cancer.

[310] The antibody-drug conjugate according to [303], wherein the antibody-drug conjugate is for use in treating ovarian cancer.

[311] An antibody-drug conjugate for use in treating a disease through being administered in combination with a kinase inhibitor, wherein the antibody-drug conjugate is an antibody-drug conjugate represented by the following formula:

[Formula 6]

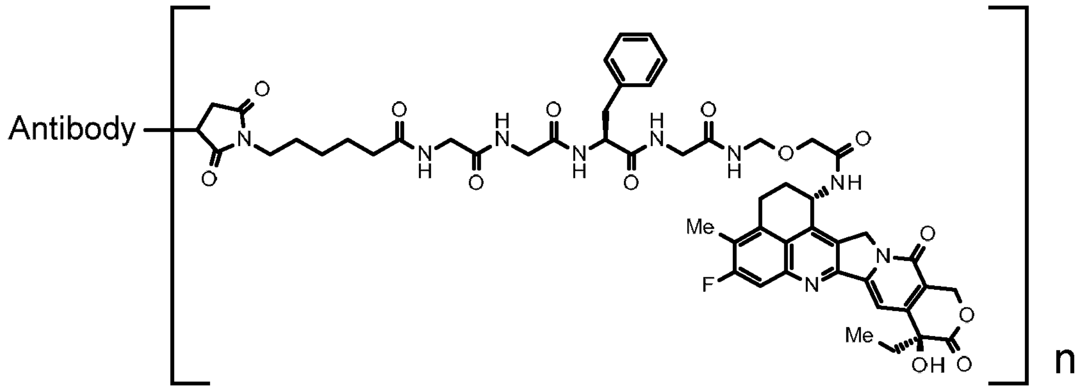

wherein a drug-linker is conjugated to an antibody via a thioether bond, and n is the average number of units of the drug-linker conjugated per antibody molecule, and

the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[312] The antibody-drug conjugate according to [311], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[313] The antibody-drug conjugate according to [312], wherein the kinase inhibitor is a CDK4/6 inhibitor.

[314] The antibody-drug conjugate according to [313], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[315] The antibody-drug conjugate according to [312], wherein the kinase inhibitor is an mTOR inhibitor.

[316] The antibody-drug conjugate according to [315], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[317] The antibody-drug conjugate according to [312], wherein the kinase inhibitor is a PI3K inhibitor.

[318] The antibody-drug conjugate according to [317], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[319] The antibody-drug conjugate according to [312], wherein the kinase inhibitor is an RAF inhibitor.

[320] The antibody-drug conjugate according to [319], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[321] The antibody-drug conjugate according to [312], wherein the kinase inhibitor is a VEGFR inhibitor.

[322] The antibody-drug conjugate according to [321], wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[323] The antibody-drug conjugate according to [312], wherein the kinase inhibitor is a KIT inhibitor.

[324] The antibody-drug conjugate according to [323], wherein the KIT inhibitor is regorafenib, sorafenib, imatinib, ilorasertib, sunitinib, pazopanib, lenvatinib, or dasatinib, or a pharmacologically acceptable salt thereof.

[325] The antibody-drug conjugate according to [312], wherein the kinase inhibitor is an RET inhibitor.

[326] The antibody-drug conjugate according to [325], wherein the RET inhibitor is regorafenib, sorafenib, vandetanib, lenvatinib, or sunitinib, or a pharmacologically acceptable salt thereof.

[327] The antibody-drug conjugate according to [312], wherein the kinase inhibitor is a PDGFR inhibitor.

[328] The antibody-drug conjugate according to [327], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically

acceptable salt thereof.

[329] The antibody-drug conjugate according to [312], wherein the kinase inhibitor is an FGFR inhibitor.

[330] The antibody-drug conjugate according to [329], wherein the FGFR inhibitor is regorafenib, sorafenib, lenvatinib, nintedanib, axitinib, or pazopanib, or a pharmacologically acceptable salt thereof.

[331] The antibody-drug conjugate according to [312], wherein the kinase inhibitor is an FLT3 inhibitor.

[332] The antibody-drug conjugate according to [331], wherein the FLT3 inhibitor is gilteritinib, quizartinib, midostaurin, sorafenib, ilorasertib, ENMD-2076, or sunitinib, or a pharmacologically acceptable salt thereof.

[333] The antibody-drug conjugate according to [312], wherein the kinase inhibitor is an ALK inhibitor.

[334] The antibody-drug conjugate according to [333], wherein the ALK inhibitor is brigatinib, crizotinib, ceritinib, alectinib, or lorlatinib, or a pharmacologically acceptable salt thereof.

[335] The antibody-drug conjugate according to [312], wherein the kinase inhibitor is a CSF-1R inhibitor.

[336] The antibody-drug conjugate according to [335], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.

[337] The antibody-drug conjugate according to [312], wherein the kinase inhibitor is an EGFR inhibitor.

[338] The antibody-drug conjugate according to [337], wherein the EGFR inhibitor is gefitinib, erlotinib, afatinib, osimertinib, dacomitinib, lapatinib, neratinib, pyrotinib, or poziotinib, or a pharmacologically acceptable salt thereof.

[339] The antibody-drug conjugate according to [312], wherein the kinase inhibitor is an HER2 inhibitor.

[340] The antibody-drug conjugate according to [339], wherein the HER2 inhibitor is tucatinib, neratinib, mubritinib, lapatinib, pyrotinib, or poziotinib, or a pharmacologically acceptable salt thereof.

[341] The antibody-drug conjugate according to any one of [311] to [340], wherein the antibody in the antibody-drug conjugate is an anti-HER2 antibody, an anti-HER3 antibody, an anti-TROP2 antibody, an anti-B7-H3 antibody, or an anti-CDH6 antibody.

[342] The antibody-drug conjugate according to [341], wherein the antibody in the antibody-drug conjugate is an anti-HER2 antibody.

[343] The antibody-drug conjugate according to [342], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[344] The antibody-drug conjugate according to [342], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[345] The antibody-drug conjugate according to [342], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[346] The antibody-drug conjugate according to [342], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[347] The antibody-drug conjugate according to any one of [342] to [346], wherein the average number of units of the drug-linker conjugated per antibody molecule in the antibody-drug conjugate is in the range of from 7 to 8.

[348] The antibody-drug conjugate according to [341], wherein the antibody in the antibody-drug conjugate is an anti-HER3 antibody.

[349] The antibody-drug conjugate according to [348], wherein the anti-HER3 antibody is an antibody comprising a heavy chain consisting of the amino acid sequence represented by SEQ ID NO: 3 and a light chain consisting of the amino acid sequence represented by SEQ ID NO: 4.

[350] The antibody-drug conjugate according to [349], wherein the anti-HER3 antibody lacks a lysine residue at the carboxyl terminus of the heavy chain.

[351] The antibody-drug conjugate according to any one of [348] to [350], wherein the average number of units of the drug-linker conjugated per antibody molecule in the antibody-drug conjugate is in the range of from 7 to 8.

[352] The antibody-drug conjugate according to [341], wherein the antibody in the antibody-drug conjugate is an anti-TROP2 antibody.

[353] The antibody-drug conjugate according to [352], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[354] The antibody-drug conjugate according to [353], wherein the anti-TROP2 antibody lacks a lysine residue at the carboxyl terminus of the heavy chain.

[355] The antibody-drug conjugate according to any one of [352] to [354], wherein the average number of units of the drug-linker conjugated per antibody molecule in the antibody-drug conjugate is in the range of from 3.5 to 4.5.

[356] The antibody-drug conjugate according to [341], wherein the antibody in the antibody-drug conjugate is an anti-B7-H3 antibody.

[357] The antibody-drug conjugate according to [356], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[358] The antibody-drug conjugate according to [357], wherein the anti-B7-H3 antibody lacks a lysine residue at the carboxyl terminus of the heavy chain.

[359] The antibody-drug conjugate according to any one of [356] to [358], wherein the average number of units of the drug-linker conjugated per antibody molecule in the antibody-drug conjugate is in the range of from 3.5 to 4.5.

[360] The antibody-drug conjugate according to [341], wherein the antibody in the antibody-drug conjugate is an anti-CDH6 antibody.

[361] The antibody-drug conjugate according to [360], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

[362] The antibody-drug conjugate according to [361], wherein the anti-CDH6 antibody lacks a lysine residue at the carboxyl terminus of the heavy chain.

[363] The antibody-drug conjugate according to any one of [360] to [362], wherein the average number of units of the drug-linker conjugated per antibody molecule in the antibody-drug conjugate is in the range of from 7 to 8.

[364] The antibody-drug conjugate according to any one of [311] to [363], wherein the antibody-drug conjugate and the kinase inhibitor are separately contained as active components in different formulations, and are administered simultaneously or at different times.

[365] The antibody-drug conjugate according to any one of [311] to [364], wherein the antibody-drug conjugate is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[366] The antibody-drug conjugate according to [365], wherein the antibody-drug conjugate is for use in treating breast cancer.

[367] The antibody-drug conjugate according to [365], wherein the antibody-drug conjugate is for use in treating colorectal cancer.

[368] The antibody-drug conjugate according to [365], wherein the antibody-drug conjugate is for use in treating gastric cancer.

[369] The antibody-drug conjugate according to [365], wherein the antibody-drug conjugate is for use in treating lung cancer.

[370] The antibody-drug conjugate according to [365], wherein the antibody-drug conjugate is for use in treating pancreatic cancer.

[371] The antibody-drug conjugate according to [365], wherein the antibody-drug conjugate is for use in treating kidney cancer.

[372] The antibody-drug conjugate according to [365], wherein the antibody-drug conjugate is for use in treating ovarian cancer.

[373] Use of an antibody-drug conjugate for the manufacture of a medicament for treating a disease through being administered in combination with a kinase inhibitor, wherein a drug-linker represented by the following formula:

[Formula 7]

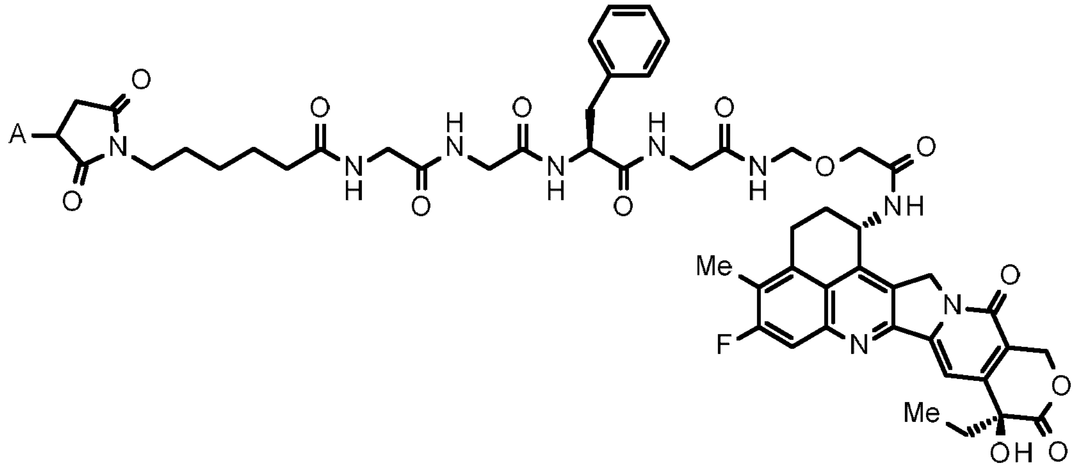

wherein A represents a connecting position to an antibody, is conjugated to the antibody via a thioether bond in the antibody-drug conjugate, and

the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[374] The use according to [373], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[375] The use according to [374], wherein the kinase inhibitor is a CDK4/6 inhibitor.

[376] The use according to [375], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[377] The use according to [374], wherein the kinase inhibitor is an mTOR inhibitor.

[378] The use according to [377], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[379] The use according to [374], wherein the kinase inhibitor is a PI3K inhibitor.

[380] The use according to [379], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[381] The use according to [374], wherein the kinase inhibitor is an RAF inhibitor.

[382] The use according to [381], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[383] The use according to [374], wherein the kinase inhibitor is a VEGFR inhibitor.

[384] The use according to [383], wherein the VEGFR inhibitor is regorafenib, sorafenib, vandetanib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[385] The use according to [374], wherein the kinase inhibitor is a KIT inhibitor.

[386] The use according to [385], wherein the KIT inhibitor is regorafenib, sorafenib, imatinib, ilorasertib, sunitinib, pazopanib, lenvatinib, or dasatinib, or a pharmacologically acceptable salt thereof.

[387] The use according to [374], wherein the kinase inhibitor is an RET inhibitor.

[388] The use according to [387], wherein the RET inhibitor is regorafenib, sorafenib, vandetanib, lenvatinib, or sunitinib, or a pharmacologically acceptable salt thereof.

[389] The use according to [374], wherein the kinase inhibitor is a PDGFR inhibitor.

[390] The use according to [389], wherein the PDGFR inhibitor is regorafenib, sorafenib, sunitinib, axitinib, pazopanib,

lenvatinib, nintedanib, ilorasertib, imatinib, nilotinib, or dasatinib, or a pharmacologically acceptable salt thereof.
[391] The use according to [374], wherein the kinase inhibitor is an FGFR inhibitor.
[392] The use according to [391], wherein the FGFR inhibitor is regorafenib, sorafenib, lenvatinib, nintedanib, axitinib, or pazopanib, or a pharmacologically acceptable salt thereof.
[393] The use according to [374], wherein the kinase inhibitor is an FLT3 inhibitor.
[394] The use according to [393], wherein the FLT3 inhibitor is gilteritinib, quizartinib, midostaurin, sorafenib, ilorasertib, ENMD-2076, or sunitinib, or a pharmacologically acceptable salt thereof.
[395] The use according to [374], wherein the kinase inhibitor is an ALK inhibitor.
[396] The use according to [395], wherein the ALK inhibitor is brigatinib, crizotinib, ceritinib, alectinib, or lorlatinib, or a pharmacologically acceptable salt thereof.
[397] The use according to [374], wherein the kinase inhibitor is a CSF-1R inhibitor.
[398] The use according to [397], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.
[399] The use according to [374], wherein the kinase inhibitor is an EGFR inhibitor.
[400] The use according to [399], wherein the EGFR inhibitor is gefitinib, erlotinib, afatinib, osimertinib, dacomitinib, lapatinib, neratinib, pyrotinib, or poziotinib, or a pharmacologically acceptable salt thereof.
[401] The use according to [374], wherein the kinase inhibitor is an HER2 inhibitor.
[402] The use according to [401], wherein the HER2 inhibitor is tucatinib, neratinib, mubritinib, lapatinib, pyrotinib, or poziotinib, or a pharmacologically acceptable salt thereof.
[403] The use according to any one of [373] to [402], wherein the antibody in the use is an anti-HER2 antibody, an anti-HER3 antibody, an anti-TROP2 antibody, an anti-B7-H3 antibody, or an anti-CDH6 antibody.
[404] The use according to [403], wherein the antibody in the use is an anti-HER2 antibody.
[405] The use according to [404], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.
[406] The use according to [404], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[407] The use according to [404], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[408] The use according to [404], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[409] The use according to any one of [404] to [408], wherein the average number of units of the drug-linker conjugated per antibody molecule in the use is in the range of from 7 to 8.
[410] The use according to [403], wherein the antibody in the use is an anti-HER3 antibody.
[411] The use according to [410], wherein the anti-HER3 antibody is an antibody comprising a heavy chain consisting of the amino acid sequence represented by SEQ ID NO: 3 and a light chain consisting of the amino acid sequence represented by SEQ ID NO: 4.
[412] The use according to [411], wherein the anti-HER3 antibody lacks a lysine residue at the carboxyl terminus of the heavy chain.
[413] The use according to any one of [410] to [412], wherein the average number of units of the drug-linker conjugated per antibody molecule in the use is in the range of from 7 to 8.
[414] The use according to [403], wherein the antibody in the use is an anti-TROP2 antibody.
[415] The use according to [414], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[416] The use according to [415], wherein the anti-TROP2 antibody lacks a lysine residue at the carboxyl terminus of the heavy chain.
[417] The use according to any one of [414] to [416], wherein the average number of units of the drug-linker conjugated per antibody molecule in the use is in the range of from 3.5 to 4.5.
[418] The use according to [403], wherein the antibody in the use is an anti-B7-H3 antibody.

[419] The use according to [418], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[420] The use according to [419], wherein the anti-B7-H3 antibody lacks a lysine residue at the carboxyl terminus of the heavy chain.
[421] The use according to any one of [418] to [420], wherein the average number of units of the drug-linker conjugated per antibody molecule in the use is in the range of from 3.5 to 4.5.
[422] The use according to [403], wherein the antibody in the use is an anti-CDH6 antibody.
[423] The use according to [422], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.
[424] The use according to [423], wherein the anti-CDH6 antibody lacks a lysine residue at the carboxyl terminus of the heavy chain.
[425] The use according to any one of [422] to [424], wherein the average number of units of the drug-linker conjugated per antibody molecule in the use is in the range of from 7 to 8.
[426] The use according to any one of [373] to [425], wherein the antibody-drug conjugate and the kinase inhibitor are separately contained as active components in different formulations, and are administered simultaneously or at different times.
[427] The use according to any one of [373] to [426], wherein the use is for treating at least one selected from the group consisting of breast cancer, gastric cancer, colorectal cancer, lung cancer, esophageal cancer, head-and-neck cancer, gastroespophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.
[428] The use according to [427], wherein the use is for treating breast cancer.
[429] The use according to [427], wherein the use is for treating colorectal cancer.
[430] The use according to [427], wherein the use is for treating gastric cancer.
[431] The use according to [427], wherein the use is for treating lung cancer.
[432] The use according to [427], wherein the use is for treating pancreatic cancer.
[433] The use according to [427], wherein the use is for treating kidney cancer.
[434] The use according to [427], wherein the use is for treating ovarian cancer.
[435] Use of an antibody-drug conjugate for the manufacture of a medicament for treating a disease through being administered in combination with a kinase inhibitor, wherein the antibody-drug conjugate is an antibody-drug conjugate represented by the following formula:

[Formula 8]

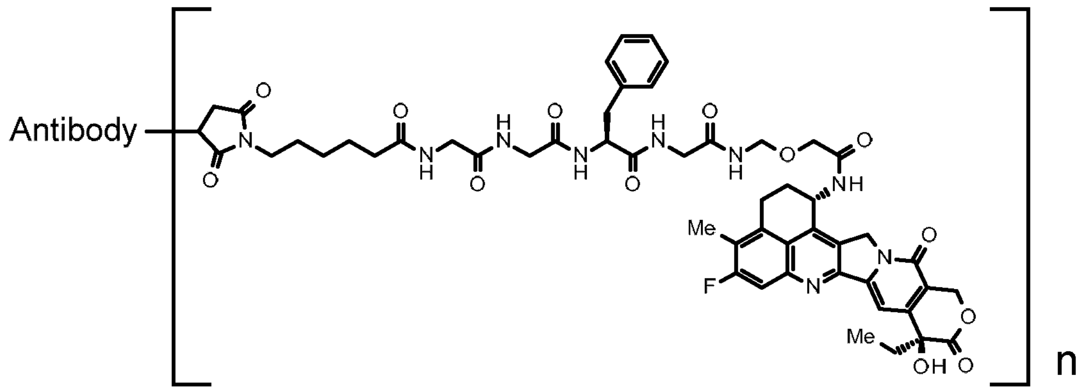

wherein a drug-linker is conjugated to an antibody via a thioether bond, and n is the average number of units of the drug-linker conjugated per antibody molecule, and
the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src

inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[436] The use according to [435], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[437] The use according to [436], wherein the kinase inhibitor is a CDK4/6 inhibitor.

[438] The use according to [437], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[439] The use according to [436], wherein the kinase inhibitor is an mTOR inhibitor.

[440] The use according to [439], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[441] The use according to [436], wherein the kinase inhibitor is a PI3K inhibitor.

[442] The use according to [441], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[443] The use according to [436], wherein the kinase inhibitor is an RAF inhibitor.

[444] The use according to [443], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[445] The use according to [436], wherein the kinase inhibitor is a VEGFR inhibitor.

[446] The use according to [445], wherein the VEGFR inhibitor is regorafenib, sorafenib, vandetanib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[447] The use according to [436], wherein the kinase inhibitor is a KIT inhibitor.

[448] The use according to [447], wherein the KIT inhibitor is regorafenib, sorafenib, imatinib, ilorasertib, sunitinib, pazopanib, lenvatinib, or dasatinib, or a pharmacologically acceptable salt thereof.

[449] The use according to [436], wherein the kinase inhibitor is an RET inhibitor.

[450] The use according to [449], wherein the RET inhibitor is regorafenib, sorafenib, vandetanib, lenvatinib, or sunitinib, or a pharmacologically acceptable salt thereof.

[451] The use according to [436], wherein the kinase inhibitor is a PDGFR inhibitor.

[452] The use according to [451], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.

[453] The use according to [436], wherein the kinase inhibitor is an FGFR inhibitor.

[454] The use according to [453], wherein the FGFR inhibitor is regorafenib, sorafenib, lenvatinib, nintedanib, axitinib, or pazopanib, or a pharmacologically acceptable salt thereof.

[455] The use according to [436], wherein the kinase inhibitor is an FLT3 inhibitor.

[456] The use according to [455], wherein the FLT3 inhibitor is gilteritinib, quizartinib, midostaurin, sorafenib, ilorasertib, ENMD-2076, or sunitinib, or a pharmacologically acceptable salt thereof.

[457] The use according to [436], wherein the kinase inhibitor is an ALK inhibitor.

[458] The use according to [457], wherein the ALK inhibitor is brigatinib, crizotinib, ceritinib, alectinib, or lorlatinib, or a pharmacologically acceptable salt thereof.

[459] The use according to [436], wherein the kinase inhibitor is a CSF-1R inhibitor.

[460] The use according to [459], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.

[461] The use according to [436], wherein the kinase inhibitor is an EGFR inhibitor.

[462] The use according to [461], wherein the EGFR inhibitor is gefitinib, erlotinib, afatinib, osimertinib, dacomitinib, lapatinib, neratinib, pyrotinib, or poziotinib, or a pharmacologically acceptable salt thereof.

[463] The use according to [436], wherein the kinase inhibitor is an HER2 inhibitor.

[464] The use according to [463], wherein the HER2 inhibitor is tucatinib, neratinib, mubritinib, lapatinib, pyrotinib, or poziotinib, or a pharmacologically acceptable salt thereof.

[465] The use according to any one of [435] to [464], wherein the antibody in the antibody-drug conjugate is an anti-HER2 antibody, an anti-HER3 antibody, an anti-TROP2 antibody, an anti-B7-H3 antibody, or an anti-CDH6 antibody.

[466] The use according to [465], wherein the antibody in the antibody-drug conjugate is an anti-HER2 antibody.

[467] The pharmaceutical composition according to [466], wherein the anti-HER2 antibody is an antibody comprising a heavy chain comprising CDRH1 consisting of an amino acid sequence consisting of amino acid residues 26 to

33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[468] The pharmaceutical composition according to [466], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[469] The pharmaceutical composition according to [466], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[470] The use according to [466], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[471] The use according to any one of [466] to [470], wherein the average number of units of the drug-linker conjugated per antibody molecule in the antibody-drug conjugate is in the range of from 7 to 8.

[472] The use according to [465], wherein the antibody in the antibody-drug conjugate is an anti-HER3 antibody.

[473] The use according to [472], wherein the anti-HER3 antibody is an antibody comprising a heavy chain consisting of the amino acid sequence represented by SEQ ID NO: 3 and a light chain consisting of the amino acid sequence represented by SEQ ID NO: 4.

[474] The use according to [473], wherein the anti-HER3 antibody lacks a lysine residue at the carboxyl terminus of the heavy chain.

[475] The use according to any one of [472] to [474], wherein the average number of units of the drug-linker conjugated per antibody molecule in the antibody-drug conjugate is in the range of from 7 to 8.

[476] The use according to [465], wherein the antibody in the antibody-drug conjugate is an anti-TROP2 antibody.

[477] The use according to [476], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[478] The use according to [477], wherein the anti-TROP2 antibody lacks a lysine residue at the carboxyl terminus of the heavy chain.

[479] The use according to any one of [476] to [478], wherein the average number of units of the drug-linker conjugated per antibody molecule in the antibody-drug conjugate is in the range of from 3.5 to 4.5.

[480] The use according to [465], wherein the antibody in the antibody-drug conjugate is an anti-B7-H3 antibody.

[481] The use according to [480], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[482] The use according to [481], wherein the anti-B7-H3 antibody lacks a lysine residue at the carboxyl terminus of the heavy chain.

[483] The use according to any one of [480] to [482], wherein the average number of units of the drug-linker conjugated per antibody molecule in the antibody-drug conjugate is in the range of from 3.5 to 4.5.

[484] The use according to [465], wherein the antibody in the antibody-drug conjugate is an anti-CDH6 antibody.

[485] The use according to [484], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

[486] The use according to [485], wherein the anti-CDH6 antibody lacks a lysine residue at the carboxyl terminus of the heavy chain.

[487] The use according to any one of [484] to [486], wherein the average number of units of the drug-linker conjugated per antibody molecule in the antibody-drug conjugate is in the range of from 7 to 8.

[488] The use according to any one of [435] to [487], wherein the antibody-drug conjugate and the kinase inhibitor are separately contained as active components in different formulations, and are administered simultaneously or at different times.

[489] The use according to any one of [435] to[488], wherein the use is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[490] The use according to [489], wherein the use is for treating breast cancer.

[491] The use according to [489], wherein the use is for treating colorectal cancer.

[492] The use according to [489], wherein the use is for treating gastric cancer.

[493] The use according to [489], wherein the use is for treating lung cancer.

[494] The use according to [489], wherein the use is for treating pancreatic cancer.

[495] The use according to [489], wherein the use is for treating kidney cancer.

[496] The use according to [489], wherein the use is for treating ovarian cancer.

[497] A pharmaceutical composition wherein an antibody-drug conjugate and a kinase inhibitor are administered in combination, and the antibody-drug conjugate is an antibody-drug conjugate in which a drug-linker represented by the following formula:

[Formula 9]

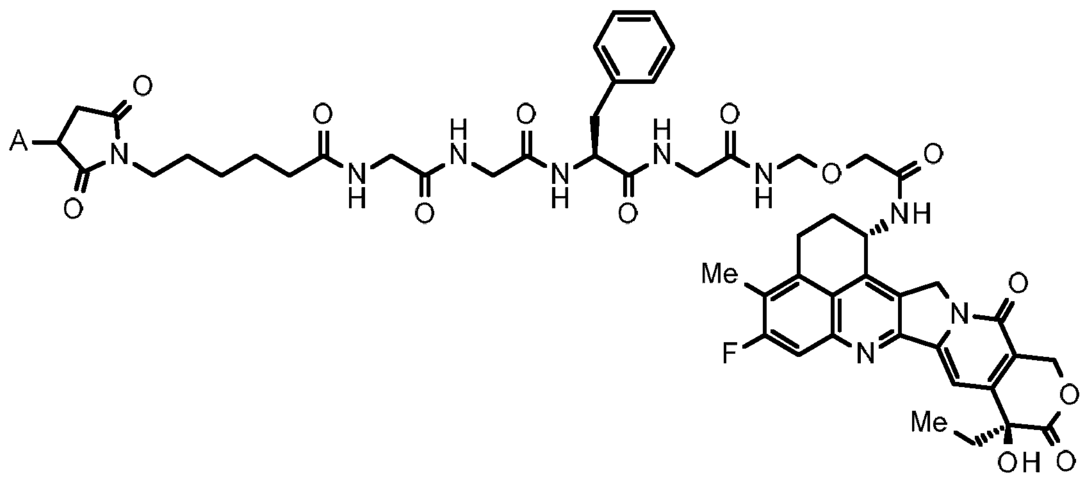

wherein A represents a connecting position to an antibody, is conjugated to the antibody via a thioether bond, and the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.

[498] The pharmaceutical composition according to [497], wherein the kinase inhibitor is abemaciclib or a pharmacologically acceptable salt thereof.

[499] The pharmaceutical composition according to [497], wherein the kinase inhibitor is palbociclib or a pharmacologically acceptable salt thereof.

[500] The pharmaceutical composition according to [497], wherein the kinase inhibitor is everolimus or a pharmacologically acceptable salt thereof.

[501] The pharmaceutical composition according to [497], wherein the kinase inhibitor is taselisib or a pharmacologically acceptable salt thereof.

[502] The pharmaceutical composition according to [497], wherein the kinase inhibitor is alpelisib or a pharmacologically acceptable salt thereof.

[503] The pharmaceutical composition according to [497], wherein the kinase inhibitor is regorafenib or a pharmacologically acceptable salt thereof.

[504] The pharmaceutical composition according to [497], wherein the kinase inhibitor is cabozantinib or a pharmacologically acceptable salt thereof.

[505] The pharmaceutical composition according to [497], wherein the kinase inhibitor is sunitinib or a pharmacologically acceptable salt thereof.

[506] The pharmaceutical composition according to [497], wherein the kinase inhibitor is nintedanib or a pharmacologically acceptable salt thereof.

[507] The pharmaceutical composition according to [497], wherein the kinase inhibitor is brigatinib or a pharmacologically acceptable salt thereof.

[508] The pharmaceutical composition according to [497], wherein the kinase inhibitor is erlotinib or a pharmacologically acceptable salt thereof.

[509] The pharmaceutical composition according to [497], wherein the kinase inhibitor is neratinib or a pharmacologically acceptable salt thereof.

[510] The pharmaceutical composition according to [497], wherein the kinase inhibitor is poziotinib or a pharmacologically acceptable salt thereof.

[511] The pharmaceutical composition according to [497], wherein the kinase inhibitor is tucatinib or a pharmacologically acceptable salt thereof.

[512] The pharmaceutical composition according to [497], wherein the kinase inhibitor is mubritinib or a pharmacologically acceptable salt thereof.

[513] The pharmaceutical composition according to any one of [497] to [512], wherein the antibody in the antibody-drug conjugate is an anti-HER2 antibody, an anti-HER3 antibody, an anti-TROP2 antibody, an anti-B7-H3 antibody, or an anti-CDH6 antibody.

[514] The pharmaceutical composition according to [513], wherein the antibody in the antibody-drug conjugate is an anti-HER2 antibody.

[515] The pharmaceutical composition according to [514], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[516] The pharmaceutical composition according to [514], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[517] The pharmaceutical composition according to [514], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[518] The pharmaceutical composition according to [514], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[519] The pharmaceutical composition according to any one of [514] to [518], wherein the average number of units of the drug-linker conjugated per antibody molecule in the antibody-drug conjugate is in the range of from 7 to 8.

[520] The pharmaceutical composition according to [513], wherein the antibody in the antibody-drug conjugate is an anti-HER3 antibody.

[521] The pharmaceutical composition according to [520], wherein the anti-HER3 antibody is an antibody comprising a heavy chain consisting of the amino acid sequence represented by SEQ ID NO: 3 and a light chain consisting of the amino acid sequence represented by SEQ ID NO: 4.

[522] The pharmaceutical composition according to [521], wherein the anti-HER3 antibody lacks a lysine residue at the carboxyl terminus of the heavy chain.

[523] The pharmaceutical composition according to any one of [520] to [522], wherein the average number of units of the drug-linker conjugated per antibody molecule in the antibody-drug conjugate is in the range of from 7 to 8.

[524] The pharmaceutical composition according to [513], wherein the antibody in the antibody-drug conjugate is an anti-TROP2 antibody.

[525] The pharmaceutical composition according to [524], wherein the anti-TROP2 antibody is an antibody comprising a heavy chain consisting of an amino acid sequence consisting of amino acid residues 20 to 470 of SEQ ID NO: 5

and a light chain consisting of an amino acid sequence consisting of amino acid residues 21 to 234 of SEQ ID NO: 6.
[526] The pharmaceutical composition according to [525], wherein the anti-TROP2 antibody lacks a lysine residue at the carboxyl terminus of the heavy chain.
[527] The pharmaceutical composition according to any one of [524] to [526], wherein the average number of units of the drug-linker conjugated per antibody molecule in the antibody-drug conjugate is in the range of from 3.5 to 4.5.
[528] The pharmaceutical composition according to [513], wherein the antibody in the antibody-drug conjugate is an anti-B7-H3 antibody.
[529] The pharmaceutical composition according to [528], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[530] The pharmaceutical composition according to [529], wherein the anti-B7-H3 antibody lacks a lysine residue at the carboxyl terminus of the heavy chain.
[531] The pharmaceutical composition according to any one of [528] to [530], wherein the average number of units of the drug-linker conjugated per antibody molecule in the antibody-drug conjugate is in the range of from 3.5 to 4.5.
[532] The pharmaceutical composition according to [513], wherein the antibody in the antibody-drug conjugate is an anti-CDH6 antibody.
[533] The pharmaceutical composition according to [532], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.
[534] The pharmaceutical composition according to [533], wherein the anti-CDH6 antibody lacks a lysine residue at the carboxyl terminus of the heavy chain.
[535] The pharmaceutical composition according to any one of [532] to [534], wherein the average number of units of the drug-linker conjugated per antibody molecule in the antibody-drug conjugate is in the range of from 7 to 8.
[536] The pharmaceutical composition according to any one of [497] to [535], wherein the antibody-drug conjugate and the kinase inhibitor are separately contained as active components in different formulations, and are administered simultaneously or at different times.
[537] The pharmaceutical composition according to any one of [497] to [536], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.
[538] The pharmaceutical composition according to [537], wherein the pharmaceutical composition is for use in treating breast cancer.
[539] The pharmaceutical composition according to [537], wherein the pharmaceutical composition is for use in treating colorectal cancer.
[540] The pharmaceutical composition according to [537], wherein the pharmaceutical composition is for use in treating gastric cancer.
[541] The pharmaceutical composition according to [537], wherein the pharmaceutical composition is for use in treating lung cancer.
[542] The pharmaceutical composition according to [537], wherein the pharmaceutical composition is for use in treating pancreatic cancer.
[543] The pharmaceutical composition according to [537], wherein the pharmaceutical composition is for use in treating kidney cancer.
[544] The pharmaceutical composition according to [537], wherein the pharmaceutical composition is for use in treating ovarian cancer.
[545] A pharmaceutical composition wherein an antibody-drug conjugate and a kinase inhibitor are administered in combination, and the antibody-drug conjugate is an antibody-drug conjugate represented by the following formula:

[Formula 10]

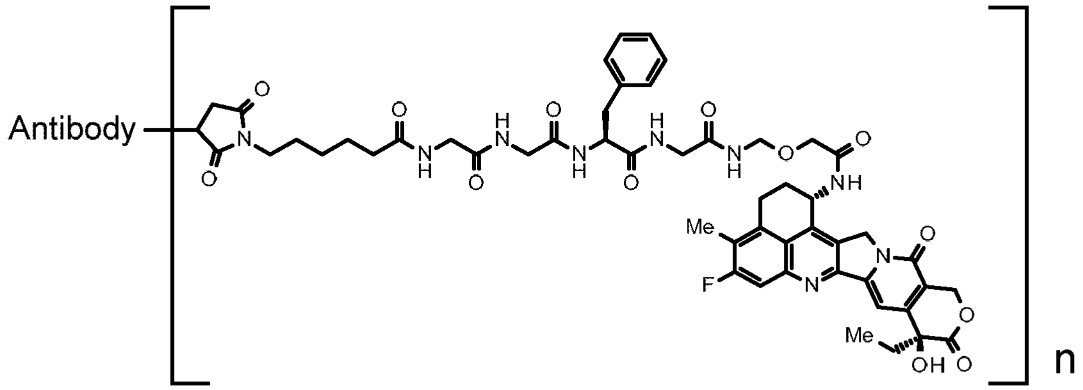

wherein a drug-linker is conjugated to an antibody via a thioether bond, and n is the average number of units of the drug-linker conjugated per antibody molecule, and
the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.

[546] The pharmaceutical composition according to [545], wherein the kinase inhibitor is abemaciclib or a pharmacologically acceptable salt thereof.

[547] The pharmaceutical composition according to [545], wherein the kinase inhibitor is palbociclib or a pharmacologically acceptable salt thereof.

[548] The pharmaceutical composition according to [545], wherein the kinase inhibitor is everolimus or a pharmacologically acceptable salt thereof.

[549] The pharmaceutical composition according to [545], wherein the kinase inhibitor is taselisib or a pharmacologically acceptable salt thereof.

[550] The pharmaceutical composition according to [545], wherein the kinase inhibitor is alpelisib or a pharmacologically acceptable salt thereof.

[551] The pharmaceutical composition according to [545], wherein the kinase inhibitor is regorafenib or a pharmacologically acceptable salt thereof.

[552] The pharmaceutical composition according to [545], wherein the kinase inhibitor is cabozantinib or a pharmacologically acceptable salt thereof.

[553] The pharmaceutical composition according to [545], wherein the kinase inhibitor is sunitinib or a pharmacologically acceptable salt thereof.

[554] The pharmaceutical composition according to [545], wherein the kinase inhibitor is nintedanib or a pharmacologically acceptable salt thereof.

[555] The pharmaceutical composition according to [545], wherein the kinase inhibitor is brigatinib or a pharmacologically acceptable salt thereof.

[556] The pharmaceutical composition according to [545], wherein the kinase inhibitor is erlotinib or a pharmacologically acceptable salt thereof.

[557] The pharmaceutical composition according to [545], wherein the kinase inhibitor is neratinib or a pharmacologically acceptable salt thereof.

[558] The pharmaceutical composition according to [545], wherein the kinase inhibitor is poziotinib or a pharmacologically acceptable salt thereof.
[559] The pharmaceutical composition according to [545], wherein the kinase inhibitor is tucatinib or a pharmacologically acceptable salt thereof.
[560] The pharmaceutical composition according to [545], wherein the kinase inhibitor is mubritinib or a pharmacologically acceptable salt thereof.
[561] The pharmaceutical composition according to any one of [545] to [560], wherein the antibody in the antibody-drug conjugate is an anti-HER2 antibody, an anti-HER3 antibody, an anti-TROP2 antibody, an anti-B7-H3 antibody, or an anti-CDH6 antibody.
[562] The pharmaceutical composition according to [561], wherein the antibody in the antibody-drug conjugate is an anti-HER2 antibody.
[563] The pharmaceutical composition according to [562], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.
[564] The pharmaceutical composition according to [562], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[565] The pharmaceutical composition according to [562], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[566] The pharmaceutical composition according to [562], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[567] The pharmaceutical composition according to any one of [562] to [566], wherein the average number of units of the drug-linker conjugated per antibody molecule in the antibody-drug conjugate is in the range of from 7 to 8.
[568] The pharmaceutical composition according to [561], wherein the antibody in the antibody-drug conjugate is an anti-HER3 antibody.
[569] The pharmaceutical composition according to [568], wherein the anti-HER3 antibody is an antibody comprising a heavy chain consisting of the amino acid sequence represented by SEQ ID NO: 3 and a light chain consisting of the amino acid sequence represented by SEQ ID NO: 4.
[570] The pharmaceutical composition according to [569], wherein the anti-HER3 antibody lacks a lysine residue at the carboxyl terminus of the heavy chain.
[571] The pharmaceutical composition according to any one of [568] to [570], wherein the average number of units of the drug-linker conjugated per antibody molecule in the antibody-drug conjugate is in the range of from 7 to 8.
[572] The pharmaceutical composition according to [561], wherein the antibody in the antibody-drug conjugate is an anti-TROP2 antibody.
[573] The pharmaceutical composition according to [572], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[574] The pharmaceutical composition according to [573], wherein the anti-TROP2 antibody lacks a lysine residue at the carboxyl terminus of the heavy chain.
[575] The pharmaceutical composition according to any one of [572] to [574], wherein the average number of units of the drug-linker conjugated per antibody molecule in the antibody-drug conjugate is in the range of from 3.5 to 4.5.
[576] The pharmaceutical composition according to [561], wherein the antibody in the antibody-drug conjugate is an anti-B7-H3 antibody.
[577] The pharmaceutical composition according to [576], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[578] The pharmaceutical composition according to [577], wherein the anti-B7-H3 antibody lacks a lysine residue at the carboxyl terminus of the heavy chain.
[579] The pharmaceutical composition according to any one of [576] to [578], wherein the average number of units of the drug-linker conjugated per antibody molecule in the antibody-drug conjugate is in the range of from 3.5 to 4.5.
[580] The pharmaceutical composition according to [561], wherein the antibody in the antibody-drug conjugate is

an anti-CDH6 antibody.
[581] The pharmaceutical composition according to [580], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.
[582] The pharmaceutical composition according to [581], wherein the anti-CDH6 antibody lacks a lysine residue at the carboxyl terminus of the heavy chain.
[583] The pharmaceutical composition according to any one of [580] to [582], wherein the average number of units of the drug-linker conjugated per antibody molecule in the antibody-drug conjugate is in the range of from 7 to 8.
[584] The pharmaceutical composition according to any one of [545] to [583], wherein the antibody-drug conjugate and the kinase inhibitor are separately contained as active components in different formulations, and are administered simultaneously or at different times.
[585] The pharmaceutical composition according to any one of [545] to [584], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.
[586] The pharmaceutical composition according to [585], wherein the pharmaceutical composition is for use in treating breast cancer.
[587] The pharmaceutical composition according to [585], wherein the pharmaceutical composition is for use in treating colorectal cancer.
[588] The pharmaceutical composition according to [585], wherein the pharmaceutical composition is for use in treating gastric cancer.
[589] The pharmaceutical composition according to [585], wherein the pharmaceutical composition is for use in treating lung cancer.
[590] The pharmaceutical composition according to [585], wherein the pharmaceutical composition is for use in treating pancreatic cancer.
[591] The pharmaceutical composition according to [585], wherein the pharmaceutical composition is for use in treating kidney cancer.
[592] The pharmaceutical composition according to [585], wherein the pharmaceutical composition is for use in treating ovarian cancer.
[593] A method of treatment, comprising administering an antibody-drug conjugate and a kinase inhibitor in combination to a subject in need of treatment, wherein the antibody-drug conjugate is an antibody-drug conjugate in which a drug-linker represented by the following formula:

[Formula 11]

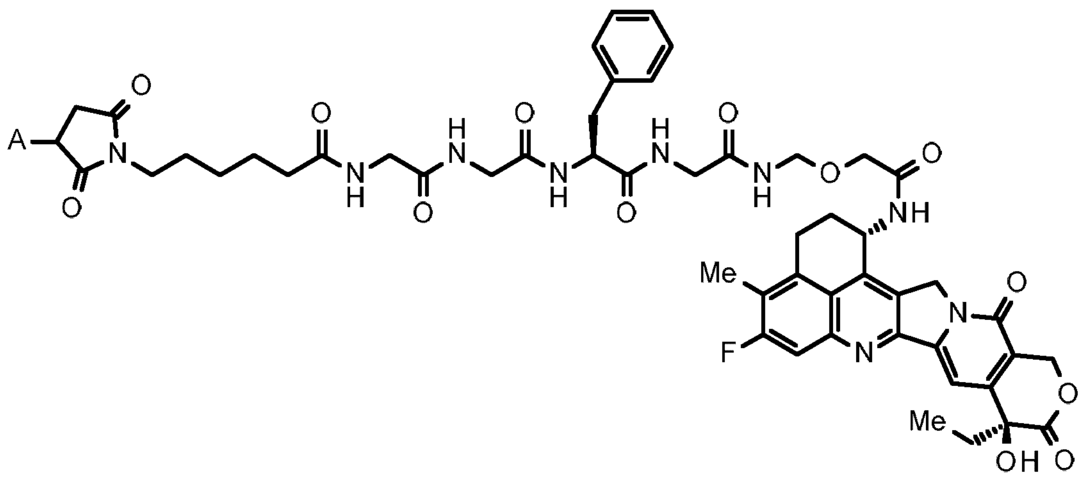

wherein A represents a connecting position to an antibody, is conjugated to the antibody via a thioether bond, and

the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.

[594] The method of treatment according to [593], wherein the kinase inhibitor is abemaciclib or a pharmacologically acceptable salt thereof.

[595] The method of treatment according to [593], wherein the kinase inhibitor is palbociclib or a pharmacologically acceptable salt thereof.

[596] The method of treatment according to [593], wherein the kinase inhibitor is everolimus or a pharmacologically acceptable salt thereof.

[597] The method of treatment according to [593], wherein the kinase inhibitor is taselisib or a pharmacologically acceptable salt thereof.

[598] The method of treatment according to [593], wherein the kinase inhibitor is alpelisib or a pharmacologically acceptable salt thereof.

[599] The method of treatment according to [593], wherein the kinase inhibitor is regorafenib or a pharmacologically acceptable salt thereof.

[600] The method of treatment according to [593], wherein the kinase inhibitor is cabozantinib or a pharmacologically acceptable salt thereof.

[601] The method of treatment according to [593], wherein the kinase inhibitor is sunitinib or a pharmacologically acceptable salt thereof.

[602] The method of treatment according to [593], wherein the kinase inhibitor is nintedanib or a pharmacologically acceptable salt thereof.

[603] The method of treatment according to [593], wherein the kinase inhibitor is brigatinib or a pharmacologically acceptable salt thereof.

[604] The method of treatment according to [593], wherein the kinase inhibitor is erlotinib or a pharmacologically acceptable salt thereof.

[605] The method of treatment according to [593], wherein the kinase inhibitor is neratinib or a pharmacologically acceptable salt thereof.

[606] The method of treatment according to [593], wherein the kinase inhibitor is poziotinib or a pharmacologically acceptable salt thereof.

[607] The method of treatment according to [593], wherein the kinase inhibitor is tucatinib or a pharmacologically acceptable salt thereof.

[608] The method of treatment according to [593], wherein the kinase inhibitor is mubritinib or a pharmacologically acceptable salt thereof.

[609] The method of treatment according to any one of [593] to [608], wherein the antibody in the antibody-drug conjugate is an anti-HER2 antibody, an anti-HER3 antibody, an anti-TROP2 antibody, an anti-B7-H3 antibody, or an anti-CDH6 antibody.

[610] The method of treatment according to [609], wherein the antibody in the antibody-drug conjugate is an anti-HER2 antibody.

[611] The method of treatment according to [610], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[612] The method of treatment according to [610], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[613] The method of treatment according to [610], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[614] The method of treatment according to [610], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[615] The method of treatment according to any one of [610] to [614], wherein the average number of units of the drug-linker conjugated per antibody molecule in the antibody-drug conjugate is in the range of from 7 to 8.

[616] The method of treatment according to [609], wherein the antibody in the antibody-drug conjugate is an anti-HER3 antibody.

[617] The method of treatment according to [616], wherein the anti-HER3 antibody is an antibody comprising a heavy chain consisting of the amino acid sequence represented by SEQ ID NO: 3 and a light chain consisting of the amino acid sequence represented by SEQ ID NO: 4.

[618] The method of treatment according to [617], wherein the anti-HER3 antibody lacks a lysine residue at the carboxyl terminus of the heavy chain.

[619] The method of treatment according to any one of [616] to [618], wherein the average number of units of the drug-linker conjugated per antibody molecule in the antibody-drug conjugate is in the range of from 7 to 8.

[620] The method of treatment according to [609], wherein the antibody in the antibody-drug conjugate is an anti-TROP2 antibody.

[621] The method of treatment according to [620], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[622] The method of treatment according to [621], wherein the anti-TROP2 antibody lacks a lysine residue at the carboxyl terminus of the heavy chain.

[623] The method of treatment according to any one of [620] to [622], wherein the average number of units of the drug-linker conjugated per antibody molecule in the antibody-drug conjugate is in the range of from 3.5 to 4.5.

[624] The method of treatment according to [609], wherein the antibody in the antibody-drug conjugate is an anti-B7-H3 antibody.

[625] The method of treatment according to [624], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[626] The method of treatment according to [625], wherein the anti-B7-H3 antibody lacks a lysine residue at the carboxyl terminus of the heavy chain.

[627] The method of treatment according to any one of [624] to [626], wherein the average number of units of the drug-linker conjugated per antibody molecule in the antibody-drug conjugate is in the range of from 3.5 to 4.5.

[628] The method of treatment according to [609], wherein the antibody in the antibody-drug conjugate is an anti-CDH6 antibody.

[629] The method of treatment according to [628], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

[630] The method of treatment according to [629], wherein the anti-CDH6 antibody lacks a lysine residue at the carboxyl terminus of the heavy chain.

[631] The method of treatment according to any one of [628] to [630], wherein the average number of units of the drug-linker conjugated per antibody molecule in the antibody-drug conjugate is in the range of from 7 to 8.

[632] The method of treatment according to any one of [593] to [631], wherein the antibody-drug conjugate and the kinase inhibitor are separately contained as active components in different formulations, and are administered simultaneously or at different times.

[633] The pharmaceutical composition according to any one of [593] to [632], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.
[634] The method of treatment according to [633], wherein the method of treatment is for treating breast cancer.
[635] The method of treatment according to [633], wherein the method of treatment is for treating colorectal cancer.
[636] The method of treatment according to [633], wherein the method of treatment is for treating gastric cancer.
[637] The method of treatment according to [633], wherein the method of treatment is for treating lung cancer.
[638] The method of treatment according to [633], wherein the method of treatment is for treating pancreatic cancer.
[639] The method of treatment according to [633], wherein the method of treatment is for treating kidney cancer.
[640] The method of treatment according to [633], wherein the method of treatment is for treating ovarian cancer.
[641] A method of treatment, comprising administering an antibody-drug conjugate and a kinase inhibitor in combination to a subject in need of treatment, wherein the antibody-drug conjugate is an antibody-drug conjugate represented by the following formula:

[Formula 12]

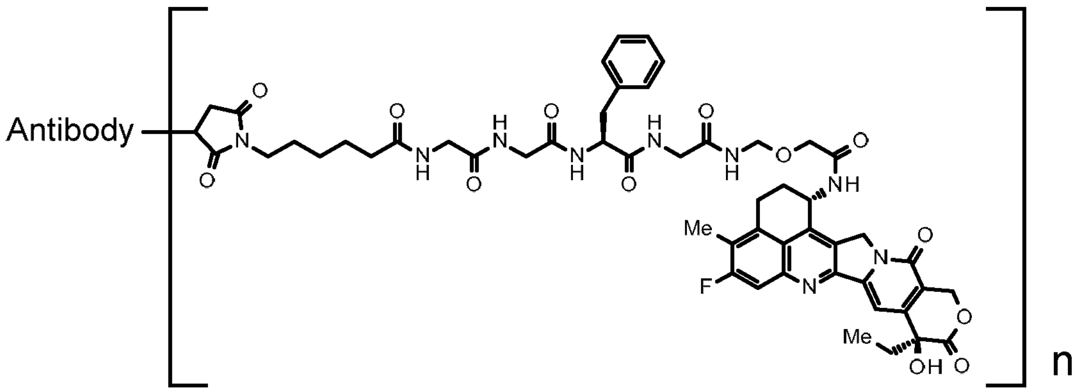

wherein a drug-linker is conjugated to an antibody via a thioether bond, and n is the average number of units of the drug-linker conjugated per antibody molecule, and
the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.
[642] The method of treatment according to [641], wherein the kinase inhibitor is abemaciclib or a pharmacologically acceptable salt thereof.
[643] The method of treatment according to [641], wherein the kinase inhibitor is palbociclib or a pharmacologically acceptable salt thereof.
[644] The method of treatment according to [641], wherein the kinase inhibitor is everolimus or a pharmacologically acceptable salt thereof.
[645] The method of treatment according to [641], wherein the kinase inhibitor is taselisib or a pharmacologically acceptable salt thereof.
[646] The method of treatment according to [641], wherein the kinase inhibitor is alpelisib or a pharmacologically acceptable salt thereof.
[647] The method of treatment according to [641], wherein the kinase inhibitor is regorafenib or a pharmacologically acceptable salt thereof.
[648] The method of treatment according to [641], wherein the kinase inhibitor is cabozantinib or a pharmacologically

acceptable salt thereof.

[649] The method of treatment according to [641], wherein the kinase inhibitor is sunitinib or a pharmacologically acceptable salt thereof.

[650] The method of treatment according to [641], wherein the kinase inhibitor is nintedanib or a pharmacologically acceptable salt thereof.

[651] The method of treatment according to [641], wherein the kinase inhibitor is brigatinib or a pharmacologically acceptable salt thereof.

[652] The method of treatment according to [641], wherein the kinase inhibitor is erlotinib or a pharmacologically acceptable salt thereof.

[653] The method of treatment according to [641], wherein the kinase inhibitor is neratinib or a pharmacologically acceptable salt thereof.

[654] The method of treatment according to [641], wherein the kinase inhibitor is poziotinib or a pharmacologically acceptable salt thereof.

[655] The method of treatment according to [641], wherein the kinase inhibitor is tucatinib or a pharmacologically acceptable salt thereof.

[656] The method of treatment according to [641], wherein the kinase inhibitor is mubritinib or a pharmacologically acceptable salt thereof.

[657] The method of treatment according to any one of [641] to [656], wherein the antibody in the antibody-drug conjugate is an anti-HER2 antibody, an anti-HER3 antibody, an anti-TROP2 antibody, an anti-B7-H3 antibody, or an anti-CDH6 antibody.

[658] The method of treatment according to [657], wherein the antibody in the antibody-drug conjugate is an anti-HER2 antibody.

[659] The method of treatment according to [658], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[660] The method of treatment according to [658], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[661] The method of treatment according to [658], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[662] The method of treatment according to [658], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[663] The method of treatment according to any one of [658] to [662], wherein the average number of units of the drug-linker conjugated per antibody molecule in the antibody-drug conjugate is in the range of from 7 to 8.

[664] The method of treatment according to [657], wherein the antibody in the antibody-drug conjugate is an anti-HER3 antibody.

[665] The method of treatment according to [664], wherein the anti-HER3 antibody is an antibody comprising a heavy chain consisting of the amino acid sequence represented by SEQ ID NO: 3 and a light chain consisting of the amino acid sequence represented by SEQ ID NO: 4.

[666] The method of treatment according to [665], wherein the anti-HER3 antibody lacks a lysine residue at the carboxyl terminus of the heavy chain.

[667] The method of treatment according to any one of [664] to [666], wherein the average number of units of the drug-linker conjugated per antibody molecule in the antibody-drug conjugate is in the range of from 7 to 8.

[668] The method of treatment according to [657], wherein the antibody in the antibody-drug conjugate is an anti-TROP2 antibody.

[669] The method of treatment according to [668], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[670] The method of treatment according to [669], wherein the anti-TROP2 antibody lacks a lysine residue at the carboxyl terminus of the heavy chain.

[671] The method of treatment according to any one of [668] to [670], wherein the average number of units of the drug-linker conjugated per antibody molecule in the antibody-drug conjugate is in the range of from 3.5 to 4.5.

[672] The method of treatment according to [657], wherein the antibody in the antibody-drug conjugate is an anti-B7-H3 antibody.

[673] The method of treatment according to [672], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[674] The method of treatment according to [673], wherein the anti-B7-H3 antibody lacks a lysine residue at the carboxyl terminus of the heavy chain.

[675] The method of treatment according to any one of [672] to [674], wherein the average number of units of the drug-linker conjugated per antibody molecule in the antibody-drug conjugate is in the range of from 3.5 to 4.5.

[676] The method of treatment according to [657], wherein the antibody in the antibody-drug conjugate is an anti-CDH6 antibody.

[677] The method of treatment according to [676], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

[678] The method of treatment according to [677], wherein the anti-CDH6 antibody lacks a lysine residue at the carboxyl terminus of the heavy chain.

[679] The method of treatment according to any one of [676] to [678], wherein the average number of units of the drug-linker conjugated per antibody molecule in the antibody-drug conjugate is in the range of from 7 to 8.

[680] The method of treatment according to any one of [641] to [679], wherein the antibody-drug conjugate and the kinase inhibitor are separately contained as active components in different formulations, and are administered simultaneously or at different times.

[681] The method of treatment according to any one of [641] to [680], wherein the method of treatment is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[682] The method of treatment according to [681], wherein the method of treatment is for treating breast cancer.

[683] The method of treatment according to [681], wherein the method of treatment is for treating colorectal cancer.

[684] The method of treatment according to [681], wherein the method of treatment is for treating gastric cancer.

[685] The method of treatment according to [681], wherein the method of treatment is for treating lung cancer.

[686] The method of treatment according to [681], wherein the method of treatment is for treating pancreatic cancer.

[687] The method of treatment according to [681], wherein the method of treatment is for treating kidney cancer.

[688] The method of treatment according to [681], wherein the method of treatment is for treating ovarian cancer.

[689] An antibody-drug conjugate for use in treating a disease through being administered in combination with a kinase inhibitor, wherein a drug-linker represented by the following formula:

[Formula 13]

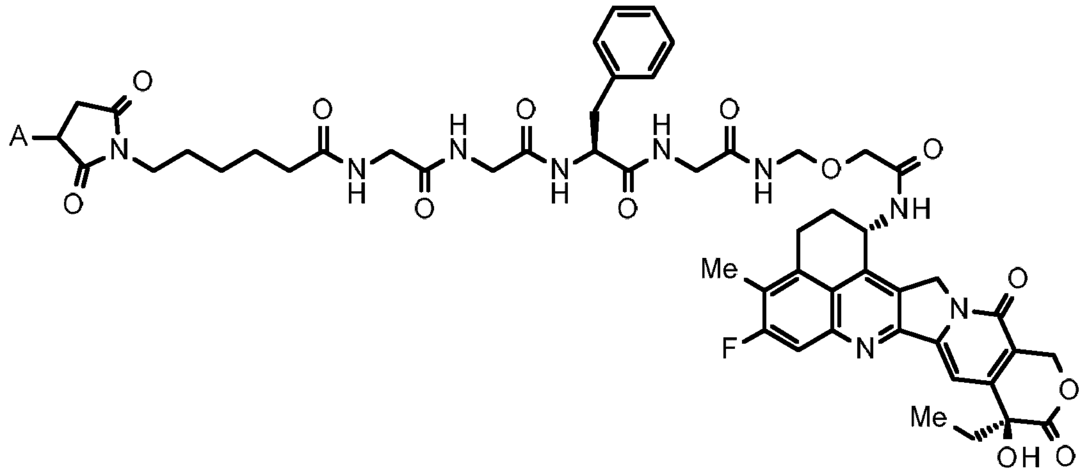

wherein A represents a connecting position to an antibody, is conjugated to the antibody via a thioether bond in the antibody-drug conjugate, and

the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.

[690] The antibody-drug conjugate according to [689], wherein the kinase inhibitor is abemaciclib or a pharmacologically acceptable salt thereof.

[691] The antibody-drug conjugate according to [689], wherein the kinase inhibitor is palbociclib or a pharmacologically acceptable salt thereof.

[692] The antibody-drug conjugate according to [689], wherein the kinase inhibitor is everolimus or a pharmacologically acceptable salt thereof.

[693] The antibody-drug conjugate according to [689], wherein the kinase inhibitor is taselisib or a pharmacologically acceptable salt thereof.

[694] The antibody-drug conjugate according to [689], wherein the kinase inhibitor is alpelisib or a pharmacologically acceptable salt thereof.

[695] The antibody-drug conjugate according to [689], wherein the kinase inhibitor is regorafenib or a pharmacologically acceptable salt thereof.

[696] The antibody-drug conjugate according to [689], wherein the kinase inhibitor is cabozantinib or a pharmacologically acceptable salt thereof.

[697] The antibody-drug conjugate according to [689], wherein the kinase inhibitor is sunitinib or a pharmacologically acceptable salt thereof.

[698] The antibody-drug conjugate according to [689], wherein the kinase inhibitor is nintedanib or a pharmacologically acceptable salt thereof.

[699] The antibody-drug conjugate according to [689], wherein the kinase inhibitor is brigatinib or a pharmacologically acceptable salt thereof.

[700] The antibody-drug conjugate according to [689], wherein the kinase inhibitor is erlotinib or a pharmacologically acceptable salt thereof.

[701] The antibody-drug conjugate according to [689], wherein the kinase inhibitor is neratinib or a pharmacologically acceptable salt thereof.

[702] The antibody-drug conjugate according to [689], wherein the kinase inhibitor is poziotinib or a pharmacologically acceptable salt thereof.

[703] The antibody-drug conjugate according to [689], wherein the kinase inhibitor is tucatinib or a pharmacologically acceptable salt thereof.

[704] The antibody-drug conjugate according to [689], wherein the kinase inhibitor is mubritinib or a pharmacologically acceptable salt thereof.

[705] The antibody-drug conjugate according to any one of [689] to [704], wherein the antibody in the antibody-drug conjugate is an anti-HER2 antibody, an anti-HER3 antibody, an anti-TROP2 antibody, an anti-B7-H3 antibody, or an anti-CDH6 antibody.

[706] The antibody-drug conjugate according to [705], wherein the antibody in the antibody-drug conjugate is an anti-HER2 antibody.

[707] The antibody-drug conjugate according to [706], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[708] The antibody-drug conjugate according to [706], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[709] The antibody-drug conjugate according to [706], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[710] The antibody-drug conjugate according to [706], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[711] The antibody-drug conjugate according to any one of [706] to [710], wherein the average number of units of the drug-linker conjugated per antibody molecule in the antibody-drug conjugate is in the range of from 7 to 8.

[712] The antibody-drug conjugate according to [705], wherein the antibody in the antibody-drug conjugate is an anti-HER3 antibody.

[713] The antibody-drug conjugate according to [712], wherein the anti-HER3 antibody is an antibody comprising a heavy chain consisting of the amino acid sequence represented by SEQ ID NO: 3 and a light chain consisting of the amino acid sequence represented by SEQ ID NO: 4.

[714] The antibody-drug conjugate according to [713], wherein the anti-HER3 antibody lacks a lysine residue at the carboxyl terminus of the heavy chain.

[715] The antibody-drug conjugate according to any one of [712] to [714], wherein the average number of units of the drug-linker conjugated per antibody molecule in the antibody-drug conjugate is in the range of from 7 to 8.

[716] The antibody-drug conjugate according to [705], wherein the antibody in the antibody-drug conjugate is an anti-TROP2 antibody.

[717] The antibody-drug conjugate according to [716], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[718] The antibody-drug conjugate according to [717], wherein the anti-TROP2 antibody lacks a lysine residue at the carboxyl terminus of the heavy chain.

[719] The antibody-drug conjugate according to any one of [716] to [718], wherein the average number of units of the drug-linker conjugated per antibody molecule in the antibody-drug conjugate is in the range of from 3.5 to 4.5.

[720] The antibody-drug conjugate according to [705], wherein the antibody in the antibody-drug conjugate is an anti-B7-H3 antibody.

[721] The antibody-drug conjugate according to [720], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[722] The antibody-drug conjugate according to [721], wherein the anti-B7-H3 antibody lacks a lysine residue at the carboxyl terminus of the heavy chain.

[723] The antibody-drug conjugate according to any one of [720] to [722], wherein the average number of units of the drug-linker conjugated per antibody molecule in the antibody-drug conjugate is in the range of from 3.5 to 4.5.
[724] The antibody-drug conjugate according to [705], wherein the antibody in the antibody-drug conjugate is an anti-CDH6 antibody.
[725] The antibody-drug conjugate according to [724], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.
[726] The antibody-drug conjugate according to [725], wherein the anti-CDH6 antibody lacks a lysine residue at the carboxyl terminus of the heavy chain.
[727] The antibody-drug conjugate according to any one of [724] to [726], wherein the average number of units of the drug-linker conjugated per antibody molecule in the antibody-drug conjugate is in the range of from 7 to 8.
[728] The antibody-drug conjugate according to any one of [689] to [727], wherein the antibody-drug conjugate and the kinase inhibitor are separately contained as active components in different formulations, and are administered simultaneously or at different times.
[729] The antibody-drug conjugate according to any one of [689] to [728], wherein the antibody-drug conjugate is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.
[730] The antibody-drug conjugate according to [729], wherein the antibody-drug conjugate is for use in treating breast cancer.
[731] The antibody-drug conjugate according to [729], wherein the antibody-drug conjugate is for use in treating colorectal cancer.
[732] The antibody-drug conjugate according to [729], wherein the antibody-drug conjugate is for use in treating gastric cancer.
[733] The antibody-drug conjugate according to [729], wherein the antibody-drug conjugate is for use in treating lung cancer.
[734] The antibody-drug conjugate according to [729], wherein the antibody-drug conjugate is for use in treating pancreatic cancer.
[735] The antibody-drug conjugate according to [729], wherein the antibody-drug conjugate is for use in treating kidney cancer.
[736] The antibody-drug conjugate according to [729], wherein the antibody-drug conjugate is for use in treating ovarian cancer.
[737] An antibody-drug conjugate for use in treating a disease through being administered in combination with a kinase inhibitor, wherein the antibody-drug conjugate is an antibody-drug conjugate represented by the following formula:

[Formula 14]

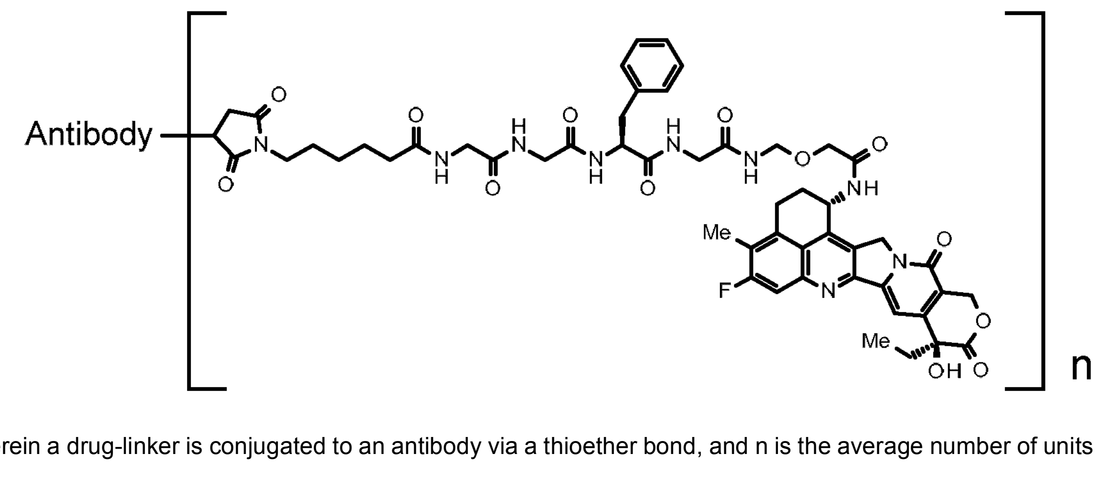

wherein a drug-linker is conjugated to an antibody via a thioether bond, and n is the average number of units of the

drug-linker conjugated per antibody molecule, and

the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.

[738] The antibody-drug conjugate according to [737], wherein the kinase inhibitor is abemaciclib or a pharmacologically acceptable salt thereof.

[739] The antibody-drug conjugate according to [737], wherein the kinase inhibitor is palbociclib or a pharmacologically acceptable salt thereof.

[740] The antibody-drug conjugate according to [737], wherein the kinase inhibitor is everolimus or a pharmacologically acceptable salt thereof.

[741] The antibody-drug conjugate according to [737], wherein the kinase inhibitor is taselisib or a pharmacologically acceptable salt thereof.

[742] The antibody-drug conjugate according to [737], wherein the kinase inhibitor is alpelisib or a pharmacologically acceptable salt thereof.

[743] The antibody-drug conjugate according to [737], wherein the kinase inhibitor is regorafenib or a pharmacologically acceptable salt thereof.

[744] The antibody-drug conjugate according to [737], wherein the kinase inhibitor is cabozantinib or a pharmacologically acceptable salt thereof.

[745] The antibody-drug conjugate according to [737], wherein the kinase inhibitor is sunitinib or a pharmacologically acceptable salt thereof.

[746] The antibody-drug conjugate according to [737], wherein the kinase inhibitor is nintedanib or a pharmacologically acceptable salt thereof.

[747] The antibody-drug conjugate according to [737], wherein the kinase inhibitor is brigatinib or a pharmacologically acceptable salt thereof.

[748] The antibody-drug conjugate according to [737], wherein the kinase inhibitor is erlotinib or a pharmacologically acceptable salt thereof.

[749] The antibody-drug conjugate according to [737], wherein the kinase inhibitor is neratinib or a pharmacologically acceptable salt thereof.

[750] The antibody-drug conjugate according to [737], wherein the kinase inhibitor is poziotinib or a pharmacologically acceptable salt thereof.

[751] The antibody-drug conjugate according to [737], wherein the kinase inhibitor is tucatinib or a pharmacologically acceptable salt thereof.

[752] The antibody-drug conjugate according to [737], wherein the kinase inhibitor is mubritinib or a pharmacologically acceptable salt thereof.

[753] The antibody-drug conjugate according to any one of [737] to [752], wherein the antibody in the antibody-drug conjugate is an anti-HER2 antibody, an anti-HER3 antibody, an anti-TROP2 antibody, an anti-B7-H3 antibody, or an anti-CDH6 antibody.

[754] The antibody-drug conjugate according to [753], wherein the antibody in the antibody-drug conjugate is an anti-HER2 antibody.

[755] The antibody-drug conjugate according to [754], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89

to 97 of SEQ ID NO: 2.

[756] The antibody-drug conjugate according to [754], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[757] The antibody-drug conjugate according to [754], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[758] The antibody-drug conjugate according to [754], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[759] The antibody-drug conjugate according to any one of [754] to [758], wherein the average number of units of the drug-linker conjugated per antibody molecule in the antibody-drug conjugate is in the range of from 7 to 8.

[760] The antibody-drug conjugate according to [753], wherein the antibody in the antibody-drug conjugate is an anti-HER3 antibody.

[761] The antibody-drug conjugate according to [760], wherein the anti-HER3 antibody is an antibody comprising a heavy chain consisting of the amino acid sequence represented by SEQ ID NO: 3 and a light chain consisting of the amino acid sequence represented by SEQ ID NO: 4.

[762] The antibody-drug conjugate according to [761], wherein the anti-HER3 antibody lacks a lysine residue at the carboxyl terminus of the heavy chain.

[763] The antibody-drug conjugate according to any one of [760] to [762], wherein the average number of units of the drug-linker conjugated per antibody molecule in the antibody-drug conjugate is in the range of from 7 to 8.

[764] The antibody-drug conjugate according to [753], wherein the antibody in the antibody-drug conjugate is an anti-TROP2 antibody.

[765] The antibody-drug conjugate according to [764], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[766] The antibody-drug conjugate according to [765], wherein the anti-TROP2 antibody lacks a lysine residue at the carboxyl terminus of the heavy chain.

[767] The antibody-drug conjugate according to any one of [764] to [766], wherein the average number of units of the drug-linker conjugated per antibody molecule in the antibody-drug conjugate is in the range of from 3.5 to 4.5.

[768] The antibody-drug conjugate according to [753], wherein the antibody in the antibody-drug conjugate is an anti-B7-H3 antibody.

[769] The antibody-drug conjugate according to [768], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[770] The antibody-drug conjugate according to [769], wherein the anti-B7-H3 antibody lacks a lysine residue at the carboxyl terminus of the heavy chain.

[771] The antibody-drug conjugate according to any one of [768] to [770], wherein the average number of units of the drug-linker conjugated per antibody molecule in the antibody-drug conjugate is in the range of from 3.5 to 4.5.

[772] The antibody-drug conjugate according to [753], wherein the antibody in the antibody-drug conjugate is an anti-CDH6 antibody.

[773] The antibody-drug conjugate according to [772], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

[774] The antibody-drug conjugate according to [773], wherein the anti-CDH6 antibody lacks a lysine residue at the carboxyl terminus of the heavy chain.

[775] The antibody-drug conjugate according to any one of [772] to [774], wherein the average number of units of the drug-linker conjugated per antibody molecule in the antibody-drug conjugate is in the range of from 7 to 8.

[776] The antibody-drug conjugate according to any one of [737] to [775], wherein the antibody-drug conjugate and the kinase inhibitor are separately contained as active components in different formulations, and are administered simultaneously or at different times.

[777] The antibody-drug conjugate according to any one of [737] to [776], wherein the antibody-drug conjugate is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis

cancer, leukemia, malignant lymphoma, plasmacytoma, myeloma, glioblastoma multiforme, osteosarcoma, sarcoma, and melanoma.

[778] The antibody-drug conjugate according to [777], wherein the antibody-drug conjugate is for use in treating breast cancer.

[779] The antibody-drug conjugate according to [777], wherein the antibody-drug conjugate is for use in treating colorectal cancer.

[780] The antibody-drug conjugate according to [777], wherein the antibody-drug conjugate is for use in treating gastric cancer.

[781] The antibody-drug conjugate according to [777], wherein the antibody-drug conjugate is for use in treating lung cancer.

[782] The antibody-drug conjugate according to [777], wherein the antibody-drug conjugate is for use in treating pancreatic cancer.

[783] The antibody-drug conjugate according to [777], wherein the antibody-drug conjugate is for use in treating kidney cancer.

[784] The antibody-drug conjugate according to [777], wherein the antibody-drug conjugate is for use in treating ovarian cancer.

[785] Use of an antibody-drug conjugate for the manufacture of a medicament for treating a disease through being administered in combination with a kinase inhibitor, wherein a drug-linker represented by the following formula:

[Formula 15]

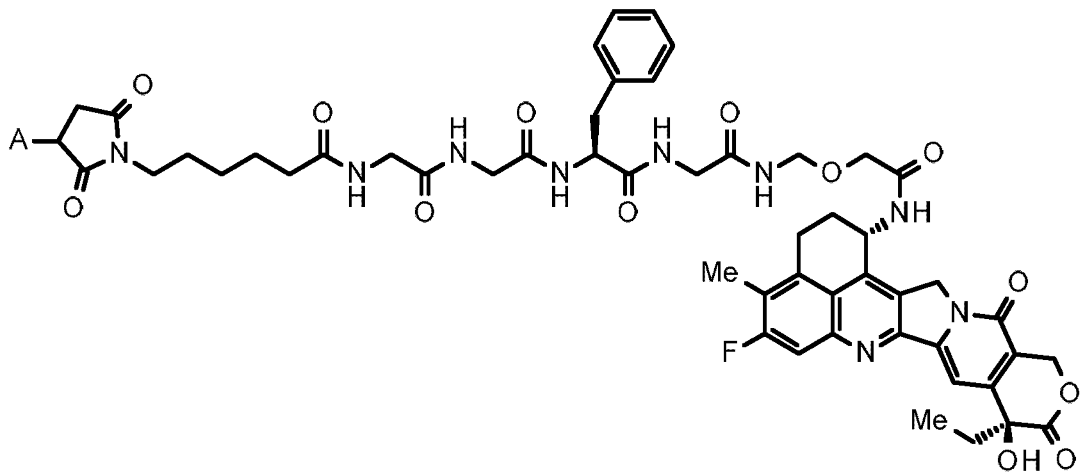

wherein A represents a connecting position to an antibody, is conjugated to the antibody via a thioether bond in the antibody-drug conjugate, and

the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.

[786] The use according to [785], wherein the kinase inhibitor is abemaciclib or a pharmacologically acceptable salt thereof.

[787] The use according to [785], wherein the kinase inhibitor is palbociclib or a pharmacologically acceptable salt

thereof.
[788] The use according to [785], wherein the kinase inhibitor is everolimus or a pharmacologically acceptable salt thereof.
[789] The use according to [785], wherein the kinase inhibitor is taselisib or a pharmacologically acceptable salt thereof.
[790] The use according to [785], wherein the kinase inhibitor is alpelisib or a pharmacologically acceptable salt thereof.
[791] The use according to [785], wherein the kinase inhibitor is regorafenib or a pharmacologically acceptable salt thereof.
[792] The use according to [785], wherein the kinase inhibitor is cabozantinib or a pharmacologically acceptable salt thereof.
[793] The use according to [785], wherein the kinase inhibitor is sunitinib or a pharmacologically acceptable salt thereof.
[794] The use according to [785], wherein the kinase inhibitor is nintedanib or a pharmacologically acceptable salt thereof.
[795] The use according to [785], wherein the kinase inhibitor is brigatinib or a pharmacologically acceptable salt thereof.
[796] The use according to [785], wherein the kinase inhibitor is erlotinib or a pharmacologically acceptable salt thereof.
[797] The use according to [785], wherein the kinase inhibitor is neratinib or a pharmacologically acceptable salt thereof.
[798] The use according to [785], wherein the kinase inhibitor is poziotinib or a pharmacologically acceptable salt thereof.
[799] The use according to [785], wherein the kinase inhibitor is tucatinib or a pharmacologically acceptable salt thereof.
[800] The use according to [785], wherein the kinase inhibitor is mubritinib or a pharmacologically acceptable salt thereof.
[801] The use according to any one of [785] to [800], wherein the antibody in the antibody-drug conjugate is an anti-HER2 antibody, an anti-HER3 antibody, an anti-TROP2 antibody, an anti-B7-H3 antibody, or an anti-CDH6 antibody.
[802] The use according to [801], wherein the antibody in the antibody-drug conjugate is an anti-HER2 antibody.
[803] The use according to [802], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.
[804] The pharmaceutical composition according to [802], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[805] The use according to [802], wherein the anti-HER2 antibody is an antibody comprising a heavy chain consisting of the amino acid sequence represented by SEQ ID NO: 1 and a light chain consisting of the amino acid sequence represented by SEQ ID NO: 2.
[806] The use according to [802], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[807] The use according to any one of [802] to [806], wherein the average number of units of the drug-linker conjugated per antibody molecule in the antibody-drug conjugate is in the range of from 7 to 8.
[808] The use according to [801], wherein the antibody in the antibody-drug conjugate is an anti-HER3 antibody.
[809] The use according to [808], wherein the anti-HER3 antibody is an antibody comprising a heavy chain consisting of the amino acid sequence represented by SEQ ID NO: 3 and a light chain consisting of the amino acid sequence represented by SEQ ID NO: 4.
[810] The use according to [809], wherein the anti-HER3 antibody lacks a lysine residue at the carboxyl terminus of the heavy chain.
[811] The use according to any one of [808] to [810], wherein the average number of units of the drug-linker conjugated per antibody molecule in the antibody-drug conjugate is in the range of from 7 to 8.
[812] The use according to [801], wherein the antibody in the antibody-drug conjugate is an anti-TROP2 antibody.

[813] The use according to [812], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[814] The use according to [813], wherein the anti-TROP2 antibody lacks a lysine residue at the carboxyl terminus of the heavy chain.
[815] The use according to any one of [812] to [814], wherein the average number of units of the drug-linker conjugated per antibody molecule in the antibody-drug conjugate is in the range of from 3.5 to 4.5.
[816] The use according to [801], wherein the antibody in the antibody-drug conjugate is an anti-B7-H3 antibody.
[817] The use according to [816], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[818] The use according to [817], wherein the anti-B7-H3 antibody lacks a lysine residue at the carboxyl terminus of the heavy chain.
[819] The use according to any one of [816] to [818], wherein the average number of units of the drug-linker conjugated per antibody molecule in the antibody-drug conjugate is in the range of from 3.5 to 4.5.
[820] The use according to [801], wherein the antibody in the antibody-drug conjugate is an anti-CDH6 antibody.
[821] The use according to [820], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.
[822] The use according to [821], wherein the anti-CDH6 antibody lacks a lysine residue at the carboxyl terminus of the heavy chain.
[823] The use according to any one of [820] to [822], wherein the average number of units of the drug-linker conjugated per antibody molecule in the antibody-drug conjugate is in the range of from 7 to 8.
[824] The use according to any one of [785] to [823], wherein the antibody-drug conjugate and the kinase inhibitor are separately contained as active components in different formulations, and are administered simultaneously or at different times.
[825] The use according to any one of [785] to [824], wherein the use is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.
[826] The use according to [825], wherein the use is for treating breast cancer.
[827] The use according to [825], wherein the use is for treating colorectal cancer.
[828] The use according to [825], wherein the use is for treating gastric cancer.
[829] The use according to [825], wherein the use is for treating lung cancer.
[830] The use according to [825], wherein the use is for treating pancreatic cancer.
[831] The use according to [825], wherein the use is for treating kidney cancer.
[832] The use according to [825], wherein the use is for treating ovarian cancer.
[833] Use of an antibody-drug conjugate for the manufacture of a medicament for treating a disease through being administered in combination with a kinase inhibitor, wherein the antibody-drug conjugate is an antibody-drug conjugate represented by the following formula:

[Formula 16]

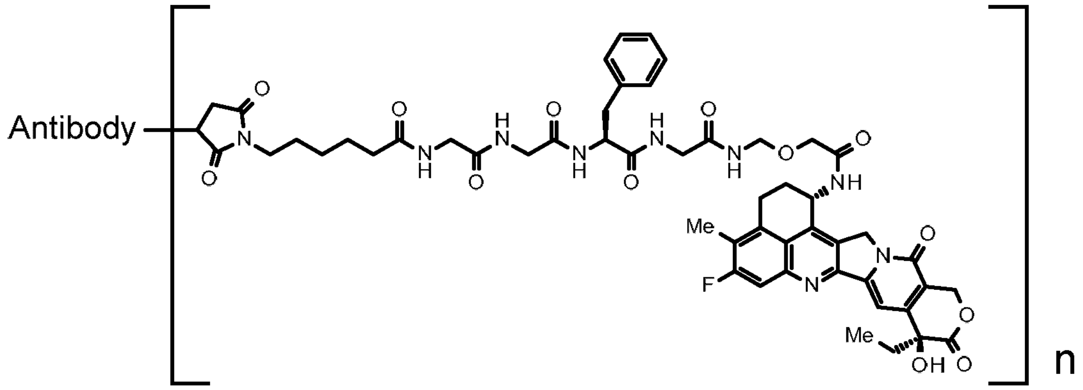

wherein a drug-linker is conjugated to an antibody via a thioether bond, and n is the average number of units of the drug-linker conjugated per antibody molecule, and
the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.
[834] The use according to [833], wherein the kinase inhibitor is abemaciclib or a pharmacologically acceptable salt thereof.
[835] The use according to [833], wherein the kinase inhibitor is palbociclib or a pharmacologically acceptable salt thereof.
[836] The use according to [833], wherein the kinase inhibitor is everolimus or a pharmacologically acceptable salt thereof.
[837] The use according to [833], wherein the kinase inhibitor is taselisib or a pharmacologically acceptable salt thereof.
[838] The use according to [833], wherein the kinase inhibitor is alpelisib or a pharmacologically acceptable salt thereof.
[839] The use according to [833], wherein the kinase inhibitor is regorafenib or a pharmacologically acceptable salt thereof.
[840] The use according to [833], wherein the kinase inhibitor is cabozantinib or a pharmacologically acceptable salt thereof.
[841] The use according to [833], wherein the kinase inhibitor is sunitinib or a pharmacologically acceptable salt thereof.
[842] The use according to [833], wherein the kinase inhibitor is nintedanib or a pharmacologically acceptable salt thereof.
[843] The use according to [833], wherein the kinase inhibitor is brigatinib or a pharmacologically acceptable salt thereof.
[844] The use according to [833], wherein the kinase inhibitor is erlotinib or a pharmacologically acceptable salt thereof.
[845] The use according to [833], wherein the kinase inhibitor is neratinib or a pharmacologically acceptable salt thereof.
[846] The use according to [833], wherein the kinase inhibitor is poziotinib or a pharmacologically acceptable salt

thereof.
[847] The use according to [833], wherein the kinase inhibitor is tucatinib or a pharmacologically acceptable salt thereof.
[848] The use according to [833], wherein the kinase inhibitor is mubritinib or a pharmacologically acceptable salt thereof.
[849] The use according to any one of [833] to [848], wherein the antibody in the antibody-drug conjugate is an anti-HER2 antibody, an anti-HER3 antibody, an anti-TROP2 antibody, an anti-B7-H3 antibody, or an anti-CDH6 antibody.
[850] The use according to [849], wherein the antibody in the antibody-drug conjugate is an anti-HER2 antibody.
[851] The use according to [850], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.
[852] The use according to [850], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[853] The use according to [850], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[854] The use according to [850], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[855] The use according to any one of [850] to [854], wherein the average number of units of the drug-linker conjugated per antibody molecule in the antibody-drug conjugate is in the range of from 7 to 8.
[856] The use according to [849], wherein the antibody in the antibody-drug conjugate is an anti-HER3 antibody.
[857] The use according to [856], wherein the anti-HER3 antibody is an antibody comprising a heavy chain consisting of the amino acid sequence represented by SEQ ID NO: 3 and a light chain consisting of the amino acid sequence represented by SEQ ID NO: 4.
[858] The use according to [857], wherein the anti-HER3 antibody lacks a lysine residue at the carboxyl terminus of the heavy chain.
[859] The use according to any one of [856] to [858], wherein the average number of units of the drug-linker conjugated per antibody molecule in the antibody-drug conjugate is in the range of from 7 to 8.
[860] The use according to [849], wherein the antibody in the antibody-drug conjugate is an anti-TROP2 antibody.
[861] The use according to [860], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[862] The use according to [861], wherein the anti-TROP2 antibody lacks a lysine residue at the carboxyl terminus of the heavy chain.
[863] The use according to any one of [860]to [862], wherein the average number of units of the drug-linker conjugated per antibody molecule in the antibody-drug conjugate is in the range of from 3.5 to 4.5.
[864] The use according to [849], wherein the antibody in the antibody-drug conjugate is an anti-B7-H3 antibody.
[865] The use according to [864], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[866] The use according to [865], wherein the anti-B7-H3 antibody lacks a lysine residue at the carboxyl terminus of the heavy chain.
[867] The use according to any one of [864] to [866], wherein the average number of units of the drug-linker conjugated per antibody molecule in the antibody-drug conjugate is in the range of from 3.5 to 4.5.
[868] The use according to [849], wherein the antibody in the antibody-drug conjugate is an anti-CDH6 antibody.
[869] The use according to [868], wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.
[870] The use according to [869], wherein the anti-CDH6 antibody lacks a lysine residue at the carboxyl terminus of the heavy chain.
[871] The use according to any one of [868]to [870], wherein the average number of units of the drug-linker conjugated

per antibody molecule in the antibody-drug conjugate is in the range of from 7 to 8.

[872] The use according to any one of [833] to [871], wherein the antibody-drug conjugate and the kinase inhibitor are separately contained as active components in different formulations, and are administered simultaneously or at different times.

[873] The use according to any one of [833] to [872], wherein the use is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[874] The use according to [873], wherein the use is for treating breast cancer.

[875] The use according to [873], wherein the use is for treating colorectal cancer.

[876] The use according to [873], wherein the use is for treating gastric cancer.

[877] The use according to [873], wherein the use is for treating lung cancer.

[878] The use according to [873], wherein the use is for treating pancreatic cancer.

[879] The use according to [873], wherein the use is for treating kidney cancer.

[880] The use according to [873], wherein the use is for treating ovarian cancer.

[881] A pharmaceutical composition wherein an anti-cancer agent and a kinase inhibitor are administered in combination, and the anti-cancer agent releases a drug represented by the following formula:

[Formula 17]

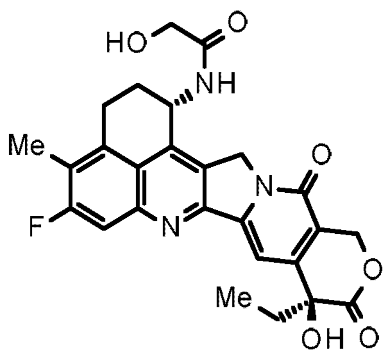

in a tumor, and

the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[882] The pharmaceutical composition according to [881], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[883] The pharmaceutical composition according to [882], wherein the kinase inhibitor is a CDK4/6 inhibitor.

[884] The pharmaceutical composition according to [883], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[885] The pharmaceutical composition according to [882], wherein the kinase inhibitor is an mTOR inhibitor.

[886] The pharmaceutical composition according to [885], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[887] The pharmaceutical composition according to [882], wherein the kinase inhibitor is a PI3K inhibitor.

[888] The pharmaceutical composition according to [887], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib,

CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[889] The pharmaceutical composition according to [882], wherein the kinase inhibitor is an RAF inhibitor.

[890] The pharmaceutical composition according to [889], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[891] The pharmaceutical composition according to [882], wherein the kinase inhibitor is a VEGFR inhibitor.

[892] The pharmaceutical composition according to [891], wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[893] The pharmaceutical composition according to [882], wherein the kinase inhibitor is a KIT inhibitor.

[894] The pharmaceutical composition according to [893], wherein the KIT inhibitor is regorafenib, sorafenib, imatinib, ilorasertib, sunitinib, pazopanib, lenvatinib, or dasatinib, or a pharmacologically acceptable salt thereof.

[895] The pharmaceutical composition according to [882], wherein the kinase inhibitor is an RET inhibitor.

[896] The pharmaceutical composition according to [895], wherein the RET inhibitor is regorafenib, sorafenib, vandetanib, lenvatinib, or sunitinib, or a pharmacologically acceptable salt thereof.

[897] The pharmaceutical composition according to [882], wherein the kinase inhibitor is a PDGFR inhibitor.

[898] The pharmaceutical composition according to [897], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.

[899] The pharmaceutical composition according to [882], wherein the kinase inhibitor is an FGFR inhibitor.

[900] The pharmaceutical composition according to [897], wherein the FGFR inhibitor is regorafenib, sorafenib, lenvatinib, nintedanib, axitinib, or pazopanib, or a pharmacologically acceptable salt thereof.

[901] The pharmaceutical composition according to [882], wherein the kinase inhibitor is an FLT3 inhibitor.

[902] The pharmaceutical composition according to [901], wherein the FLT3 inhibitor is gilteritinib, quizartinib, midostaurin, sorafenib, ilorasertib, ENMD-2076, or sunitinib, or a pharmacologically acceptable salt thereof.

[903] The pharmaceutical composition according to [882], wherein the kinase inhibitor is an ALK inhibitor.

[904] The pharmaceutical composition according to [903], wherein the ALK inhibitor is brigatinib, crizotinib, ceritinib, alectinib, or lorlatinib, or a pharmacologically acceptable salt thereof.

[905] The pharmaceutical composition according to [882], wherein the kinase inhibitor is a CSF-1R inhibitor.

[906] The pharmaceutical composition according to [905], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.

[907] The pharmaceutical composition according to [882], wherein the kinase inhibitor is an EGFR inhibitor.

[908] The pharmaceutical composition according to [907], wherein the EGFR inhibitor is gefitinib, erlotinib, afatinib, osimertinib, dacomitinib, lapatinib, neratinib, pyrotinib, or poziotinib, or a pharmacologically acceptable salt thereof.

[909] The pharmaceutical composition according to [882], wherein the kinase inhibitor is an HER2 inhibitor.

[910] The pharmaceutical composition according to [909], wherein the HER2 inhibitor is tucatinib, neratinib, mubritinib, lapatinib, pyrotinib, or poziotinib, or a pharmacologically acceptable salt thereof.

[911] The pharmaceutical composition according to any one of [881] to [910], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget’s disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[912] A method of treatment comprising administering an anti-cancer agent and a kinase inhibitor in combination to a subject in need of treatment, wherein the anti-cancer agent releases a drug represented by the following formula:

[Formula 18]

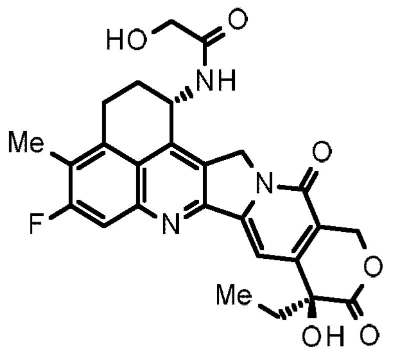

in a tumor, and

the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[913] The method of treatment according to [912], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[914] The method of treatment according to [913], wherein the kinase inhibitor is a CDK4/6 inhibitor.

[915] The method of treatment according to [914], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[916] The method of treatment according to [913], wherein the kinase inhibitor is an mTOR inhibitor.

[917] The method of treatment according to [916], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[918] The method of treatment according to [913], wherein the kinase inhibitor is a PI3K inhibitor.

[919] The method of treatment according to [918], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[920] The method of treatment according to [913], wherein the kinase inhibitor is an RAF inhibitor.

[921] The method of treatment according to [920], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[922] The method of treatment according to [913], wherein the kinase inhibitor is a VEGFR inhibitor.

[923] The method of treatment according to [922], wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[924] The method of treatment according to [913], wherein the kinase inhibitor is a KIT inhibitor.

[925] The method of treatment according to [924], wherein the KIT inhibitor is regorafenib, sorafenib, imatinib, ilorasertib, sunitinib, pazopanib, lenvatinib, or dasatinib, or a pharmacologically acceptable salt thereof.

[926] The method of treatment according to [913], wherein the kinase inhibitor is an RET inhibitor.

[927] The method of treatment according to [926], wherein the RET inhibitor is regorafenib, sorafenib, vandetanib, lenvatinib, or sunitinib, or a pharmacologically acceptable salt thereof.

[928] The method of treatment according to [913], wherein the kinase inhibitor is a PDGFR inhibitor.

[929] The method of treatment according to [928], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.

[930] The method of treatment according to [913], wherein the kinase inhibitor is an FGFR inhibitor.

[931] The method of treatment according to [930], wherein the FGFR inhibitor is regorafenib, sorafenib, lenvatinib,

nintedanib, axitinib, or pazopanib, or a pharmacologically acceptable salt thereof.

[932] The method of treatment according to [913], wherein the kinase inhibitor is an FLT3 inhibitor.

[933] The method of treatment according to [932], wherein the FLT3 inhibitor is gilteritinib, quizartinib, midostaurin, sorafenib, ilorasertib, ENMD-2076, or sunitinib, or a pharmacologically acceptable salt thereof.

[934] The method of treatment according to [913], wherein the kinase inhibitor is an ALK inhibitor.

[935] The method of treatment according to [934], wherein the ALK inhibitor is brigatinib, crizotinib, ceritinib, alectinib, or lorlatinib, or a pharmacologically acceptable salt thereof.

[936] The method of treatment according to [913], wherein the kinase inhibitor is a CSF-1R inhibitor.

[937] The method of treatment according to [936], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.

[938] The method of treatment according to [913], wherein the kinase inhibitor is an EGFR inhibitor.

[939] The method of treatment according to [938], wherein the EGFR inhibitor is gefitinib, erlotinib, afatinib, osimertinib, dacomitinib, lapatinib, neratinib, pyrotinib, or poziotinib, or a pharmacologically acceptable salt thereof.

[940] The method of treatment according to [913], wherein the kinase inhibitor is an HER2 inhibitor.

[941] The method of treatment according to [940], wherein the HER2 inhibitor is tucatinib, neratinib, mubritinib, lapatinib, pyrotinib, or poziotinib, or a pharmacologically acceptable salt thereof.

[942] The method of treatment according to any one of [912] to [941], wherein the method of treatment is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[943] An anti-cancer agent for use in treating a disease through being administered in combination with a kinase inhibitor, wherein the anti-cancer agent releases a drug represented by the following formula:

[Formula 19]

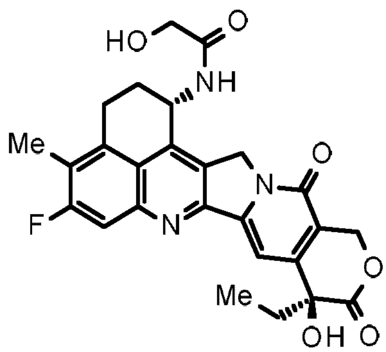

in a tumor, and

the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[944] The anti-cancer agent according to [943], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[945] The anti-cancer agent according to [944], wherein the kinase inhibitor is a CDK4/6 inhibitor.

[946] The anti-cancer agent according to [945], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[947] The anti-cancer agent according to [944], wherein the kinase inhibitor is an mTOR inhibitor.

[948] The anti-cancer agent according to [947], wherein the mTOR inhibitor is everolimus, sirolimus, temsirolimus,

TAK-228, CC-223, AZD8055, dactolisib, apitolisib, gedatolisib, LY3023414, PF-04691502, NVP-BGT226, or PQR309, or a pharmacologically acceptable salt thereof.
[949] The anti-cancer agent according to [944], wherein the kinase inhibitor is a PI3K inhibitor.
[950] The anti-cancer agent according to [949], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.
[951] The anti-cancer agent according to [944], wherein the kinase inhibitor is an RAF inhibitor.
[952] The anti-cancer agent according to [951], wherein the RAF inhibitor is regorafenib, sorafenib, vemurafenib, dabrafenib, encorafenib, RAF265, GDC-5573, LY3009120, or RO5126766, or a pharmacologically acceptable salt thereof.
[953] The anti-cancer agent according to [944], wherein the kinase inhibitor is a VEGFR inhibitor.
[954] The anti-cancer agent according to [953], wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.
[955] The anti-cancer agent according to [944], wherein the kinase inhibitor is a KIT inhibitor.
[956] The anti-cancer agent according to [955], wherein the KIT inhibitor is regorafenib, sorafenib, imatinib, ilorasertib, sunitinib, pazopanib, lenvatinib, or dasatinib, or a pharmacologically acceptable salt thereof.
[957] The anti-cancer agent according to [944], wherein the kinase inhibitor is an RET inhibitor.
[958] The anti-cancer agent according to [957], wherein the RET inhibitor is regorafenib, sorafenib, vandetanib, lenvatinib, or sunitinib, or a pharmacologically acceptable salt thereof.
[959] The anti-cancer agent according to [944], wherein the kinase inhibitor is a PDGFR inhibitor.
[960] The anti-cancer agent according to [959], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.
[961] The anti-cancer agent according to [944], wherein the kinase inhibitor is an FGFR inhibitor.
[962] The anti-cancer agent according to [961], wherein the FGFR inhibitor is regorafenib, sorafenib, lenvatinib, nintedanib, axitinib, or pazopanib, or a pharmacologically acceptable salt thereof.
[963] The anti-cancer agent according to [944], wherein the kinase inhibitor is an FLT3 inhibitor.
[964] The anti-cancer agent according to [963], wherein the FLT3 inhibitor is gilteritinib, quizartinib, midostaurin, sorafenib, ilorasertib, ENMD-2076, or sunitinib, or a pharmacologically acceptable salt thereof.
[965] The anti-cancer agent according to [944], wherein the kinase inhibitor is an ALK inhibitor.
[966] The anti-cancer agent according to [965], wherein the ALK inhibitor is brigatinib, crizotinib, ceritinib, alectinib, or lorlatinib, or a pharmacologically acceptable salt thereof.
[967] The anti-cancer agent according to [944], wherein the kinase inhibitor is a CSF-1R inhibitor.
[968] The anti-cancer agent according to [967], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.
[969] The anti-cancer agent according to [944], wherein the kinase inhibitor is an EGFR inhibitor.
[970] The anti-cancer agent according to [969], wherein the EGFR inhibitor is gefitinib, erlotinib, afatinib, osimertinib, dacomitinib, lapatinib, neratinib, pyrotinib, or poziotinib, or a pharmacologically acceptable salt thereof.
[971] The anti-cancer agent according to [944], wherein the kinase inhibitor is an HER2 inhibitor.
[972] The anti-cancer agent according to [971], wherein the HER2 inhibitor is tucatinib, neratinib, mubritinib, lapatinib, pyrotinib, or poziotinib, or a pharmacologically acceptable salt thereof.
[973] The anti-cancer agent according to any one of [943] to [972], wherein the anti-cancer agent is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.
[974] Use of an anti-cancer agent for the manufacture of a medicament for treating a disease through being administered in combination with a kinase inhibitor, wherein the anti-cancer agent releases a drug represented by the following formula:

[Formula 20]

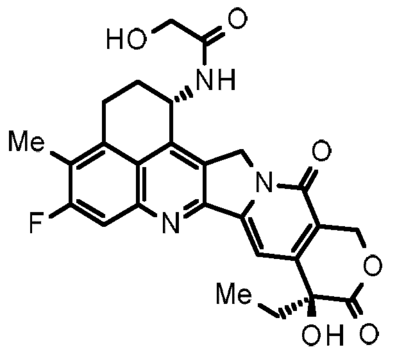

in a tumor, and

the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

[975] The use according to [974], wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

[976] The use according to [975], wherein the kinase inhibitor is a CDK4/6 inhibitor.

[977] The use according to [976], wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

[978] The use according to [975], wherein the kinase inhibitor is an mTOR inhibitor.

[979] The use according to [978], wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

[980] The use according to [975], wherein the kinase inhibitor is a PI3K inhibitor.

[981] The use according to [980], wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

[982] The use according to [975], wherein the kinase inhibitor is an RAF inhibitor.

[983] The use according to [982], wherein the RAF inhibitor is regorafenib, sorafenib, vemurafenib, dabrafenib, encorafenib, RAF265, GDC-5573, LY3009120, or RO5126766, or a pharmacologically acceptable salt thereof.

[984] The use according to [975], wherein the kinase inhibitor is a VEGFR inhibitor.

[985] The use according to [984], wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

[986] The use according to [975], wherein the kinase inhibitor is a KIT inhibitor.

[987] The use according to [986], wherein the KIT inhibitor is regorafenib, sorafenib, imatinib, ilorasertib, sunitinib, pazopanib, lenvatinib, or dasatinib, or a pharmacologically acceptable salt thereof.

[988] The use according to [975], wherein the kinase inhibitor is an RET inhibitor.

[989] The use according to [988], wherein the RET inhibitor is regorafenib, sorafenib, vandetanib, lenvatinib, or sunitinib, or a pharmacologically acceptable salt thereof.

[990] The use according to [975], wherein the kinase inhibitor is a PDGFR inhibitor.

[991] The use according to [990], wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.

[992] The use according to [975], wherein the kinase inhibitor is an FGFR inhibitor.

[993] The use according to [992], wherein the FGFR inhibitor is regorafenib, sorafenib, lenvatinib, nintedanib, axitinib, or pazopanib, or a pharmacologically acceptable salt thereof.

[994] The use according to [975], wherein the kinase inhibitor is an FLT3 inhibitor.

[995] The use according to [994], wherein the FLT3 inhibitor is gilteritinib, quizartinib, midostaurin, sorafenib, ilorasertib, ENMD-2076, or sunitinib, or a pharmacologically acceptable salt thereof.
[996] The use according to [975], wherein the kinase inhibitor is an ALK inhibitor.
[997] The use according to [996], wherein the ALK inhibitor is brigatinib, crizotinib, ceritinib, alectinib, or lorlatinib, or a pharmacologically acceptable salt thereof.
[998] The use according to [975], wherein the kinase inhibitor is a CSF-1R inhibitor.
[999] The use according to [998], wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.
[1000] The use according to [975], wherein the kinase inhibitor is an EGFR inhibitor.
[1001] The use according to [1000], wherein the EGFR inhibitor is gefitinib, erlotinib, afatinib, osimertinib, dacomitinib, lapatinib, neratinib, pyrotinib, or poziotinib, or a pharmacologically acceptable salt thereof.
[1002] The use according to [975], wherein the kinase inhibitor is an HER2 inhibitor.
[1003] The use according to [1002], wherein the HER2 inhibitor is tucatinib, neratinib, mubritinib, lapatinib, pyrotinib, or poziotinib, or a pharmacologically acceptable salt thereof.
[1004] The use according to any one of [974] to [1003], wherein the use is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.
[1005] A pharmaceutical composition wherein an anti-cancer agent and a kinase inhibitor are administered in combination, and the anti-cancer agent releases a drug represented by the following formula:

[Formula 21]

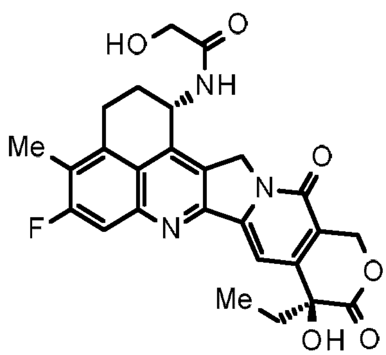

in a tumor, and
the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.
[1006] The pharmaceutical composition according to [1005], wherein the kinase inhibitor is abemaciclib or a pharmacologically acceptable salt thereof.
[1007] The pharmaceutical composition according to [1005], wherein the kinase inhibitor is palbociclib or a pharmacologically acceptable salt thereof.
[1008] The pharmaceutical composition according to [1005], wherein the kinase inhibitor is everolimus or a pharmacologically acceptable salt thereof.
[1009] The pharmaceutical composition according to [1005], wherein the kinase inhibitor is taselisib or a pharmacologically acceptable salt thereof.
[1010] The pharmaceutical composition according to [1005], wherein the kinase inhibitor is alpelisib or a pharmacologically acceptable salt thereof.
[1011] The pharmaceutical composition according to [1005], wherein the kinase inhibitor is regorafenib or a pharmacologically acceptable salt thereof.
[1012] The pharmaceutical composition according to [1005], wherein the kinase inhibitor is cabozantinib or a pharmacologically acceptable salt thereof.
[1013] The pharmaceutical composition according to [1005], wherein the kinase inhibitor is sunitinib or a pharmacologically acceptable salt thereof.
[1014] The pharmaceutical composition according to [1005], wherein the kinase inhibitor is nintedanib or a pharmacologically acceptable salt thereof.
[1015] The pharmaceutical composition according to [1005], wherein the kinase inhibitor is brigatinib or a pharmacologically acceptable salt thereof.
[1016] The pharmaceutical composition according to [1005], wherein the kinase inhibitor is erlotinib or a pharmacologically acceptable salt thereof.
[1017] The pharmaceutical composition according to [1005], wherein the kinase inhibitor is neratinib or a pharmacologically acceptable salt thereof.
[1018] The pharmaceutical composition according to [1005], wherein the kinase inhibitor is poziotinib or a pharmacologically acceptable salt thereof.
[1019] The pharmaceutical composition according to [1005], wherein the kinase inhibitor is tucatinib or a pharmacologically acceptable salt thereof.
[1020] The pharmaceutical composition according to [1005], wherein the kinase inhibitor is mubritinib or a pharmacologically acceptable salt thereof.
[1021] The pharmaceutical composition according to any one of [1005] to [1020], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.
[1022] A method of treatment comprising administering an anti-cancer agent and a kinase inhibitor in combination to a subject in need of treatment, wherein the anti-cancer agent releases a drug represented by the following formula:

[Formula 22]

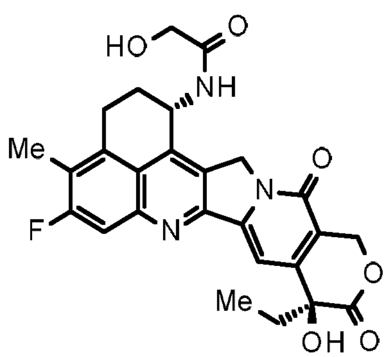

in a tumor, and
the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib,

sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.

[1023] The method of treatment according to [1022], wherein the kinase inhibitor is abemaciclib or a pharmacologically acceptable salt thereof.

[1024] The method of treatment according to [1022], wherein the kinase inhibitor is palbociclib or a pharmacologically acceptable salt thereof.

[1025] The method of treatment according to [1022], wherein the kinase inhibitor is everolimus or a pharmacologically acceptable salt thereof.

[1026] The method of treatment according to [1022], wherein the kinase inhibitor is taselisib or a pharmacologically acceptable salt thereof.

[1027] The method of treatment according to [1022], wherein the kinase inhibitor is alpelisib or a pharmacologically acceptable salt thereof.

[1028] The method of treatment according to [1022], wherein the kinase inhibitor is regorafenib or a pharmacologically acceptable salt thereof.

[1029] The method of treatment according to [1022], wherein the kinase inhibitor is cabozantinib or a pharmacologically acceptable salt thereof.

[1030] The method of treatment according to [1022], wherein the kinase inhibitor is sunitinib or a pharmacologically acceptable salt thereof.

[1031] The method of treatment according to [1022], wherein the kinase inhibitor is nintedanib or a pharmacologically acceptable salt thereof.

[1032] The method of treatment according to [1022], wherein the kinase inhibitor is brigatinib or a pharmacologically acceptable salt thereof.

[1033] The method of treatment according to [1022], wherein the kinase inhibitor is erlotinib or a pharmacologically acceptable salt thereof.

[1034] The method of treatment according to [1022], wherein the kinase inhibitor is neratinib or a pharmacologically acceptable salt thereof.

[1035] The method of treatment according to [1022], wherein the kinase inhibitor is poziotinib or a pharmacologically acceptable salt thereof.

[1036] The method of treatment according to [1022], wherein the kinase inhibitor is tucatinib or a pharmacologically acceptable salt thereof.

[1037] The method of treatment according to [1022], wherein the kinase inhibitor is mubritinib or a pharmacologically acceptable salt thereof.

[1038] The pharmaceutical composition according to any one of [1022] to [1037], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

[1039] An anti-cancer agent for use in treating a disease through being administered in combination with a kinase inhibitor, wherein the anti-cancer agent releases a drug represented by the following formula:

[Formula 23]

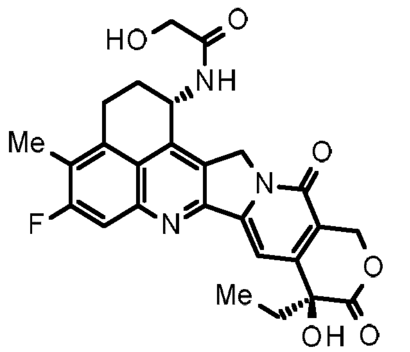

in a tumor, and

the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.

[1040] The anti-cancer agent according to [1039], wherein the kinase inhibitor is abemaciclib or a pharmacologically acceptable salt thereof.

[1041] The anti-cancer agent according to [1039], wherein the kinase inhibitor is palbociclib or a pharmacologically acceptable salt thereof.

[1042] The anti-cancer agent according to [1039], wherein the kinase inhibitor is everolimus or a pharmacologically acceptable salt thereof.

[1043] The anti-cancer agent according to [1039], wherein the kinase inhibitor is taselisib or a pharmacologically acceptable salt thereof.

[1044] The anti-cancer agent according to [1039], wherein the kinase inhibitor is alpelisib or a pharmacologically acceptable salt thereof.

[1045] The anti-cancer agent according to [1039], wherein the kinase inhibitor is regorafenib or a pharmacologically acceptable salt thereof.

[1046] The anti-cancer agent according to [1039], wherein the kinase inhibitor is cabozantinib or a pharmacologically acceptable salt thereof.

[1047] The anti-cancer agent according to [1039], wherein the kinase inhibitor is sunitinib or a pharmacologically acceptable salt thereof.

[1048] The anti-cancer agent according to [1039], wherein the kinase inhibitor is nintedanib or a pharmacologically acceptable salt thereof.

[1049] The anti-cancer agent according to [1039], wherein the kinase inhibitor is brigatinib or a pharmacologically acceptable salt thereof.

[1050] The anti-cancer agent according to [1039], wherein the kinase inhibitor is erlotinib or a pharmacologically acceptable salt thereof.

[1051] The anti-cancer agent according to [1039], wherein the kinase inhibitor is neratinib or a pharmacologically acceptable salt thereof.

[1052] The anti-cancer agent according to [1039], wherein the kinase inhibitor is poziotinib or a pharmacologically acceptable salt thereof.

[1053] The anti-cancer agent according to [1039], wherein the kinase inhibitor is tucatinib or a pharmacologically

acceptable salt thereof.

[1054] The anti-cancer agent according to [1039], wherein the kinase inhibitor is mubritinib or a pharmacologically acceptable salt thereof.

[1055] The anti-cancer agent according to any one of [1039] to [1054], wherein the anti-cancer agent is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, and melanoma.

[1056] Use of an anti-cancer agent for the manufacture of a medicament for treating a disease through being administered in combination with a kinase inhibitor, wherein the anti-cancer agent releases a drug represented by the following formula:

[Formula 24]

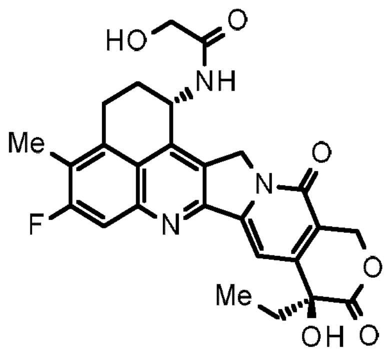

in a tumor, and

the kinase inhibitor is at least one selected from the group consisting of abemaciclib, palbociclib, ribociclib, trilaciclib, G1T38, PF-06873600, TP-1287, FN-1501, KRX-0601, everolimus, sirolimus, temsirolimus, TAK-228, CC-223, AZD8055, dactolisib, apitolisib, gedatolisib, LY3023414, PF-04691502, NVP-BGT226, PQR309, taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, rigosertib, ipatasertib, uprosertib, MK-2206, BAY1125976, AZD5363, TAS-117, ONC201, BVD-523, CC-90003, GDC-0994, LY3214996, MK-8353, trametinib, binimetinib, selumetinib, refametinib, pimasertib, cobimetinib, E6201, PD-0325901, RO5126766, GDC-0623, regorafenib, sorafenib, vemurafenib, dabrafenib, encorafenib, RAF265, GDC-5573, LY3009120, dinaciclib, milciclib, seliciclib, alvocidib, roniciclib, voruciclib, AT7519, PHA-793887, CYC-065, MK-8776, LY2606368, LY2603618, CBP501, GDC-0425, CCT245737, AZD1775, volasertib, alisertib, ilorasertib, ENMD-2076, AMG900, imatinib, dasatinib, bosutinib, nilotinib, ponatinib, vandetanib, sunitinib, axitinib, pazopanib, lenvatinib, nintedanib, cabozantinib, tivozanib, brivanib, linifanib, lucitanib, ibrutinib, acalabrutinib, tirabrutinib, gilteritinib, quizartinib, midostaurin, brigatinib, crizotinib, ceritinib, alectinib, lorlatinib, ruxolitinib, tofacitinib, baricitinib, pacritinib, capmatinib, tepotinib, pexidartinib, BLZ-945, JNJ-40346527, JNJ-28312141, entrectinib, GR-389988, gefitinib, erlotinib, afatinib, osimertinib, dacomitinib, lapatinib, neratinib, pyrotinib, poziotinib, tucatinib, and mubritinib, and pharmacologically acceptable salts thereof.

[1057] The use according to [1056], wherein the kinase inhibitor is abemaciclib or a pharmacologically acceptable salt thereof.

[1058] The use according to [1056], wherein the kinase inhibitor is palbociclib or a pharmacologically acceptable salt thereof.

[1059] The use according to [1056], wherein the kinase inhibitor is everolimus or a pharmacologically acceptable salt thereof.

[1060] The use according to [1056], wherein the kinase inhibitor is taselisib or a pharmacologically acceptable salt thereof.

[1061] The use according to [1056], wherein the kinase inhibitor is alpelisib or a pharmacologically acceptable salt thereof.

[1062] The use according to [1056], wherein the kinase inhibitor is regorafenib or a pharmacologically acceptable salt thereof.

[1063] The use according to [1056], wherein the kinase inhibitor is cabozantinib or a pharmacologically acceptable

salt thereof.

[1064] The use according to [1056], wherein the kinase inhibitor is sunitinib or a pharmacologically acceptable salt thereof.

[1065] The use according to [1056], wherein the kinase inhibitor is nintedanib or a pharmacologically acceptable salt thereof.

[1066] The use according to [1056], wherein the kinase inhibitor is brigatinib or a pharmacologically acceptable salt thereof.

[1067] The use according to [1056], wherein the kinase inhibitor is erlotinib or a pharmacologically acceptable salt thereof.

[1068] The use according to [1056], wherein the kinase inhibitor is neratinib or a pharmacologically acceptable salt thereof.

[1069] The use according to [1056], wherein the kinase inhibitor is poziotinib or a pharmacologically acceptable salt thereof.

[1070] The use according to [1056], wherein the kinase inhibitor is tucatinib or a pharmacologically acceptable salt thereof.

[1071] The use according to [1056], wherein the kinase inhibitor is mubritinib or a pharmacologically acceptable salt thereof.

[1072] The use according to any one of [1056] to [1071], wherein the use is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

## Advantageous Effects of Invention

**[0013]** The present invention can provide a pharmaceutical composition wherein a specific antibody-drug conjugate and a kinase inhibitor are administered in combination, and/or a method of treatment wherein a specific antibody-drug conjugate and a kinase inhibitor are administered in combination to a subject.

## Brief Description of Drawings

**[0014]**

[Figure 1] Figure 1 is a diagram showing the amino acid sequence of a heavy chain of an anti-HER2 antibody (SEQ ID NO: 1).

[Figure 2] Figure 2 is a diagram showing the amino acid sequence of a light chain of an anti-HER2 antibody (SEQ ID NO: 2).

[Figure 3] Figure 3 is a diagram showing the amino acid sequence of a heavy chain of an anti-HER3 antibody (SEQ ID NO: 3).

[Figure 4] Figure 4 is a diagram showing the amino acid sequence of a light chain of an anti-HER3 antibody (SEQ ID NO: 4).

[Figure 5] Figure 5 is a diagram showing the amino acid sequence of a heavy chain of an anti-TROP2 antibody (SEQ ID NO: 5).

[Figure 6] Figure 6 is a diagram showing the amino acid sequence of a light chain of an anti-TROP2 antibody (SEQ ID NO: 6).

[Figure 7] Figure 7 is a diagram showing the amino acid sequence of a heavy chain of an anti-B7-H3 antibody (SEQ ID NO: 7).

[Figure 8] Figure 8 is a diagram showing the amino acid sequence of a light chain of an anti-B7-H3 antibody (SEQ ID NO: 8).

[Figure 9] Figure 9 is a diagram showing the amino acid sequence of a heavy chain of an anti-CDH6 antibody (SEQ ID NO: 9).

[Figure 10] Figure 10 is a diagram showing the amino acid sequence of a light chain of an anti-CDH6 antibody (SEQ ID NO: 10).

[Figure 11] Figure 11 is a diagram showing tumor growth inhibitory effects on mice with subcutaneously transplanted MDA-MB-453 cells for a single administration group with an antibody-drug conjugate (1) and that with everolimus, and for a combined administration group with the antibody-drug conjugate (1) and everolimus.

[Figure 12] Figure 12 is a diagram showing tumor growth inhibitory effects on mice with subcutaneously transplanted MDA-MB-453 cells for a single administration group with an antibody-drug conjugate (1) and that with taselisib, and for a combined administration group with the antibody-drug conjugate (1) and taselisib.
[Figure 13] Figure 13 is a diagram showing tumor growth inhibitory effects on mice with subcutaneously transplanted MDA-MB-453 cells for a single administration group with an antibody-drug conjugate (1) and that with abemaciclib, and for a combined administration group with the antibody-drug conjugate (1) and abemaciclib.
[Figure 14] Figure 14 is a diagram showing tumor growth inhibitory effects on mice with subcutaneously transplanted JIMT-1 cells for a single administration group with an antibody-drug conjugate (1) and that with everolimus, and for a combined administration group with the antibody-drug conjugate (1) and everolimus.
[Figure 15] Figure 15 is a diagram showing tumor growth inhibitory effects on mice with subcutaneously transplanted COLO201 cells for a single administration group with an antibody-drug conjugate (1) and that with regorafenib, and for a combined administration group with the antibody-drug conjugate (1) and regorafenib.
[Figure 16] Figure 16 is a diagram showing tumor growth inhibitory effects on mice with subcutaneously transplanted KPL-4 cells for a single administration group with an antibody-drug conjugate (1) and that with tucatinib, and for a combined administration group with the antibody-drug conjugate (1) and tucatinib.
[Figure 17] Figure 17 is a diagram showing tumor growth inhibitory effects on mice with subcutaneously transplanted MDA-MB-453 cells for a single administration group with an antibody-drug conjugate (1) and that with tucatinib, and for a combined administration group with the antibody-drug conjugate (1) and tucatinib.
[Figure 18] Figure 18 is a diagram showing tumor growth inhibitory effects on mice with subcutaneously transplanted NCI-N87 cells for a single administration group with an antibody-drug conjugate (1) and that with tucatinib, and for a combined administration group with the antibody-drug conjugate (1) and tucatinib.
[Figure 19] Figure 19 is a diagram showing tumor growth inhibitory effects on mice with subcutaneously transplanted KPL-4 cells for a single administration group with an antibody-drug conjugate (2) and that with neratinib, and for a combined administration group with the antibody-drug conjugate (2) and neratinib.
[Figure 20] Figure 20 is a diagram showing tumor growth inhibitory effects on mice with subcutaneously transplanted 786-O cells for a single administration group with an antibody-drug conjugate (3) and that with cabozantinib, and for a combined administration group with the antibody-drug conjugate (3) and cabozantinib.
[Figure 21] Figure 21 is a diagram showing tumor growth inhibitory effects on mice with subcutaneously transplanted MDA-MB-453 cells for a single administration group with an antibody-drug conjugate (2) and that with palbociclib, and for a combined administration group with the antibody-drug conjugate (2) and palbociclib.
[Figure 22] Figure 22 is a diagram showing tumor growth inhibitory effects on mice with subcutaneously transplanted MDA-MB-453 cells for a single administration group with an antibody-drug conjugate (2) and that with alpelisib, and for a combined administration group with the antibody-drug conjugate (2) and alpelisib.

## Description of Embodiments

**[0015]** Hereinafter, preferred modes for carrying out the present invention are described. The embodiments described below are given merely for illustrating one example of a typical embodiment of the present invention and are not intended to limit the scope of the present invention.

### 1. Antibody-drug conjugate

**[0016]** The antibody-drug conjugate used in the present invention is an antibody-drug conjugate in which a drug-linker represented by the following formula:

[Formula 25]

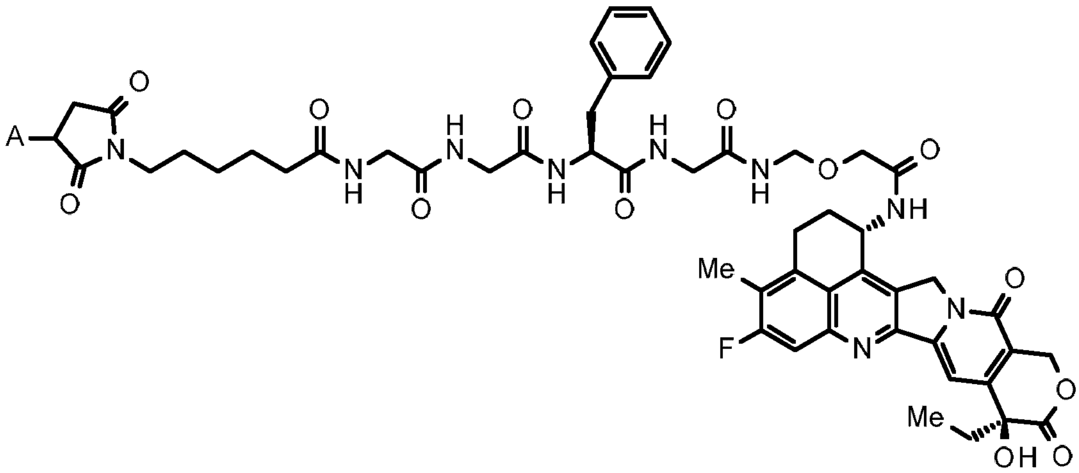

wherein A represents the connecting position to an antibody, is conjugated to the antibody via a thioether bond.

**[0017]** In the present invention, the partial structure consisting of a linker and a drug in the antibody-drug conjugate is referred to as a "drug-linker". The drug-linker is connected to a thiol group (in other words, the sulfur atom of a cysteine residue) formed at an interchain disulfide bond site (two sites between heavy chains, and two sites between a heavy chain and a light chain) in the antibody.

**[0018]** The drug-linker of the present invention includes exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinolin-10,13-dione, (also expressed as chemical name: (1S,9S)-1-amino-9-ethyl-5-fluoro-2,3-dihydro-9-hydroxy-4-methyl-1H,12H-benzo[de]pyrano[3',4':6,7]indolizino[1,2-b]quinolin-10,13(9H,15H)-dione)), which is a topoisomerase I inhibitor, as a component. Exatecan is a camptothecin derivative having an antitumor effect, represented by the following formula:

[Formula 26]

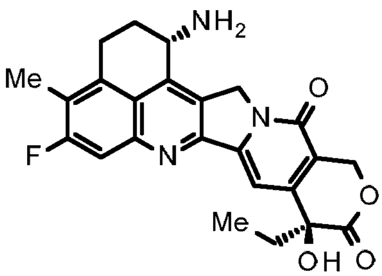

**[0019]** The antibody-drug conjugate used in the present invention can be also represented by the following formula:

[Formula 27]

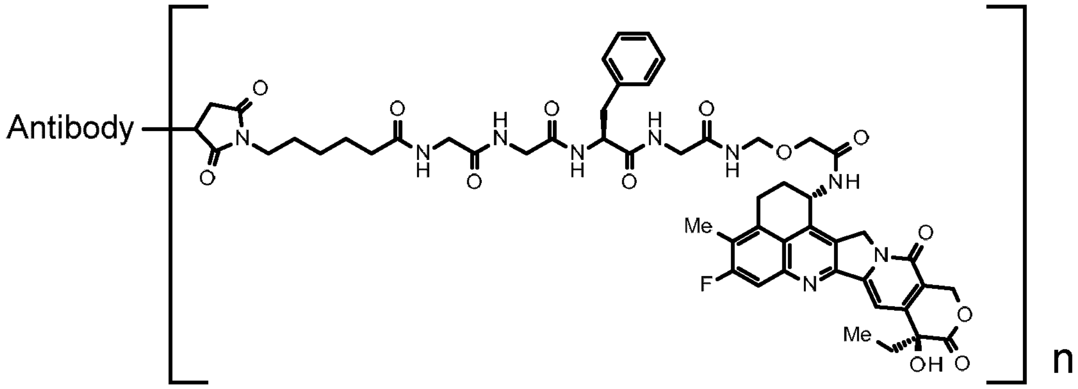

wherein, the drug-linker is conjugated to an antibody via a thioether bond. The meaning of n is the same as that of what is called the average number of conjugated drug molecules (DAR; Drug-to-Antibody Ratio), and indicates the average number of units of the drug-linker conjugated per antibody molecule.

**[0020]** After migrating into cancer cells, the antibody-drug conjugate used in the present invention is cleaved at the linker portion to release the compound represented by the following formula.

[Formula 28]

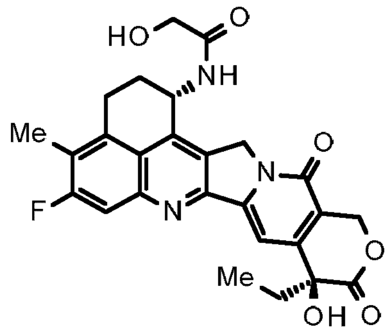

**[0021]** The aforementioned compound is inferred to be the original source of the antitumor activity of the antibody-drug conjugate used in the present invention, and has been confirmed to have a topoisomerase I inhibitory effect (Ogitani Y. et al., Clinical Cancer Research, 2016, Oct 15;22(20):5097-5108, Epub 2016 Mar 29) .

**[0022]** The antibody-drug conjugate used in the present invention is known to have a bystander effect (Ogitani Y. et al., Cancer Science (2016) 107, 1039-1046).

**[0023]** The bystander effect is exerted through a process such that the antibody-drug conjugate used in the present invention is internalized in cancer cells expressing a target and the aforementioned compound is released then exerts an antitumor effect also on cancer cells which are present therearound and not expressing the target.

**[0024]** This bystander effect is exerted as an excellent antitumor effect even when the antibody-drug conjugate is used in combination with a kinase inhibitor according to the present invention.

2. Antibody in antibody-drug conjugate

**[0025]** The antibody in the antibody-drug conjugate used in the present invention may be derived from any species, and is preferably an antibody derived from a human, a rat, a mouse, or a rabbit. In cases when the antibody is derived from species other than human species, it is preferably chimerized or humanized using a well known technique. The antibody of the present invention may be a polyclonal antibody or a monoclonal antibody and is preferably a monoclonal antibody.

**[0026]** The antibody in the antibody-drug conjugate used in the present invention is an antibody preferably having a characteristic of being capable of targeting cancer cells, and is preferably an antibody possessing, for example, a property of recognizing a cancer cell, a property of binding to a cancer cell, a property of internalizing in a cancer cell, and/or cytocidal activity against cancer cells.

**[0027]** The binding activity of the antibody against cancer cells can be confirmed using flow cytometry. The internalization of the antibody into cancer cells can be confirmed using (1) an assay of visualizing an antibody incorporated in

cells under a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may be used.

**[0028]** The antitumor activity of the antibody can be confirmed in vitro by determining inhibitory activity against cell growth. For example, a cancer cell line overexpressing a target protein for the antibody is cultured, and the antibody is added at varying concentrations into the culture system to determine inhibitory activity against focus formation, colony formation, and spheroid growth. The antitumor activity can be confirmed in vivo, for example, by administering the antibody to a nude mouse with a transplanted cancer cell line highly expressing the target protein, and determining change in the cancer cell.

**[0029]** Since the compound conjugated in the antibody-drug conjugate exerts an antitumor effect, it is preferred but not essential that the antibody itself should have an antitumor effect. For the purpose of specifically and selectively exerting the cytotoxic activity of the antitumor compound against cancer cells, it is important and also preferred that the antibody should have the property of internalizing to migrate into cancer cells.

**[0030]** The antibody in the antibody-drug conjugate used in the present invention can be obtained by a procedure known in the art. For example, the antibody of the present invention can be obtained using a method usually carried out in the art, which involves immunizing animals with an antigenic polypeptide and collecting and purifying antibodies produced in vivo. The origin of the antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an antibody applicable to a human disease.

**[0031]** Alternatively, antibody-producing cells which produce antibodies against the antigen are fused with myeloma cells according to a method known in the art (e.g., Kohler and Milstein, Nature (1975) 256, p. 495-497; and Kennet, R. ed., Monoclonal Antibodies, p. 365-367, Plenum Press, N.Y. (1980)) to establish hybridomas, from which monoclonal antibodies can in turn be obtained.

**[0032]** The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigenexpressing cells or a cell line expressing the antigen.

**[0033]** The antibody in the antibody-drug conjugate used in the present invention is preferably a recombinant antibody obtained by artificial modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using a known method.

**[0034]** As the chimeric antibody, an antibody in which antibody variable and constant regions are derived from different species, for example, a chimeric antibody in which a mouse- or rat-derived antibody variable region is connected to a human-derived antibody constant region can be exemplified (Proc. Natl. Acad. Sci. USA, 81, 6851-6855, (1984)).

**[0035]** As the humanized antibody, an antibody obtained by integrating only the complementarity determining region (CDR) of a heterologous antibody into a human-derived antibody (Nature (1986) 321, pp. 522-525), and an antibody obtained by grafting a part of the amino acid residues of the framework of a heterologous antibody as well as the CDR sequence of the heterologous antibody to a human antibody by a CDR-grafting method (International Publication No. WO 90/07861), and an antibody humanized using a gene conversion mutagenesis strategy (U.S. Patent No. 5821337) can be exemplified.

**[0036]** As the human antibody, an antibody generated by using a human antibody-producing mouse having a human chromosome fragment including genes of a heavy chain and light chain of a human antibody (see Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology:Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727, etc.) can be exemplified. As an alternative, an antibody obtained by phage display, the antibody being selected from a human antibody library (see Wormstone, I. M. et. al, Investigative Ophthalmology & Visual Science. (2002)43 (7), p.2301-2308; Carmen, S. et. al., Briefings in Functional Genomics and Proteomics (2002), 1(2), p.189-203; Siriwardena, D. et. al., Ophthalmology (2002) 109(3), p.427-431, etc.) can be exemplified.

**[0037]** In the antibody in the antibody-drug conjugate used in present invention, modified variants of the antibody are also included. The modified variant refers to a variant obtained by subjecting the antibody according to the present invention to chemical or biological modification. Examples of the chemically modified variant include variants including

a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N terminus by being expressed in a prokaryotic host cell. Further, an antibody labeled so as to enable the detection or isolation of the antibody or an antigen according to the present invention, for example, an enzyme-labeled antibody, a fluorescence-labeled antibody, and an affinity-labeled antibody are also included in the meaning of the modified variant. Such a modified variant of the antibody according to the present invention is useful for improving the stability and blood retention of the antibody, reducing the antigenicity thereof, detecting or isolating an antibody or an antigen, and so on.

**[0038]** Further, by regulating the modification of a glycan which is linked to the antibody according to the present invention (glycosylation, defucosylation, etc.), it is possible to enhance antibody-dependent cellular cytotoxic activity. As the technique for regulating the modification of a glycan of antibodies, International Publication No. WO 99/54342, International Publication No. WO 00/61739, International Publication No. WO 02/31140, International Publication No. WO 2007/133855, International Publication No. WO 2013/120066, etc. are known. However, the technique is not limited thereto. In the antibody according to the present invention, antibodies in which the modification of a glycan is regulated are also included.

**[0039]** It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (the activation of complement, antibody-dependent cellular cytotoxicity, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The type of deletion variant having a deletion at the carboxyl terminus of the heavy chain of the antibody according to the present invention is not limited to the above variants as long as the antigen-binding affinity and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions. However, an antibody in which one amino acid residue at the carboxyl terminus has been deleted in both of the two heavy chains in the antibody according to the present invention can be preferably exemplified.

**[0040]** As isotypes of the antibody according to the present invention, for example, IgG (IgG1, IgG2, IgG3, IgG4) can be exemplified. Preferably, IgG1 or IgG2 can be exemplified.

**[0041]** Examples of antibodies in the antibody-drug conjugate used in the present invention include, but are not limited to, an anti-HER2 antibody, an anti-HER3 antibody, an anti-TROP2 antibody, an anti-B7-H3 antibody, an anti-CDH6 antibody, an anti-CD3 antibody, an anti-CD30 antibody, an anti-CD33 antibody, an anti-CD37 antibody, an anti-CD56 antibody, an anti-CD98 antibody, an anti-DR5 antibody, an anti-EGFR antibody, an anti-EPHA2 antibody, an anti-FGFR2 antibody, an anti-FGFR4 antibody, an anti-FOLR1 antibody, an anti-VEGF antibody, an anti-CD20 antibody, an anti-CD22 antibody, an anti-CD70 antibody, an anti-PSMA antibody, an anti-CEA antibody, an anti-Mesothelin antibody, an anti-A33 antibody, an anti-CanAg antibody, an anti-Cripto antibody, an anti-G250 antibody, an anti-MUC1 antibody, an anti-GPNMB antibody, an anti-Integrin antibody, an anti-Tenascin-C antibody, and an anti-SLC44A4 antibody. Further an anti-HER2 antibody, an anti-HER3 antibody, an anti-TROP2 antibody, an anti-B7-H3 antibody, and an anti-CDH6 antibody can be preferably exemplified.

**[0042]** In the present invention, the term "anti-HER2 antibody" refers to an antibody which binds specifically to HER2 (Human Epidermal Growth Factor Receptor Type 2; ErbB-2), and preferably has an activity of internalization in HER2-expressing cells by binding to HER2.

**[0043]** Examples of the anti-HER2 antibody include trastuzumab (U.S. Patent No. 5821337) and pertuzumab (International Publication No. WO 01/00245). Preferably, trastuzumab can be exemplified.

**[0044]** In the present invention, the term "anti-HER3 antibody" refers to an antibody which binds specifically to HER3 (Human Epidermal Growth Factor Receptor Type 3; ErbB-3), and preferably has an activity of internalization in HER3-expressing cells by binding to HER3.

**[0045]** Examples of the anti-HER3 antibody include patritumab (U3-1287), U1-59 (International Publication No. WO 2007/077028), MM-121 (seribantumab), an anti-ERBB3 antibody described in International Publication No. WO 2008/100624, RG-7116 (lumretuzumab), and LJM-716 (elgemtumab). Preferably, patritumab and U1-59 can be exemplified.

**[0046]** In the present invention, the term "anti-TROP2 antibody" refers to an antibody which binds specifically to TROP2 (TACSTD2: Tumor-associated calcium signal transducer 2; EGP-1), and preferably has an activity of internalization in TROP2-expressing cells by binding to TROP2.

**[0047]** Examples of the anti-TROP2 antibody include hTINA1-H1L1 (International Publication No. WO 2015/098099).

**[0048]** In the present invention, the term "anti-B7-H3 antibody" refers to an antibody which binds specifically to B7-H3 (B cell antigen #7 homolog 3; PD-L3; CD276), and preferably has an activity of internalization in B7-H3-expressing cells by binding to B7-H3.

**[0049]** Examples of the anti-B7-H3 antibody include M30-H1-L4 (International Publication No. WO 2014/057687).

**[0050]** In the present invention, the term "anti-CDH6 antibody" refers to an antibody which binds specifically to CDH6 (Cadherin-6), and preferably has an activity of internalization in CDH6-expressing cells by binding to CDH6.

**[0051]** Examples of the anti-CDH6 antibody include H01L02 (International Publication No. WO 2018/212136).

3. Production of antibody-drug conjugate

**[0052]** A drug-linker intermediate for use in production of the antibody-drug conjugate according to the present invention is represented by the following formula.

[Formula 29]

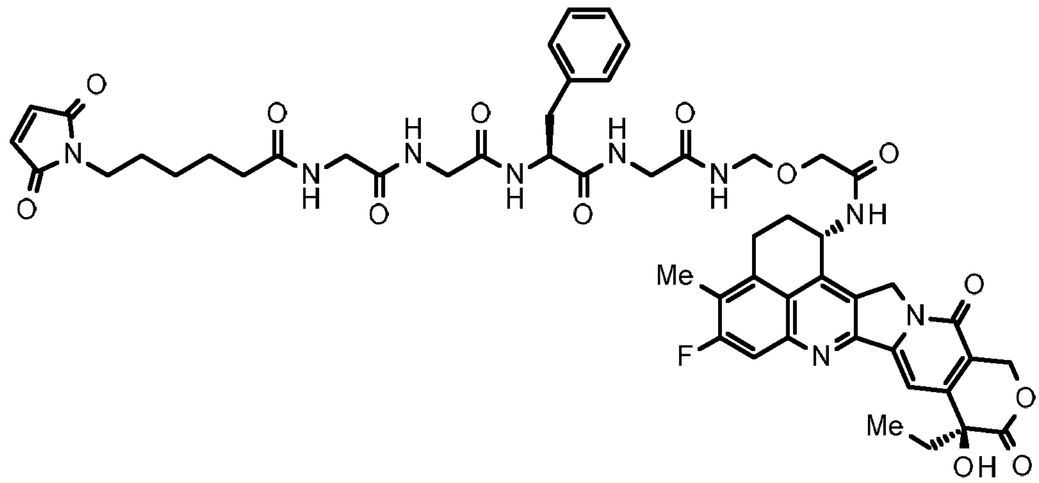

**[0053]** The drug-linker intermediate can be expressed as the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to descriptions in International Publication No. WO 2014/057687, International Publication No. WO 2015/098099, International Publication No. WO 2015/115091, International Publication No. WO 2015/155998, International Publication No. WO 2019/044947, and so on.

**[0054]** The antibody-drug conjugate used in the present invention can be produced by reacting the above-described drug-linker intermediate and an antibody having a thiol group (alternatively referred to as a sulfhydryl group).

**[0055]** The antibody having a sulfhydryl group can be obtained by a method well known in the art (Hermanson, G. T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the antibody in a buffer solution containing a chelating agent such as ethylenediamine tetraacetic acid (EDTA), an antibody having a sulfhydryl group with partially or completely reduced interchain disulfides within the antibody can be obtained.

**[0056]** Further, by using 2 to 20 molar equivalents of the drug-linker intermediate per the antibody having a sulfhydryl group, an antibody-drug conjugate in which 2 to 8 drug molecules are conjugated per antibody molecule can be produced.

**[0057]** The average number of conjugated drug molecules per antibody molecule of the antibody-drug conjugate produced can be determined, for example, by a method of calculation based on measurement of UV absorbance for the antibody-drug conjugate and the conjugation precursor thereof at two wavelengths of 280 nm and 370 nm (UV method), or a method of calculation based on quantification through HPLC measurement for fragments obtained by treating the antibody-drug conjugate with a reducing agent (HPLC method).

**[0058]** Conjugation between the antibody and the drug-linker intermediate and calculation of the average number of

conjugated drug molecules per antibody molecule of the antibody-drug conjugate can be performed with reference to descriptions in International Publication No. WO 2014/057687, International Publication No. WO 2015/098099, International Publication No. WO 2015/115091, International Publication No. WO 2015/155998, International Publication No. WO 2017/002776, International Publication No. WO 2018/212136, and so on.

**[0059]** In the present invention, the term "anti-HER2 antibody-drug conjugate" refers to an antibody-drug conjugate such that the antibody in the antibody-drug conjugate according to the present invention is an anti-HER2 antibody.

**[0060]** The anti-HER2 antibody is preferably an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2;

more preferably an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2; and

even more preferably an antibody comprising a heavy chain consisting of an amino acid sequence represented by SEQ ID NO: 1 and a light chain consisting of an amino acid sequence represented by SEQ ID NO: 2, or an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**[0061]** The average number of units of the drug-linker conjugated per antibody molecule in the anti-HER2 antibody-drug conjugate is preferably 2 to 8, more preferably 3 to 8, even more preferably 7 to 8, even more preferably 7.5 to 8, and even more preferably about 8.

**[0062]** The anti-HER2 antibody-drug conjugate used in the present invention can be produced with reference to descriptions in International Publication No. WO 2015/115091 and so on.

**[0063]** In the present invention, the term "anti-HER3 antibody-drug conjugate" refers to an antibody-drug conjugate such that the antibody in the antibody-drug conjugate according to the present invention is an anti-HER3 antibody.

**[0064]** The anti-HER3 antibody is preferably an antibody comprising a heavy chain comprising CDRH1 consisting of an amino acid sequence consisting of amino acid residues 26 to 35 of SEQ ID NO: 3, CDRH2 consisting of an amino acid sequence consisting of amino acid residues 50 to 65 of SEQ ID NO: 3, and CDRH3 consisting of an amino acid sequence consisting of amino acid residues 98 to 106 of SEQ ID NO: 3, and a light chain comprising CDRL1 consisting of an amino acid sequence consisting of amino acid residues 24 to 39 of SEQ ID NO: 4, CDRL2 consisting of an amino acid sequence consisting of amino acid residues 56 to 62 of SEQ ID NO: 4, and CDRL3 consisting of an amino acid sequence consisting of amino acid residues 95 to 103 of SEQ ID NO: 4;

more preferably an antibody comprising a heavy chain comprising a heavy chain variable region consisting of an amino acid sequence consisting of amino acid residues 1 to 117 of SEQ ID NO: 3 and a light chain comprising a light chain variable region consisting of an amino acid sequence consisting of amino acid residues 1 to 113 of SEQ ID NO: 4; and

even more preferably an antibody comprising a heavy chain consisting of an amino acid sequence represented by SEQ ID NO: 3 and a light chain consisting of an amino acid sequence represented by SEQ ID NO: 4, or a variant of the antibody in which the lysine residue at the carboxyl terminus of the heavy chain is deleted.

**[0065]** The average number of units of the drug-linker conjugated per antibody molecule in the anti-HER3 antibody-drug conjugate is preferably 2 to 8, more preferably 3 to 8, even more preferably 7 to 8, even more preferably 7.5 to 8, and even more preferably about 8.

**[0066]** The anti-HER3 antibody-drug conjugate can be produced with reference to descriptions in International Publication No. WO 2015/155998 and so on.

**[0067]** In the present invention, the term "anti-TROP2 antibody-drug conjugate" refers to an antibody-drug conjugate such that the antibody in the antibody-drug conjugate is an anti-TROP2 antibody.

**[0068]** The anti-TROP2 antibody is preferably an antibody comprising a heavy chain comprising CDRH1 consisting of an amino acid sequence consisting of amino acid residues 50 to 54 of SEQ ID NO: 5, CDRH2 consisting of an amino acid sequence consisting of amino acid residues 69 to 85 of SEQ ID NO: 5, and CDRH3 consisting of an amino acid sequence consisting of amino acid residues 118 to 129 of SEQ ID NO: 5, and a light chain comprising CDRL1 consisting

of an amino acid sequence consisting of amino acid residues 44 to 54 of SEQ ID NO: 6, CDRL2 consisting of an amino acid sequence consisting of amino acid residues 70 to 76 of SEQ ID NO: 6, and CDRL3 consisting of an amino acid sequence consisting of amino acid residues 109 to 117 of SEQ ID NO: 6;

more preferably an antibody comprising a heavy chain comprising a heavy chain variable region consisting of an amino acid sequence consisting of amino acid residues 20 to 140 of SEQ ID NO: 5 and a light chain comprising a light chain variable region consisting of an amino acid sequence consisting of amino acid residues 21 to 129 of SEQ ID NO: 6; and

even more preferably an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6, or a variant of the antibody in which the lysine residue at the carboxyl terminus of the heavy chain is deleted.

**[0069]** The average number of units of the drug-linker conjugated per antibody molecule in the anti-TROP2 antibody-drug conjugate used in the present invention is preferably 2 to 8, more preferably 3 to 5, even more preferably 3.5 to 4.5, and even more preferably about 4.

**[0070]** The anti-TROP2 antibody-drug conjugate can be produced with reference to descriptions in International Publication No. WO 2015/098099, International Publication No. WO 2017/002776, and so on.

**[0071]** In the present invention, the term "anti-B7-H3 antibody-drug conjugate" refers to an antibody-drug conjugate such that the antibody in the antibody-drug conjugate according to the present invention is an anti-B7-H3 antibody.

**[0072]** The anti-B7-H3 antibody is preferably an antibody comprising a heavy chain comprising CDRH1 consisting of an amino acid sequence consisting of amino acid residues 50 to 54 of SEQ ID NO: 7, CDRH2 consisting of an amino acid sequence consisting of amino acid residues 69 to 85 of SEQ ID NO: 7, and CDRH3 consisting of an amino acid sequence consisting of amino acid residues 118 to 130 of SEQ ID NO: 7, and a light chain comprising CDRL1 consisting of an amino acid sequence consisting of amino acid residues 44 to 53 of SEQ ID NO: 8, CDRL2 consisting of an amino acid sequence consisting of amino acid residues 69 to 75 of SEQ ID NO: 8, and CDRL3 consisting of an amino acid sequence consisting of amino acid residues 108 to 116 of SEQ ID NO: 8;

more preferably an antibody comprising a heavy chain comprising a heavy chain variable region consisting of an amino acid sequence consisting of amino acid residues 20 to 141 of SEQ ID NO: 7 and a light chain comprising a light chain variable region consisting of an amino acid sequence consisting of amino acid residues 21 to 128 of SEQ ID NO: 8; and

even more preferably an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which the lysine residue at the carboxyl terminus of the heavy chain is deleted.

**[0073]** The average number of units of the drug-linker conjugated per antibody molecule in the anti-B7-H3 antibody-drug conjugate is preferably 2 to 8, more preferably 3 to 5, even more preferably 3.5 to 4.5, and even more preferably about 4.

**[0074]** The anti-B7-H3 antibody-drug conjugate used in the present invention can be produced with reference to descriptions in International Publication No. WO 2014/057687, International Publication No. WO 2017/002776, and so on.

**[0075]** In the present invention, the term "anti-CDH6 antibody-drug conjugate" refers to an antibody-drug conjugate such that the antibody in the antibody-drug conjugate is an anti-CDH6 antibody.

**[0076]** The anti-CDH6 antibody is preferably an antibody comprising a heavy chain comprising CDRH1 consisting of an amino acid sequence consisting of amino acid residues 45 to 54 of SEQ ID NO: 9, CDRH2 consisting of an amino acid sequence consisting of amino acid residues 69 to 78 of SEQ ID NO: 9, and CDRH3 consisting of an amino acid sequence consisting of amino acid residues 118 to 130 of SEQ ID NO: 9, and a light chain comprising CDRL1 consisting of an amino acid sequence consisting of amino acid residues 44 to 54 of SEQ ID NO: 10, CDRL2 consisting of an amino acid sequence consisting of amino acid residues 70 to 76 of SEQ ID NO: 10, and CDRL3 consisting of an amino acid sequence consisting of amino acid residues 109 to 116 of SEQ ID NO: 10;

more preferably an antibody comprising a heavy chain comprising a heavy chain variable region consisting of an amino acid sequence consisting of amino acid residues 20 to 141 of SEQ ID NO: 9 and a light chain comprising a light chain variable region consisting of an amino acid sequence consisting of amino acid residues 21 to 128 of SEQ ID NO: 10; and

even more preferably an antibody comprising a heavy chain consisting of an amino acid sequence consisting of amino acid residues 20 to 471 of SEQ ID NO: 9 and a light chain consisting of an amino acid sequence consisting

of amino acid residues 21 to 233 of SEQ ID NO: 10, or a variant of the antibody in which the lysine residue at the carboxyl terminus of the heavy chain is deleted.

**[0077]** The average number of units of the drug-linker conjugated per antibody molecule in the anti-CDH6 antibody-drug conjugate used in the present invention is preferably 2 to 8, more preferably 3 to 8, even more preferably 7.5 to 8, and even more preferably about 8.

**[0078]** The anti-CDH6 antibody-drug conjugate used in the present invention can be produced with reference to descriptions in International Publication No. WO 2018/212136 and so on.

4. Kinase inhibitor

**[0079]** In the present invention, the term "kinase inhibitor" refers to an agent that inhibits a kinase involved in cancer cell growth and tumor angiogenesis. As the kinase inhibitor in the present invention, at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor can be exemplified.

**[0080]** The term "pharmacologically acceptable salt" of the kinase inhibitor used in the present invention may be either an acid addition salt or a base addition salt. Examples of the acid addition salt can include lower alkanesulfonates such as camsylate (camphorsulfonate), mesylate (methanesulfonate), trifluoromethanesulfonate, and ethanesulfonate; arylsulfonates such as tosylate (p-toluenesulfonate) and benzenesulfonate; inorganic acid salts such as phosphate, nitrate, perchlorate, and sulfate; hydrogen halide salts such as hydrochloride, hydrobromide, hydroiodide, and hydrofluoride; organic acid salts such as acetate, malate, fumarate, succinate, citrate, tartrate, oxalate, and maleate; and amino acid salts such as ornithinate, glutamate, and aspartate. Examples of the base addition salt can include alkali metal salts such as sodium salt, potassium salt, and lithium salt; alkali earth metal salts such as calcium salt and magnesium salt; inorganic salts such as ammonium salt; organic amine salts such as dibenzylamine salt, morpholine salt, phenylglycine alkyl ester salt, ethylenediamine salt, N-methylglucamine salt, diethylamine salt, triethylamine salt, cyclohexylamine salt, dicyclohexylamine salt, N,N'-dibenzylethylenediamine salt, diethanolamine salt, N-benzyl-N-(2-phenylethoxy)amine salt, piperazine salt, tetramethylammonium salt, and tris(hydroxymethyl)aminomethane salt; and amino acid salts such as alginate.

**[0081]** The kinase inhibitor and pharmacologically acceptable salts thereof used in the present invention may each exist as a solvate, and solvates of them are also included in the meaning of the kinase inhibitor and pharmacologically acceptable salts thereof used in the present invention.

**[0082]** In the present invention, the term "CDK4/6 inhibitor" refers to an agent that inhibits cyclin dependent kinase 4 (CDK4) and inhibits cyclin dependent kinase 6 (CDK6). The CDK4/6 inhibitor in the present invention may have an effect of inhibiting a kinase other than CDK4/6. The CDK4/6 inhibitor in the present invention is not particularly limited as long as it is an agent that has the described characteristics, and preferred examples thereof can include abemaciclib (U.S. Patent No. 7855211), palbociclib (U.S. Patent No. 6936612), ribociclib (U.S. Patent No. 9193732), trilaciclib (International Publication No. WO 2012/061156), G1T38 (Oncotarget 2017, 8(26): 42343-42358), PF-06873600 (International Publication No. WO 2018/033815), TP-1287 (International Publication No. WO 2016/187316), FN-1501 (J Med Chem 2018, 61(4): 1499-1518), and KRX-0601 (International Publication No. WO 1989/007105), and pharmacologically acceptable salts thereof.

**[0083]** PF-06873600 and FN-1501 and pharmacologically acceptable salts thereof are each also known as a CDK2 inhibitor. TP-1287 and pharmacologically acceptable salts thereof are each also known as a CDK1 inhibitor and a CDK2 inhibitor. KRX-0601 and pharmacologically acceptable salts thereof are each also known as a PI3K inhibitor, a CDK1 inhibitor, a CDK2 inhibitor, and a CHK1 inhibitor.

**[0084]** In the present invention, the term "mTOR inhibitor" refers to an agent that inhibits mTOR (Mammalian target of Rapamycin), one of the serine/threonine kinases. The mTOR inhibitor in the present invention may have an effect of inhibiting a kinase other than mTOR. The mTOR inhibitor in the present invention is not particularly limited as long as it is an agent that has the described characteristics, and preferred examples thereof can include everolimus (U.S. Patent No. 5665772), sirolimus (U.S. Patent No. 5989591), temsirolimus (U.S. Patent No. 5362718), TAK-228 (Cancer Chemother Pharmacol 2017, 80(2): 261-273), CC-223 (J Med Chem 2015, 58(13): 5323-5333), AZD8055 (Cancer Res 2010, 70(1): 288-98), dactolisib (Invest New Drugs 2015, 33(2): 463-71), apitolisib (International Publication No. WO 2008/070740), gedatolisib (International Publication No. WO 2009/143313), LY3023414 (Mol Cancer Ther 2016, 15(10): 2344-2356), PF-04691502 (Mol Cancer Ther 2011, 10(11): 2189-99), NVP-BGT226 (Ann Oncol 2012, 23(9): 2399-408), and PQR309 (J Med Chem 2017, 60(17): 7524-7538), and pharmacologically acceptable salts thereof.

**[0085]** Dactolisib, apitolisib, gedatolisib, LY3023414, PF-04691502, NVP-BGT226, and PQR309, and pharmacologically acceptable salts thereof are each also known as a PI3K inhibitor.

**[0086]** In the present invention, the term "PI3K inhibitor" refers to an agent that inhibits PI3K (Phosphoinositide 3-kinase), an enzyme that phosphorylates the hydroxyl group at position 3 of the inositol ring of inositol phospholipid. The PI3K inhibitor in the present invention may have an effect of inhibiting a kinase other than PI3K. The PI3K inhibitor in the present invention is not particularly limited as long as it is an agent that has the described characteristics, and preferred examples thereof can include taselisib (International Publication No. WO 2014/140073), alpelisib (International Publication No. WO 2010/029082), TAK-117 (Org Process Res Dev 2017, 21(4): 669-673), GSK2636771 (Clin Cancer Res 2017, 23(19): 5981-92), AZD8186 (J Med Chem 2015, 58(2): 943-962), IPI-549 (ACS Med Chem Lett 2016, 7(9): 862-867), idelalisib (U.S. Patent No. 9469643), duvelisib (U.S. Patent No. 8193182), AMG319 (J Med Chem 2015, 58(1): 480-511), buparlisib (International Publication No. WO 2007/084786), pictilisib (International Publication No. WO 2007/129161), pilaralisib (International Publication No. WO 2007/044729), copanlisib (U.S. Patent No. 7511041), sonolisib (International Publication No. WO 2003/024183), CH5132799 (Bioorg Med Chem Lett 2011, 21(6): 1767-1772), ZSTK474 (J Natl Cancer Inst 2006, 98(8): 545-56), GDC-0077 (International Publication No. WO 2017/001645), dactolisib, apitolisib, gedatolisib, LY3023414, PF-04691502, NVP-BGT226, PQR309, KRX-0601, and rigosertib (International Publication No. WO 2006/010152), and pharmacologically acceptable salts thereof.

**[0087]** Rigosertib and pharmacologically acceptable salts thereof are each also known as a CDK1 inhibitor and a PLK1 inhibitor.

**[0088]** The pharmacologically acceptable salt of copanlisib is preferably a hydrochloride (copanlisib hydrochloride). The pharmacologically acceptable salt of rigosertib is preferably a sodium salt (rigosertib sodium).

**[0089]** In the present invention, the term "AKT inhibitor" refers to an agent that inhibits AKT (also called protein kinase B), a serine/threonine kinase. The AKT inhibitor in the present invention may have an effect of inhibiting a kinase other than AKT. The AKT inhibitor in the present invention is not particularly limited as long as it is an agent that has the described characteristics, and preferred examples thereof can include ipatasertib (International Publication No. WO 2008/006040), uprosertib (International Publication No. WO 2008/098104), MK-2206 (Mol Cancer Ther 2010, 9(7): 1956-67), BAY1125976 (Int J Cancer 2017, 140(2): 449-454), AZD5363 (Mol Cancer Ther 2012, 11(4): 873-87), and TAS-117 (Cancer Res 2014, 74(16): 4458-69), and pharmacologically acceptable salts thereof.

**[0090]** The pharmacologically acceptable salt of ipatasertib is preferably a hydrochloride (ipatasertib hydrochloride).

**[0091]** In the present invention, the term "ERK inhibitor" refers to an agent that inhibits extracellular signal-regulated kinase (ERK). The ERK inhibitor in the present invention may have an effect of inhibiting a kinase other than ERK. The ERK inhibitor in the present invention is not particularly limited as long as it is an agent that has the described characteristics, and preferred examples thereof can include ONC201 (Oncotarget 2014, 5(24): 12728-37), BVD-523 (Mol Cancer Ther 2017, 16(11): 2351-2363), CC-90003 (International Publication No. WO 2014/124230), GDC-0994 (J Med Chem 2016, 59(12): 5650-5660), LY3214996 (International Publication No. WO 2016/106029), and MK-8353 (ACS Med Chem Lett 2018, 9(7): 761-767), and pharmacologically acceptable salts thereof.

**[0092]** In the present invention, the term "MEK inhibitor" refers to an agent that inhibits mitogen-activated extracellular signal-regulated kinase (MEK). The MEK inhibitor in the present invention may have an effect of inhibiting a kinase other than MEK. The MEK inhibitor in the present invention is not particularly limited as long as it is an agent that has the described characteristics, and preferred examples thereof can include trametinib (U.S. Patent No. 7378423), binimetinib (U.S. Patent No. 7777050), selumetinib (International Publication No. WO 2005/023251), refametinib (International Publication No. WO 2007/014011), pimasertib (International Publication No. WO 2006/045514), cobimetinib (U.S. Patent No. 7803839), E6201 (J Pharmacol Exp Ther 2009, 331(2): 485-95), PD-0325901 (Clin Cancer Res 2010, 16(8): 2450-7), RO5126766 (ACS Med Chem Lett 2014, 5(4): 309-14), and GDC-0623 (Bioorg Med Chem Lett 2014, 24(19): 4714-4723), and pharmacologically acceptable salts thereof.

**[0093]** Trametinib is preferably a dimethyl sulfoxide adduct (trametinib dimethyl sulfoxide).

**[0094]** In the present invention, the term "RAF inhibitor" refers to an agent that inhibits the kinase activity of RAF (preferably, BRAF). The RAF inhibitor in the present invention may have an effect of inhibiting a kinase other than RAF. The RAF inhibitor in the present invention is not particularly limited as long as it is an agent that has the described characteristics, and preferred examples thereof can include regorafenib (U.S. Patent No. 7351834), sorafenib (U.S. Patent No. 7235576), vemurafenib (U.S. Patent No. 7504509), dabrafenib (U.S. Patent No. 7994185), encorafenib (U.S. Patent No. 8501758), RAF265 (ACS Med Chem Lett 2015, 6(9): 961-5), GDC-5573 (International Publication No. WO 2013/100632), LY3009120 (J Med Chem 2015, 58(10): 4165-4179), and RO5126766, and pharmacologically acceptable salts thereof.

**[0095]** Regorafenib and pharmacologically acceptable salts thereof are each also known as a VEGFR inhibitor, a KIT inhibitor, an RET inhibitor, a PDGFR inhibitor, and an FGFR inhibitor. Sorafenib and pharmacologically acceptable salts thereof are each also known as a VEGFR inhibitor, a KIT inhibitor, an RET inhibitor, a PDGFR inhibitor, an FGFR inhibitor, and an FLT3 inhibitor.

**[0096]** Regorafenib is preferably a hydrate (regorafenib hydrate). The pharmacologically acceptable salt of sorafenib is preferably a tosylate (sorafenib tosylate). The pharmacologically acceptable salt of dabrafenib is preferably a mesylate

(dabrafenib mesylate).

**[0097]** In the present invention, the term "CDK1 inhibitor" refers to an agent that inhibits cyclin dependent kinase 1 (CDK1). The CDK1 inhibitor in the present invention may have an effect of inhibiting a kinase other than CDK1. The CDK1 inhibitor in the present invention is not particularly limited as long as it is an agent that has the described characteristics, and preferred examples thereof can include dinaciclib (International Publication No. WO 2005/077954), milciclib (J Med Chem 2009, 52(16): 5152-63), seliciclib (International Publication No. WO 1997/020842), alvocidib (International Publication No. WO 2001/053293), roniciclib (International Publication No. WO 2005/037800), voruciclib (International Publication No. WO 2007/148158), AT7519 (J Med Chem 2008, 51(16): 4986-4999), TP-1287, PHA-793887 (Bioorg Chem 2010, 18(5): 1844-53), KRX-0601, and rigosertib, and pharmacologically acceptable salts thereof.

**[0098]** Dinaciclib, seliciclib, alvocidib, voruciclib, AT7519, and PHA-793887 and pharmacologically acceptable salts thereof are each also known as a CDK2 inhibitor. Milciclib and pharmacologically acceptable salts thereof are each also known as a CDK2 inhibitor and a WEE1 inhibitor. Roniciclib and pharmacologically acceptable salts thereof are each also known as a CDK2 inhibitor and an Aurora kinase inhibitor.

**[0099]** In the present invention, the term "CDK2 inhibitor" refers to an agent that inhibits cyclin dependent kinase 2 (CDK2). The CDK2 inhibitor in the present invention may have an effect of inhibiting a kinase other than CDK2. The CDK2 inhibitor in the present invention is not particularly limited as long as it is an agent that has the described characteristics, and preferred examples thereof can include CYC-065, PF-06873600, FN-1501, KRX-0601, dinaciclib, seliciclib, alvocidib, voruciclib, AT7519, PHA-793887, and roniciclib, and pharmacologically acceptable salts thereof.

**[0100]** In the present invention, the term "CHK1 inhibitor" refers to an agent that inhibits checkpoint kinase 1 (CHK1). The CHK1 inhibitor in the present invention may have an effect of inhibiting a kinase other than CHK1. The CHK1 inhibitor in the present invention is not particularly limited as long as it is an agent that has the described characteristics, and preferred examples thereof can include MK-8776 (Bioorg Med Chem Lett 2011, 21(1): 471-4), LY2606368 (Mol Cancer Ther 2015, 14(9): 2004-13), LY2603618 (Invest New Drugs 2013, 31(1): 136-44), CBP501 (Mol Cancer Ther 2007, 6(1): 147-53), GDC-0425 (Org Process Res Dev 2015, 19(6): 661-672), CCT245737 (Oncotarget 2016, 7(3): 2329-42), and KRX-0601, and pharmacologically acceptable salts thereof.

**[0101]** In the present invention, the term "WEE1 inhibitor" refers to an agent that inhibits Wee1-like protein kinase. The WEE1 inhibitor in the present invention may have an effect of inhibiting a kinase other than WEE1. The WEE1 inhibitor in the present invention is not particularly limited as long as it is an agent that has the described characteristics, and preferred examples thereof can include milciclib and MK-1775 (Mol Cancer Ther 2009, 8(11): 2992-3000), and pharmacologically acceptable salts thereof.

**[0102]** In the present invention, the term "PLK1 inhibitor" refers to an agent that inhibits Polo-like kinase 1 (PLK1). The PLK1 inhibitor in the present invention may have an effect of inhibiting a kinase other than PLK1. The PLK1 inhibitor in the present invention is not particularly limited as long as it is an agent that has the described characteristics, and preferred examples thereof can include rigosertib and volasertib (International Publication No. WO 2012/049153), and pharmacologically acceptable salts thereof.

**[0103]** In the present invention, the term "Aurora kinase inhibitor" refers to an agent that inhibits Aurora kinase. The Aurora kinase inhibitor in the present invention may have an effect of inhibiting a kinase other than Aurora kinase. The Aurora kinase inhibitor in the present invention is not particularly limited as long as it is an agent that has the described characteristics, and preferred examples thereof can include alisertib (International Publication No. WO 2005/111039), ilorasertib (International Publication No. WO 2010/065825), ENMD-2076 (Clin Cancer Res 2011, 17(4): 849-60), AMG900 (Cancer Res 2010, 70(23): 9846-54), and roniciclib, and pharmacologically acceptable salts thereof.

**[0104]** Ilorasertib and pharmacologically acceptable salts thereof are each also known as a VEGFR inhibitor, a KIT inhibitor, a PDGFR inhibitor, an FLT3 inhibitor, and a CSF-1R inhibitor. ENMD-2076 is also known as a VEGFR inhibitor and an FLT3 inhibitor.

**[0105]** In the present invention, the term "Bcr-Abl inhibitor" refers to an agent that inhibits the tyrosine kinase Bcr-Abl. The Bcr-Abl inhibitor in the present invention may have an effect of inhibiting a kinase other than Bcr-Abl. The Bcr-Abl inhibitor in the present invention is not particularly limited as long as it is an agent that has the described characteristics, and preferred examples thereof can include imatinib (U.S. Patent No. 6894051), dasatinib (U.S. Patent No. 6596746), bosutinib (U.S. Patent No. 6002008), nilotinib (U.S. Patent No. 7169791), and ponatinib (U.S. Patent No. 8114874), and pharmacologically acceptable salts thereof.

**[0106]** Imatinib and nilotinib and pharmacologically acceptable salts thereof are each also known as a KIT inhibitor and a PDGFR inhibitor. Dasatinib and pharmacologically acceptable salts thereof are each also known as an Src inhibitor, a KIT inhibitor, and a PDGFR inhibitor. Bosutinib and pharmacologically acceptable salts thereof are each also known as an Src inhibitor. Ponatinib and pharmacologically acceptable salts thereof are each also known as an EPH inhibitor, a VEGFR inhibitor, a KIT inhibitor, an RET inhibitor, a PDGFR inhibitor, an FGFR inhibitor, and an FLT3 inhibitor.

**[0107]** The pharmacologically acceptable salt of imatinib is preferably mesylate (imatinib mesylate). Dasatinib is preferably a hydrate (dasatinib hydrate). Bosutinib is preferably a hydrate (bosutinib hydrate). The pharmacologically acceptable salt of nilotinib is preferably a hydrochloride hydrate (nilotinib hydrochloride hydrate). The pharmacologically

acceptable salt of ponatinib is preferably a hydrochloride (ponatinib hydrochloride).

**[0108]** In the present invention, the term "Src inhibitor" refers to an agent that inhibits the protooncogene tyrosine protein kinase Src. The Src inhibitor in the present invention is not particularly limited as long as it is an agent that has the described characteristics, and preferred examples thereof can include dasatinib and bosutinib, and pharmacologically acceptable salts thereof.

**[0109]** In the present invention, the term "EPH inhibitor" refers to an agent that inhibits tyrosine kinase of an erythropoietin-producing hepatocellular receptor (EPH). The EPH inhibitor in the present invention is not particularly limited as long as it is an agent that has the described characteristics, and preferred examples thereof can include dasatinib and ponatinib, and pharmacologically acceptable salts thereof.

**[0110]** In the present invention, the term "VEGFR inhibitor" refers to an agent that inhibits tyrosine kinase of a vascular endothelial growth factor receptor (VEGFR). The VEGFR inhibitor in the present invention may have an effect of inhibiting a kinase other than VEGFR tyrosine kinase. The VEGFR inhibitor in the present invention is not particularly limited as long as it is an agent that has the described characteristics, and preferred examples thereof can include regorafenib, sorafenib, vandetanib (U.S. Patent No. 7173038), sunitinib (U.S. Patent No. 6573293), axitinib (U.S. Patent No. 6534524), pazopanib (U.S. Patent No. 7105530), lenvatinib (U.S. Patent No. 7253286), nintedanib (hereinafter, also referred to as "BIBF1120") (U.S. Patent No. 6762180), cabozantinib (U.S. Patent No. 7579473), tivozanib (International Publication No. WO 2002/088110), brivanib (International Publication No. WO 2004/009784), linifanib (International Publication No. WO 2014/022975), lucitanib (International Publication No. WO 2008/112408), ilorasertib, and ENMD-2076, and pharmacologically acceptable salts thereof.

**[0111]** Vandetanib and pharmacologically acceptable salts thereof are each also known as an RET inhibitor. Sunitinib and pharmacologically acceptable salts thereof are each also known as a KIT inhibitor, an RET inhibitor, a PDGFR inhibitor, an FLT3 inhibitor, and a CSF-1R inhibitor. Axitinib and pharmacologically acceptable salts thereof are each also known as a PDGFR inhibitor, an FGFR inhibitor, and a CSF-1R inhibitor. Pazopanib and pharmacologically acceptable salts thereof are each also known as a KIT inhibitor, a PDGFR inhibitor, and an FGFR inhibitor. Lenvatinib and pharmacologically acceptable salts thereof are each also known as a KIT inhibitor, an RET inhibitor, a PDGFR inhibitor, and an FGFR inhibitor. Nintedanib and pharmacologically acceptable salts thereof are each also known as a PDGFR inhibitor and an FGFR inhibitor.

**[0112]** The pharmacologically acceptable salt of sunitinib is preferably a malate (sunitinib malate). The pharmacologically acceptable salt of pazopanib is preferably a hydrochloride (pazopanib hydrochloride). The pharmacologically acceptable salt of lenvatinib is preferably a mesylate (lenvatinib mesylate). The pharmacologically acceptable salt of nintedanib is preferably an ethanesulfonate (nintedanib ethanesulfonate). The pharmacologically acceptable salt of cabozantinib is preferably a malate (cabozantinib malate). The pharmacologically acceptable salt of tivozanib is preferably a hydrochloride hydrate (tivozanib hydrochloride hydrate).

**[0113]** In the present invention, the term "KIT inhibitor" refers to an agent that inhibits tyrosine kinase of the cytokine receptor KIT (c-kit, or also called CD117). The KIT inhibitor in the present invention may have an effect of inhibiting a kinase other than KIT tyrosine kinase. The KIT inhibitor in the present invention is not particularly limited as long as it is an agent that has the described characteristics, and preferred examples thereof can include regorafenib, sorafenib, imatinib, ilorasertib, sunitinib, pazopanib, lenvatinib, and dasatinib, and pharmacologically acceptable salts thereof.

**[0114]** In the present invention, the term "RET inhibitor" refers to an agent that inhibits RET (Rearranged during transfection) tyrosine kinase. The RET inhibitor in the present invention may have an effect of inhibiting a kinase other than RET tyrosine kinase. The RET inhibitor in the present invention is not particularly limited as long as it is an agent that has the described characteristics, and preferred examples thereof can include regorafenib, sorafenib, vandetanib, lenvatinib, and sunitinib, and pharmacologically acceptable salts thereof.

**[0115]** In the present invention, the term "PDGFR inhibitor" refers to an agent that inhibits tyrosine kinase of a platelet-derived growth factor receptor (PDGFR). The PDGFR inhibitor in the present invention may have an effect of inhibiting a kinase other than PDGFR tyrosine kinase. The PDGFR inhibitor in the present invention is not particularly limited as long as it is an agent that has the described characteristics, and preferred examples thereof can include regorafenib, sorafenib, sunitinib, axitinib, pazopanib, lenvatinib, nintedanib, ilorasertib, imatinib, nilotinib, and dasatinib, and pharmacologically acceptable salts thereof.

**[0116]** In the present invention, the term "FGFR inhibitor" refers to an agent that inhibits tyrosine kinase of a fibroblast growth factor receptor (FGFR). The FGFR inhibitor in the present invention may have an effect of inhibiting a kinase other than FGFR tyrosine kinase. The FGFR inhibitor in the present invention is not particularly limited as long as it is an agent that has the described characteristics, and preferred examples thereof can include regorafenib, sorafenib, lenvatinib, nintedanib, axitinib, and pazopanib, and pharmacologically acceptable salts thereof.

**[0117]** In the present invention, the term "BTK inhibitor" refers to an agent that inhibits Bruton's tyrosine kinase (BTK). The BTK inhibitor in the present invention may have an effect of inhibiting a kinase other than BTK. The BTK inhibitor in the present invention is not particularly limited as long as it is an agent that has the described characteristics, and preferred examples thereof can include ibrutinib (U.S. Patent No. 7514444), acalabrutinib (U.S. Patent No. 9290504),

and tirabrutinib (International Publication No. WO 2011/152351), and pharmacologically acceptable salts thereof.

**[0118]** In the present invention, the term "FLT3 inhibitor" refers to an agent that inhibits FMS-like tyrosine kinase 3 (FLT3). The FLT3 inhibitor in the present invention may have an effect of inhibiting a kinase other than FLT3. The FLT3 inhibitor in the present invention is not particularly limited as long as it is an agent that has the described characteristics, and preferred examples thereof can include gilteritinib (U.S. Patent No. 7514444), quizartinib (J Med Chem 2009, 52(23): 7808-7816), midostaurin (International Publication No. WO 2003/037347), sorafenib, ilorasertib, ENMD-2076, and sunitinib, and pharmacologically acceptable salts thereof.

**[0119]** The pharmacologically acceptable salt of gilteritinib is preferably a fumarate (gilteritinib fumarate).

**[0120]** In the present invention, the term "ALK inhibitor" refers to an agent that inhibits anaplastic lymphoma kinase (ALK). The ALK inhibitor in the present invention may have an effect of inhibiting a kinase other than ALK. The ALK inhibitor in the present invention is not particularly limited as long as it is an agent that has the described characteristics, and preferred examples thereof can include brigatinib (U.S. Patent No. 9012462), crizotinib (U.S. Patent No. 7858643), ceritinib (U.S. Patent No. 7153964), alectinib (U.S. Patent No. 9126931), and lorlatinib (U.S. Patent No. 8680111), and pharmacologically acceptable salts thereof.

**[0121]** The pharmacologically acceptable salt of alectinib is preferably a hydrochloride (alectinib hydrochloride).

**[0122]** In the present invention, the term "JAK inhibitor" refers to an agent that inhibits Janus kinase (JAK). The JAK inhibitor in the present invention may have an effect of inhibiting a kinase other than JAK. The JAK inhibitor in the present invention is not particularly limited as long as it is an agent that has the described characteristics, and preferred examples thereof can include ruxolitinib (U.S. Patent No. 7598257), tofacitinib (U.S. Patent No. 7265221), baricitinib (U.S. Patent No. 8158616), and pacritinib (International Publication No. WO 2007/058627), and pharmacologically acceptable salts thereof.

**[0123]** The pharmacologically acceptable salt of ruxolitinib is preferably a phosphate (ruxolitinib phosphate). The pharmacologically acceptable salt of tofacitinib is preferably a citrate (tofacitinib citrate).

**[0124]** In the present invention, the term "MET inhibitor" refers to an agent that inhibits MET (also referred to as "c-Met"), a receptor-type tyrosine kinase whose ligand is a hepatocyte growth factor (HGF). The MET inhibitor in the present invention may have an effect of inhibiting a kinase other than MET. The MET inhibitor in the present invention is not particularly limited as long as it is an agent that has the described characteristics, and preferred examples thereof can include capmatinib (International Publication No. WO 2008/064157) and tepotinib (International Publication No. WO 2009/006959), and pharmacologically acceptable salts thereof.

**[0125]** In the present invention, the term "CSF-1R inhibitor" refers to an agent that inhibits tyrosine kinase of a colony-stimulating factor-1 receptor (CSF-1R). The CSF-1R inhibitor in the present invention may have an effect of inhibiting a kinase other than tyrosine kinase of CSF-1R. The CSF-1R inhibitor in the present invention is not particularly limited as long as it is an agent that has the described characteristics, and preferred examples thereof can include pexidartinib (Clin Cancer Res 2014, 20(12): 3146-58), BLZ-945 (International Publication No. WO 2007/121484), JNJ-40346527 (International Publication No. WO 2009/052237), JNJ-28312141 (J Med Chem 2011, 54(22): 7860-7883), ilorasertib, imatinib, sunitinib, and axitinib, and pharmacologically acceptable salts thereof.

**[0126]** The pharmacologically acceptable salt of pexidartinib is preferably a hydrochloride (pexidartinib hydrochloride).

**[0127]** In the present invention, the term "NTRK inhibitor" refers to an agent that inhibits neurotrophic tropomyosin receptor kinase (TRK). The NTRK inhibitor in the present invention may have an effect of inhibiting a kinase other than TRK. The NTRK inhibitor in the present invention is not particularly limited as long as it is an agent that has the described characteristics, and preferred examples thereof can include entrectinib (International Publication No. WO 2009/013126), and GR-389988 (International Publication No. WO 2015/089139), and pharmacologically acceptable salts thereof.

**[0128]** In the present invention, the term "EGFR inhibitor" refers to an agent that inhibits tyrosine kinase of an epidermal growth factor receptor (EGFR). The EGFR inhibitor in the present invention may have an effect of inhibiting a kinase other than tyrosine kinase of EGFR. The EGFR inhibitor in the present invention is not particularly limited as long as it is an agent that has the described characteristics, and preferred examples thereof can include gefitinib (International Publication No. WO 1996/033980), erlotinib (International Publication No. WO 1996/030347), afatinib (International Publication No. WO 2002/050043), osimertinib (International Publication No. WO 2013/014448), dacomitinib (U.S. Patent No. 7772243), lapatinib (U.S. Patent No. 713485), neratinib (J Med Chem 2005, 48(4): 1107-1131), pyrotinib (International Publication No. WO 2011/029265), and poziotinib (International Publication No. WO 2014/116070), and pharmacologically acceptable salts thereof.

**[0129]** In the present invention, the term "HER2 inhibitor" refers to an agent that inhibits tyrosine kinase of HER2 (human epidermal growth factor receptor type 2; ErbB-2). The HER2 inhibitor in the present invention may have an effect of inhibiting a kinase other than tyrosine kinase of HER2. The HER2 inhibitor in the present invention is not particularly limited as long as it is an agent that has the described characteristics, and preferred examples thereof can include tucatinib (International Publication No. WO 2013/056183), neratinib, mubritinib (International Publication No. WO 2001/077107), lapatinib, pyrotinib, and poziotinib, and pharmacologically acceptable salts thereof.

5. Medicament

**[0130]** Described in the following are a pharmaceutical composition and a method of treatment wherein the antibody-drug conjugate according to the present invention and a kinase inhibitor are administered in combination.

**[0131]** The pharmaceutical composition and method of treatment of the present invention may be characterized in that the antibody-drug conjugate and the kinase inhibitor are separately contained as active components in different formulations, and are administered simultaneously or at different times, or characterized in that the antibody-drug conjugate and the kinase inhibitor are contained as active components in a single formulation and administered.

**[0132]** In the pharmaceutical composition and method of treatment of the present invention, two or more of the kinase inhibitors used in the present invention can be administered in combination.

**[0133]** The pharmaceutical composition and method of treatment of the present invention can be used for treating cancer, and can be preferably used for treating at least one cancer selected from the group consisting of breast cancer (including triple-negative breast cancer and luminal breast cancer), gastric cancer (also called gastric adenocarcinoma), colorectal cancer (also called colon and rectal cancer, and including colon cancer and rectal cancer), lung cancer (including small cell lung cancer and non-small cell lung cancer), esophageal cancer, head-and-neck cancer (including salivary gland cancer and pharyngeal cancer), gastroesophageal junction adenocarcinoma, biliary tract cancer (including bile duct cancer), Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma, and can be more preferably used for treating at least one cancer selected from the group consisting of breast cancer, gastric cancer, colorectal cancer, lung cancer, pancreatic cancer, kidney cancer, and ovarian cancer.

**[0134]** Among the antibody-drug conjugates used in the present invention, the kind of antibody preferably used in the antibody-drug conjugate can be determined by examining the type of cancer and tumor markers. For example, if HER2 expression is found in the cancer, an anti-HER2 antibody-drug conjugate can be preferably used; if HER3 expression is found in the cancer, an anti-HER3 antibody-drug conjugate can be preferably used; if TROP2 expression is found in the cancer, an anti-TROP2 antibody-drug conjugate can be preferably used; if B7-H3 expression is found in the cancer, an anti-B7-H3 antibody-drug conjugate can be preferably used; and if CDH6 expression is found in the cancer, an anti-CDH6 antibody-drug conjugate can be preferably used.

**[0135]** The presence or absence of HER2, HER3, TROP2, B7-H3, and CDH6, and other tumor markers can be checked by, for example, collecting tumor tissue from a cancer patient, and subjecting the formalin-fixed paraffin-embedded specimen (FFPE) to an examination at a gene product (protein) level, such as an immunohistochemistry (IHC) method, a flow cytometry, a western blot method, or an examination at a gene transcription level such as an in situ hybridization method (ISH), a quantitative PCR method (q-PCR), or a microarray analysis; alternatively, it can also be checked by collecting cell-free blood circulating tumor DNA (ctDNA) from a cancer patient and subjecting it to an examination which uses a method such as next generation sequencing (NGS).

**[0136]** The pharmaceutical composition and method of treatment of the present invention can be preferably used for a mammal, and can be more preferably used for a human.

**[0137]** The antitumor effect of the pharmaceutical composition and method of treatment of the present invention can be confirmed by, for example, generating a model in which cancer cells are transplanted to a test animal, and measuring reduction in tumor volume or lifeprolonging effects due to applying the pharmaceutical composition and method of treatment of the present invention. Furthermore, comparison with the antitumor effect of single administrations of each of the antibody-drug conjugate and the kinase inhibitor used in the present invention can provide confirmation of the combined effect of the antibody-drug conjugate and the kinase inhibitor used in the present invention.

**[0138]** In addition, the antitumor effect of the pharmaceutical composition and method of treatment of the present invention can be confirmed, in a clinical study, with the Response Evaluation Criteria in Solid Tumors (RECIST) evaluation method, WHO's evaluation method, Macdonald's evaluation method, measurement of body weight, and other methods; and can be determined by indicators such as Complete response (CR), Partial response (PR), Progressive disease (PD), Objective response rate (ORR), Duration of response (DoR), Progression-free survival (PFS), and Overall survival (OS).

**[0139]** The foregoing methods can provide confirmation of superiority in terms of the antitumor effect of the pharmaceutical composition and method of treatment of the present invention compared to existing pharmaceutical compositions and methods of treatment for cancer therapy.

**[0140]** The pharmaceutical composition and method of treatment of the present invention can retard growth of cancer cells, suppress their proliferation, and further can kill cancer cells. These effects can allow cancer patients to be free from symptoms caused by cancer or can achieve an improvement in the QOL of cancer patients and attain a therapeutic effect by sustaining the lives of the cancer patients. Even if the pharmaceutical composition and method of treatment of the present invention do not accomplish the killing of cancer cells, they can achieve higher QOL of cancer patients while

achieving longer-term survival, by inhibiting or controlling the growth of cancer cells.

**[0141]** The pharmaceutical composition of the present invention can be expected to exert a therapeutic effect by application as systemic therapy to patients, and additionally, by local application to cancer tissues.

**[0142]** The pharmaceutical composition of the present invention may be administered as a pharmaceutical composition containing at least one pharmaceutically suitable ingredient. The pharmaceutically suitable ingredient can be suitably selected and applied from formulation additives or the like that are generally used in the art, in view of the dosage, administration concentration, or the like of the antibody-drug conjugate and the kinase inhibitor used in the present invention. For example, the antibody-drug conjugate used in the present invention may be administered as a pharmaceutical composition containing a buffer such as histidine buffer, an excipient such as sucrose and trehalose, and a surfactant such as Polysorbates 80 and 20. The pharmaceutical composition containing the antibody-drug conjugate used in the present invention can be preferably used as an injection, can be more preferably used as an aqueous injection or a lyophilized injection, and can be even more preferably used as a lyophilized injection.

**[0143]** In the case that the pharmaceutical composition containing the antibody-drug conjugate used in the present invention is an aqueous injection, the aqueous injection can be preferably diluted with a suitable diluent and then given as an intravenous infusion. For the diluent, a dextrose solution, physiological saline, and the like, can be exemplified, and a dextrose solution can be preferably exemplified, and a 5% dextrose solution can be more preferably exemplified.

**[0144]** In the case that the pharmaceutical composition containing the antibody-drug conjugate used in the present invention is a lyophilized injection, it can be preferably dissolved in water for injection, subsequently a required amount can be diluted with a suitable diluent and then given as an intravenous infusion. For the diluent, a dextrose solution, physiological saline, and the like, can be exemplified, and a dextrose solution can be preferably exemplified, and a 5% dextrose solution can be more preferably exemplified.

**[0145]** Examples of the administration route which may be used to administer the pharmaceutical composition of present invention include intravenous, intradermal, subcutaneous, intramuscular, and intraperitoneal routes; and preferably include an intravenous route.

**[0146]** The antibody-drug conjugate used in the present invention can be administered to a human once at intervals of 1 to 180 days, and can be preferably administered once a week, once every 2 weeks, once every 3 weeks, or once every 4 weeks, and can be even more preferably administered once every 3 weeks. Also, the antibody-drug conjugate used in the present invention can be administered at a dose of about 0.001 to 100 mg/kg, and can be preferably administered at a dose of 0.8 to 12.4 mg/kg. In the case that the antibody-drug conjugate used in the present invention is an anti-HER2 antibody-drug conjugate, it can be preferably administered once every 3 weeks at a dose of 0.8 mg/kg, 1.6 mg/kg, 3.2 mg/kg, 5.4 mg/kg, 6.4 mg/kg, 7.4 mg/kg, or 8 mg/kg. In the case that the antibody-drug conjugate used in the present invention is an anti-HER3 antibody-drug conjugate, it can be preferably administered once every 3 weeks at a dose of 1.6 mg/kg, 3.2 mg/kg, 4.8 mg/kg, 6.4 mg/kg, 8.0 mg/kg, 9.6 mg/kg, or 12.8 mg/kg. In the case that the antibody-drug conjugate used in the present invention is an anti-TROP2 antibody-drug conjugate, it can be preferably administered once every 3 weeks at a dose of 0.27 mg/kg, 0.5 mg/kg, 1.0 mg/kg, 2.0 mg/kg, 4.0 mg/kg, 6.0 mg/kg, or 8.0 mg/kg.

**[0147]** The kinase inhibitor according to the present invention can be orally administered to a human once or twice at intervals of 1 to 7 days, and can be preferably orally administered once a day or twice per day. Also, the kinase inhibitor used in the present invention can be orally administered at a dose of 0.1 mg to 3000 mg, and can be preferably administered at a dose of 2.5 mg to 600 mg. Furthermore, the kinase inhibitor used in the present invention can be administered to a human as an intravenous drip once at intervals of 1 to 180 days, and can be preferably administered as an intravenous drip once a week, once every 2 weeks, once every 3 weeks, or once every 4 weeks. Also, the kinase inhibitor used in the present invention can be administered as an intravenous drip at a dose of 0.1 mg to 3000 mg, and can be preferably administered as an intravenous drip at a dose of 10 mg to 100 mg.

**[0148]** In the case that the kinase inhibitor used in the present invention is abemaciclib or a pharmacologically acceptable salt thereof, the kinase inhibitor can be preferably orally administered twice per day at a dose of 50 mg, 100 mg, 150 mg, or 200 mg.

**[0149]** In the case that the kinase inhibitor used in the present invention is palbociclib or a pharmacologically acceptable salt thereof, the kinase inhibitor can be preferably orally administered once a day at a dose of 75 mg, 100 mg, or 125 mg.

**[0150]** In the case that the kinase inhibitor used in the present invention is ribociclib or a pharmacologically acceptable salt thereof, the kinase inhibitor can be preferably orally administered once a day at a dose of 200 mg, 400 mg, or 600 mg.

**[0151]** In the case that the kinase inhibitor used in the present invention is everolimus or a pharmacologically acceptable salt thereof, the kinase inhibitor can be preferably orally administered once a day at a dose of 2.5 mg, 5 mg, 7.5 mg, 10 mg, 15 mg, or 20 mg.

**[0152]** In the case that the kinase inhibitor used in the present invention is sirolimus or a pharmacologically acceptable salt thereof, the kinase inhibitor can be preferably orally administered once a day at a dose of 0.5 mg, 1 mg, 1.5 mg, 2 mg, 3 mg, 4 mg, 5 mg, 6 mg, 8 mg, 10 mg, 12 mg, or 15 mg.

**[0153]** In the case that the kinase inhibitor used in the present invention is temsirolimus or a pharmacologically acceptable salt thereof, the kinase inhibitor can be preferably administered as an intravenous drip once a week at a dose of 25 mg.

**[0154]** In the case that the kinase inhibitor used in the present invention is copanlisib or a pharmacologically acceptable salt thereof, the kinase inhibitor can be preferably administered as an intravenous drip once with intervals of 1 to 3 weeks at a dose of 60 mg.

**[0155]** In the case that the kinase inhibitor used in the present invention is idelalisib or a pharmacologically acceptable salt thereof, the kinase inhibitor can be preferably orally administered twice per day at a dose of 100 mg or 150 mg.

**[0156]** In the case that the kinase inhibitor used in the present invention is duvelisib or a pharmacologically acceptable salt thereof, the kinase inhibitor can be preferably orally administered twice per day at a dose of 15 mg or 25 mg.

**[0157]** In the case that the kinase inhibitor used in the present invention is trametinib or a pharmacologically acceptable salt thereof, the kinase inhibitor can be preferably orally administered once a day at a dose of 0.5 mg, 1 mg, 1.5 mg, or 2 mg.

**[0158]** In the case that the kinase inhibitor used in the present invention is binimetinib or a pharmacologically acceptable salt thereof, the kinase inhibitor can be preferably orally administered twice per day at a dose of 15 mg, 30 mg, or 45 mg.

**[0159]** In the case that the kinase inhibitor used in the present invention is cobimetinib or a pharmacologically acceptable salt thereof, the kinase inhibitor can be preferably orally administered once a day at a dose of 20 mg, 40 mg, or 60 mg.

**[0160]** In the case that the kinase inhibitor used in the present invention is regorafenib or a pharmacologically acceptable salt thereof, the kinase inhibitor can be preferably orally administered once a day at a dose of 40 mg, 80 mg, 120 mg, or 160 mg.

**[0161]** In the case that the kinase inhibitor used in the present invention is sorafenib or a pharmacologically acceptable salt thereof, the kinase inhibitor can be preferably orally administered twice per day at a dose of 200 mg or 400 mg.

**[0162]** In the case that the kinase inhibitor used in the present invention is vemurafenib or a pharmacologically acceptable salt thereof, the kinase inhibitor can be preferably orally administered twice per day at a dose of 240 mg, 480 mg, 720 mg, or 960 mg.

**[0163]** In the case that the kinase inhibitor used in the present invention is dabrafenib or a pharmacologically acceptable salt thereof, the kinase inhibitor can be preferably orally administered once a day at a dose of 50 mg, 75 mg, 100 mg, 125 mg, or 150 mg.

**[0164]** In the case that the kinase inhibitor used in the present invention is encorafenib or a pharmacologically acceptable salt thereof, the kinase inhibitor can be preferably orally administered once a day at a dose of 50 mg, 75 mg, 100 mg, 125 mg, 150 mg, 175 mg, 200 mg, 250 mg, 300 mg, 350 mg, 400 mg, or 450 mg.

**[0165]** In the case that the kinase inhibitor used in the present invention is imatinib or a pharmacologically acceptable salt thereof, the kinase inhibitor can be preferably orally administered once a day at a dose of 100 mg, 200 mg, 300 mg, 400 mg, 500 mg, or 600 mg, or twice per day at a dose of 400 mg.

**[0166]** In the case that the kinase inhibitor used in the present invention is dasatinib or a pharmacologically acceptable salt thereof, the kinase inhibitor can be preferably orally administered once a day at a dose of 20 mg, 50 mg, 70 mg, 80 mg, 100 mg, or 140 mg.

**[0167]** In the case that the kinase inhibitor used in the present invention is bosutinib or a pharmacologically acceptable salt thereof, the kinase inhibitor can be preferably orally administered once a day at a dose of 100 mg, 200 mg, 300 mg, 400 mg, 500 mg, or 600 mg.

**[0168]** In the case that the kinase inhibitor used in the present invention is nilotinib or a pharmacologically acceptable salt thereof, the kinase inhibitor can be preferably orally administered twice per day at a dose of 150 mg, 200 mg, 300 mg, or 400 mg.

**[0169]** In the case that the kinase inhibitor used in the present invention is ponatinib or a pharmacologically acceptable salt thereof, the kinase inhibitor can be preferably orally administered once a day at a dose of 15 mg, 30 mg, or 45 mg.

**[0170]** In the case that the kinase inhibitor used in the present invention is vandetanib or a pharmacologically acceptable salt thereof, the kinase inhibitor can be preferably orally administered once a day at a dose of 100 mg, 200 mg, or 300 mg.

**[0171]** In the case that the kinase inhibitor used in the present invention is axitinib or a pharmacologically acceptable salt thereof, the kinase inhibitor can be preferably orally administered twice per day at a dose of 1 mg, 2 mg, 3 mg, 4 mg, or 5 mg.

**[0172]** In the case that the kinase inhibitor used in the present invention is pazopanib or a pharmacologically acceptable salt thereof, the kinase inhibitor can be preferably orally administered once a day at a dose of 200 mg, 400 mg, 600 mg, or 800 mg.

**[0173]** In the case that the kinase inhibitor used in the present invention is lenvatinib or a pharmacologically acceptable salt thereof, the kinase inhibitor can be preferably orally administered once a day at a dose of 4 mg, 8 mg, 10 mg, 12 mg, 14 mg, 16 mg, 18 mg, 20 mg, or 24 mg.

**[0174]** In the case that the kinase inhibitor used in the present invention is nintedanib or a pharmacologically acceptable salt thereof, the kinase inhibitor can be preferably orally administered twice per day at a dose of 100 mg or 150 mg.

**[0175]** In the case that the kinase inhibitor used in the present invention is cabozantinib or a pharmacologically acceptable salt thereof, the kinase inhibitor can be preferably orally administered once a day at a dose of 20 mg, 40 mg,

or 60 mg.

**[0176]** In the case that the kinase inhibitor used in the present invention is ibrutinib or a pharmacologically acceptable salt thereof, the kinase inhibitor can be preferably orally administered once a day at a dose of 70 mg, 140 mg, 210 mg, 280 mg, 350 mg, 420 mg, 490 mg, or 560 mg.

**[0177]** In the case that the kinase inhibitor used in the present invention is acalabrutinib or a pharmacologically acceptable salt thereof, the kinase inhibitor can be preferably orally administered once per 12 hours at a dose of 100 mg.

**[0178]** In the case that the kinase inhibitor used in the present invention is gilteritinib or a pharmacologically acceptable salt thereof, the kinase inhibitor can be preferably orally administered once a day at a dose of 40 mg, 80 mg, or 120 mg.

**[0179]** In the case that the kinase inhibitor used in the present invention is midostaurin or a pharmacologically acceptable salt thereof, the kinase inhibitor can be preferably orally administered twice per day at a dose of 25 mg, 50 mg, 75 mg, or 100 mg.

**[0180]** In the case that the kinase inhibitor used in the present invention is brigatinib or a pharmacologically acceptable salt thereof, the kinase inhibitor can be preferably orally administered once a day at a dose of 30 mg, 60 mg, 90 mg, 120 mg, 150 mg, or 180 mg.

**[0181]** In the case that the kinase inhibitor used in the present invention is crizotinib or a pharmacologically acceptable salt thereof, the kinase inhibitor can be preferably orally administered twice per day at a dose of 200 mg or 250 mg.

**[0182]** In the case that the kinase inhibitor used in the present invention is ceritinib or a pharmacologically acceptable salt thereof, the kinase inhibitor can be preferably orally administered once a day at a dose of 150 mg, 300 mg, 450 mg, 600 mg, or 750 mg.

**[0183]** In the case that the kinase inhibitor used in the present invention is alectinib or a pharmacologically acceptable salt thereof, the kinase inhibitor can be preferably orally administered twice per day at a dose of 150 mg, 300 mg, 450 mg, or 600 mg.

**[0184]** In the case that the kinase inhibitor used in the present invention is lorlatinib or a pharmacologically acceptable salt thereof, the kinase inhibitor can be preferably orally administered once a day at a dose of 25 mg, 50 mg, 75 mg, or 100 mg.

**[0185]** In the case that the kinase inhibitor used in the present invention is ruxolitinib or a pharmacologically acceptable salt thereof, the kinase inhibitor can be preferably orally administered twice per day at a dose of 5 mg, 10 mg, 15 mg, 20 mg, or 25 mg.

**[0186]** In the case that the kinase inhibitor used in the present invention is tofacitinib or a pharmacologically acceptable salt thereof, the kinase inhibitor can be preferably orally administered twice per day at a dose of 5 mg or 10 mg per administration, and in using as a sustained-release formulation preferably administered once a day at a dose of 11 mg.

**[0187]** In the case that the kinase inhibitor used in the present invention is baricitinib or a pharmacologically acceptable salt thereof, the kinase inhibitor can be preferably orally administered once a day at a dose of 2 mg.

**[0188]** In the case that the kinase inhibitor used in the present invention is gefitinib or a pharmacologically acceptable salt thereof, the kinase inhibitor can be preferably orally administered once a day at a dose of 250 mg.

**[0189]** In the case that the kinase inhibitor used in the present invention is erlotinib or a pharmacologically acceptable salt thereof, the kinase inhibitor can be preferably orally administered once a day at a dose of 25 mg, 50 mg, 75 mg, 100 mg, 125 mg, or 150 mg.

**[0190]** In the case that the kinase inhibitor used in the present invention is afatinib or a pharmacologically acceptable salt thereof, the kinase inhibitor can be preferably orally administered once a day at a dose of 20 mg, 30 mg, or 40 mg.

**[0191]** In the case that the kinase inhibitor used in the present invention is osimertinib or a pharmacologically acceptable salt thereof, the kinase inhibitor can be preferably orally administered once a day at a dose of 40 mg or 80 mg.

**[0192]** In the case that the kinase inhibitor used in the present invention is dacomitinib or a pharmacologically acceptable salt thereof, the kinase inhibitor can be preferably orally administered once a day at a dose of 15 mg, 30 mg, or 45 mg.

**[0193]** In the case that the kinase inhibitor used in the present invention is lapatinib or a pharmacologically acceptable salt thereof, the kinase inhibitor can be preferably orally administered once a day at a dose of 250 mg, 500 mg, 750 mg, 1000 mg, 1250 mg, or 1500 mg.

**[0194]** In the case that the kinase inhibitor used in the present invention is neratinib or a pharmacologically acceptable salt thereof, the kinase inhibitor can be preferably orally administered once a day at a dose of 40 mg, 80 mg, 120 mg, 160 mg, 200 mg, or 240 mg.

**[0195]** The pharmaceutical composition and method of treatment of the present invention may further contain a cancer therapeutic agent other than the antibody-drug conjugate and the kinase inhibitor according to the present invention. The pharmaceutical composition and method of treatment of the present invention can also be administered in combination with another cancer therapeutic agent, thereby enhancing the antitumor effect. Other cancer therapeutic agents to be used for such purpose may be administered to a subject simultaneously with, separately from, or subsequently with the pharmaceutical composition of the present invention, or may be administered while varying the dosage interval for each. Such cancer therapeutic agents are not limited as long as they are agents having antitumor activity, and can be exemplified by at least one selected from the group consisting of irinotecan (CPT-11), cisplatin, carboplatin, oxaliplatin,

fluorouracil (5-FU), gemcitabine, capecitabine, paclitaxel, docetaxel, doxorubicin, epirubicin, cyclophosphamide, mitomycin C, a tegafur-gimeracil-oteracil combination drug, cetuximab, panitumumab, bevacizumab, ramucirumab, a trifluridinetipiracil combination drug, methotrexate, pemetrexed, tamoxifen, toremifene, fulvestrant, leuprorelin, goserelin, letrozole, anastrozole, a progesterone formulation, trastuzumab, and pertuzumab.

**[0196]** The pharmaceutical composition and method of treatment of the present invention can also be used in combination with radiotherapy. For example, a cancer patient may receive radiotherapy before and/or after or simultaneously with receiving therapy with the pharmaceutical composition of the present invention.

**[0197]** The pharmaceutical composition and method of treatment of the present invention can also be used as adjuvant chemotherapy in combination with a surgical procedure. The pharmaceutical composition of the present invention may be administered for the purpose of diminishing the size of a tumor before surgical procedure (referred to as pre-operative adjuvant chemotherapy or neoadjuvant therapy), or may be administered after a surgical procedure for the purpose of preventing the recurrence of a tumor (referred to as post-operative adjuvant chemotherapy or adjuvant therapy).

Examples

**[0198]** The present invention is specifically described in view of the examples shown below. However, the present invention is not limited to these. Further, it is by no means to be interpreted in a limited way.

Example 1: Production of antibody-drug conjugate (1)

**[0199]** In accordance with a production method described in International Publication No. WO 2015/115091 with use of an anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2), an antibody-drug conjugate in which a drug-linker represented by the following formula:

[Formula 30]

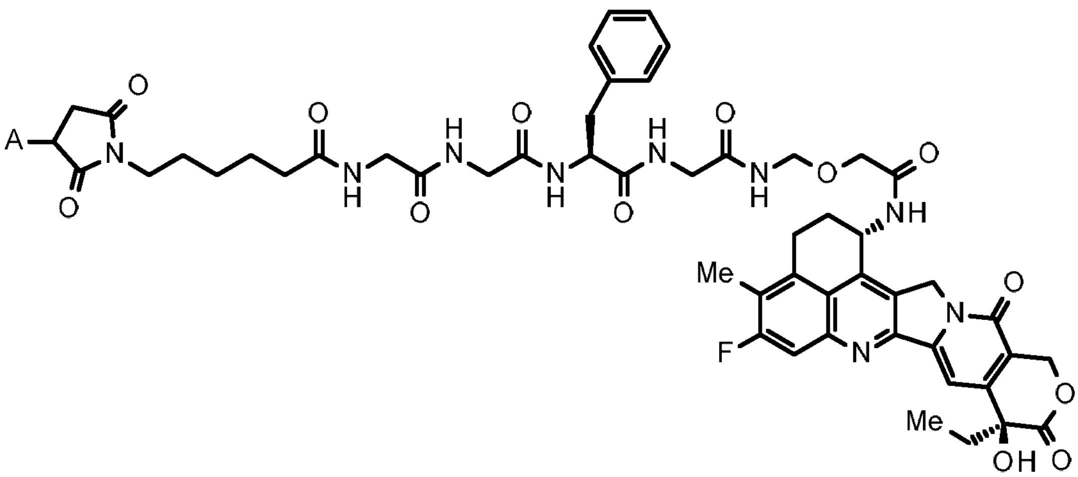

wherein A represents a connecting position to an antibody, is conjugated to the anti-HER2 antibody via a thioether bond (hereinafter, referred to as the "antibody-drug conjugate (1)") was produced. The DAR of the antibody-drug conjugate (1) is 7.7 or 7.8.

Example 2: Antitumor test (1)

**[0200]** Mouse: 5 to 6-week-old BALB/c nude mice (Charles River Laboratories Japan, Inc.) were subjected to experiment.

**[0201]** Assay and calculation expression: In all of the studies, the major axis and minor axis of a tumor were measured twice a week by using an electronic digital caliper (CD15-CX, Mitutoyo Corp.), and the tumor volume ($mm^3$) was calculated. The calculation expression is as shown below.

$$\text{Tumor volume (mm}^3\text{)} = 1/2 \times \text{Major axis (mm)} \times [\text{Minor axis (mm)}]^2$$

**[0202]** The antibody-drug conjugate (1) was diluted with ABS buffer (10 mM acetate buffer solution (pH 5.5), 5% sorbitol), and intravenously administered to the tail vein at a liquid volume of 10 mL/kg. Everolimus was suspended in 30% propylene glycol and 5% Tween 80, and orally administered at a liquid volume of 10 mL/kg. Taselisib was suspended in 0.5% methyl cellulose and 0.2% Tween 80, and orally administered at a liquid volume of 10 mL/kg. Abemaciclib was suspended in 1% hydroxyethyl cellulose and 0.1% antifoam/25 mM phosphate buffer, pH2, and orally administered at a liquid volume of 10 mL/kg.

**[0203]** MDA-MB-453 cells, a human breast cancer cell line, purchased from ATCC (American Type Culture Collection) were suspended in Matrigel basement membrane matrix, and $1 \times 10^7$ cells were subcutaneously transplanted to the right flank of each female nude mouse, and the mice were randomly grouped 7 days after the transplantation (Day 0). The antibody-drug conjugate (1) was intravenously administered to the tail vein of each mouse at a dose of 0.5 mg/kg on Day 0. Everolimus was administered once a day, five times a week, at a dose of 5 mg/kg for 3 weeks. Taselisib was administered once a day, five times a week, at a dose of 5 mg/kg for 3 weeks. Abemaciclib was administered once a day, five times a week, at a dose of 30 mg/kg for 2 weeks. Single administration groups and a combined administration group with the antibody-drug conjugate (1) and each kinase inhibitor, and a solvent administration group as a control group were established.

**[0204]** Results of combined use of the antibody-drug conjugate (1) and everolimus are shown in Figure 11. The tumor growth inhibition (TGI) of single administration of everolimus on the day of effect determination was 64%. The TGI of single administration of the antibody-drug conjugate (1) was 75%. For combined administration of the antibody-drug conjugate (1) and everolimus, on the other hand, tumor growth inhibitory effect significantly superior to that of single administration of everolimus was found (P = 0.0059 (calculated with Dunnett's test, the same applies hereinafter)). The tumor growth inhibition (TGI: 89%) was higher than those of single administration of them, demonstrating strong effect of their combined use. In the figure, the abscissa depicts days from the initiation of administration, and the ordinate depicts tumor volumes. No significant finding such as weight loss was observed in any of the single administration groups and combined administration group. In evaluation examples below for antitumor tests, the tests were conducted with the procedure used in this evaluation example, unless otherwise stated.

**[0205]** Results of combined use of the antibody-drug conjugate (1) and taselisib are shown in Figure 12. The TGI of single administration of taselisib was 67%. The TGI of single administration of the antibody-drug conjugate (1) was 75%. For combined administration of the antibody-drug conjugate (1) and taselisib, on the other hand, tumor growth inhibitory effect significantly superior to those of single administration of the antibody-drug conjugate (1) and single administration of taselisib was found (P = 0.0039 and P = 0.0043, respectively). The tumor growth inhibition (TGI: 92%) was higher than those of single administration of them, demonstrating strong effect of their combined use. No significant finding such as weight loss was observed in any of the single administration groups and combined administration group.

**[0206]** Results of combined use of the antibody-drug conjugate (1) and abemaciclib are shown in Figure 13. The TGI of single administration of abemaciclib was 68%. The TGI of single administration of the antibody-drug conjugate (1) was 75%. For combined administration of the antibody-drug conjugate (1) and abemaciclib, on the other hand, tumor growth inhibitory effect significantly superior to that of single administration of abemaciclib was found (P = 0.0429). The tumor growth inhibition (TGI: 87%) was higher than those of single administration of them, demonstrating strong effect of their combined use. No significant finding such as weight loss was observed in any of the single administration groups and combined administration group.

Example 3: Antitumor test (2)

**[0207]** JIMT-1 cells, a human breast cancer cell line, purchased from DSMZ (Deutsche Sammlung von Mikroorganismen und Zellkulturen GmbH) were suspended in physiological saline, and $5 \times 10^6$ cells were subcutaneously transplanted to the right flank of each female nude mouse, and the mice were randomly grouped 15 days after the transplantation (Day 0). The antibody-drug conjugate (1) was intravenously administered to the tail vein of each mouse at a dose of 10 mg/kg on Day 0. Everolimus was administered once a day, five times a week and seven times in total, at a dose of 5 mg/kg. Single administration groups and a combined administration group with the antibody-drug conjugate (1) and everolimus, and a solvent administration group as a control group were established.

**[0208]** Results of combined use of the antibody-drug conjugate (1) and everolimus are shown in Figure 14. The TGI of single administration of everolimus was 52%. The TGI of single administration of the antibody-drug conjugate (1) was 53%. For combined administration of the antibody-drug conjugate (1) and everolimus, on the other hand, tumor growth inhibitory effect significantly superior to those of single administration of the antibody-drug conjugate (1) and single

administration of everolimus was found (P = 0.0131 and P = 0.0054, respectively). The tumor growth inhibition (TGI: 93%) was higher than those of single administration of them, demonstrating strong effect of their combined use. No significant finding such as weight loss was observed in any of the single administration groups and combined administration group.

Example 4: Antitumor test (3)

**[0209]** COLO201 cells, a human colorectal cancer cell line, purchased from ATCC were suspended in 50% Matrigel solution, and 5 × $10^6$ cells were subcutaneously transplanted to the right flank of each female nude mouse, and the mice were randomly grouped 7 days after the transplantation (Day 0). The antibody-drug conjugate (1) was intravenously administered to the tail vein of each mouse at a dose of 3 mg/kg on Day 0. Regorafenib was dissolved in Cremophor EL/95% ethanol and then diluted with distilled water, and orally administered at a liquid volume of 10 mL/kg. Administration was performed once a day, five times a week, at a dose of 10 mg/kg for three weeks. Single administration groups and a combined administration group with the antibody-drug conjugate (1) and regorafenib, and a solvent administration group as a control group were established.

**[0210]** Results of combined use of the antibody-drug conjugate (1) and regorafenib are shown in Figure 15. The TGI of single administration of regorafenib was 81%. The TGI of single administration of the antibody-drug conjugate (1) was 67%. For combined administration of the antibody-drug conjugate (1) and regorafenib, on the other hand, tumor growth inhibitory effect significantly superior to that of single administration of the antibody-drug conjugate (1) was found (P = 0.0476). The tumor growth inhibition (TGI: 99%) was higher than those of single administration of them, demonstrating strong effect of their combined use. No significant finding such as weight loss was observed in any of the single administration groups and combined administration group.

Example 5: Antitumor test (4)

**[0211]** KPL-4 cells, a human breast cancer cell line, obtained from Dr. Junichi Kurebayashi, Kawasaki Medical School (British Journal of Cancer. (1999)79 (5/6). 707-717) were suspended in physiological saline, and 1.5 × $10^7$ cells were subcutaneously transplanted to the right flank of each female nude mouse, and the mice were randomly grouped 17 days after the transplantation (Day 0). The antibody-drug conjugate (1) was intravenously administered to the tail vein of each mouse at a dose of 7.5 mg/kg on Day 0. Tucatinib was suspended in 0.5% methyl cellulose, and orally administered at a liquid volume of 10 mL/kg. Administration was performed once a day, five times a week, at a dose of 100 mg/kg for two weeks. Single administration groups and a combined administration group with the antibody-drug conjugate (1) and tucatinib, and a solvent administration group as a control group were established.

**[0212]** Results of combined use of the antibody-drug conjugate (1) and tucatinib are shown in Figure 16. The TGI of single administration of tucatinib was 78%. The TGI of single administration of the antibody-drug conjugate (1) was 78%. For combined administration of the antibody-drug conjugate (1) and tucatinib, on the other hand, the tumor growth inhibition (TGI: 96%) was higher than those of single administration of them, demonstrating strong effect of their combined use. No significant finding such as weight loss was observed in any of the single administration groups and combined administration group.

Example 6: Antitumor test (5)

**[0213]** MDA-MB-453 cells, a human breast cancer cell line, purchased from ATCC (American Type Culture Collection) were suspended in Matrigel basement membrane matrix, and 1 × $10^7$ cells were subcutaneously transplanted to the right flank of each female nude mouse, and the mice were randomly grouped 7 days after the transplantation (Day 0). The antibody-drug conjugate (1) was intravenously administered to the tail vein of each mouse at a dose of 0.5 mg/kg on Day 0. Tucatinib was administered once a day, five times a week, at a dose of 100 mg/kg for three weeks. Single administration groups and a combined administration group with the antibody-drug conjugate (1) and tucatinib, and a solvent administration group as a control group were established.

**[0214]** Results of combined use of the antibody-drug conjugate (1) and tucatinib are shown in Figure 17. The TGI of single administration of tucatinib was 36%. The TGI of single administration of the antibody-drug conjugate (1) was 52%. For combined administration of the antibody-drug conjugate (1) and tucatinib, on the other hand, tumor growth inhibitory effect significantly superior to those of single administration of the antibody-drug conjugate (1) and single administration of tucatinib was found (P = 0.019 and P = 0.001, respectively). The tumor growth inhibition (TGI: 85%) was higher than those of single administration of them, demonstrating strong effect of their combined use. No significant finding such as weight loss was observed in any of the single administration groups and combined administration group.

Example 7: Antitumor test (6)

**[0215]** NCI-N87 cells, a human gastric cancer cell line, purchased from ATCC (American Type Culture Collection) were suspended in physiological saline, and 1 × $10^7$ cells were subcutaneously transplanted to the right flank of each female nude mouse, and the mice were randomly grouped 6 days after the transplantation (Day 0). The antibody-drug conjugate (1) was intravenously administered to the tail vein of each mouse at a dose of 2 mg/kg on Day 0. Tucatinib was administered once a day, five times a week, at a dose of 100 mg/kg for two weeks. Single administration groups and a combined administration group with the antibody-drug conjugate (1) and tucatinib, and a solvent administration group as a control group were established.

**[0216]** Results of combined use of the antibody-drug conjugate (1) and tucatinib are shown in Figure 18. The TGI of single administration of tucatinib was 65%. The TGI of single administration of the antibody-drug conjugate (1) was 71%. For combined administration of the antibody-drug conjugate (1) and tucatinib, on the other hand, tumor growth inhibitory effect significantly superior to those of single administration of the antibody-drug conjugate (1) and single administration of tucatinib was found (P = 0.0199 and P = 0.0034, respectively). The tumor growth inhibition (TGI: 92%) was higher than those of single administration of them, demonstrating strong effect of their combined use. No significant finding such as weight loss was observed in any of the single administration groups and combined administration group.

Example 8: Production of antibody-drug conjugate (2)

**[0217]** In accordance with a production method described in International Publication No. WO 2015/155998 with use of an anti-HER3 antibody (an antibody comprising a heavy chain consisting of the amino acid sequence represented by SEQ ID NO: 3 and a light chain consisting of the amino acid sequence represented by SEQ ID NO: 4), an antibody-drug conjugate in which a drug-linker represented by the following formula:

[Formula 31]

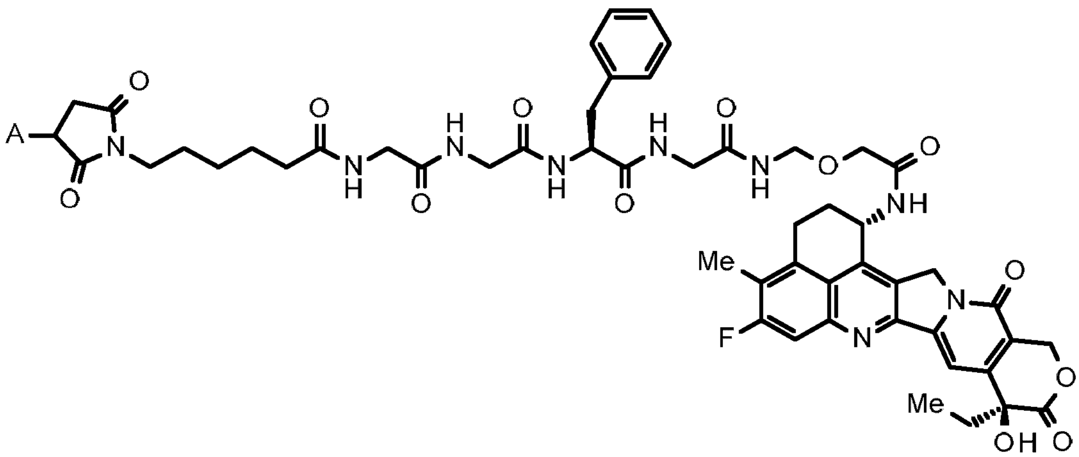

wherein A represents a connecting position to an anti-HER3 antibody,
is conjugated to the anti-HER3 antibody via a thioether bond (hereinafter, referred to as the "antibody-drug conjugate (2)") was produced. The DAR of the antibody-drug conjugate (2) is 7 to 8.

Example 9: Antitumor test (7)

**[0218]** KPL-4 cells, a human breast cancer cell line, obtained from Dr. Junichi Kurebayashi, Kawasaki Medical School (British Journal of Cancer. (1999)79 (5/6). 707-717) were suspended in physiological saline, and 1.5 × $10^7$ cells were subcutaneously transplanted to the right flank of each female nude mouse, and the mice were randomly grouped 17 days after the transplantation (Day 0). The antibody-drug conjugate (2) was diluted with ABS buffer, and intravenously administered to the tail vein of each mouse once a week at a dose of 10 mg/kg (a liquid volume of 10 mL/kg) for three weeks (Days 0, 7, 14). Neratinib was suspended in 0.5% methyl cellulose, and orally administered once a day, five times a week, at a dose of 20 mg/kg (a liquid volume of 10 mL/kg) for two weeks. Single administration groups and a combined administration group with the antibody-drug conjugate (2) and neratinib, and an ABS administration group as a control group were established.

**[0219]** Results of combined use of the antibody-drug conjugate (2) and neratinib are shown in Figure 19. The TGI of

single administration of neratinib on the day of effect determination was 84%. The TGI of single administration of the antibody-drug conjugate (2) was 67%. For combined administration of the antibody-drug conjugate (2) and neratinib, on the other hand, tumor growth inhibitory effect significantly superior to those of single administration of the antibody-drug conjugate (2) and single administration of neratinib was found (P < 0.0001 and P = 0.0098, respectively). The tumor growth inhibition (TGI: 95%) was higher than those of single administration of them, demonstrating strong effect of their combined use. No significant finding such as weight loss was observed in any of the single administration groups and combined administration group.

Example 10: Production of antibody-drug conjugate (3)

**[0220]** In accordance with a production method described in International Publication No. WO 2018/212136 with use of an anti-CDH6 antibody (an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10), an antibody-drug conjugate in which a drug-linker represented by the following formula:

[Formula 32]

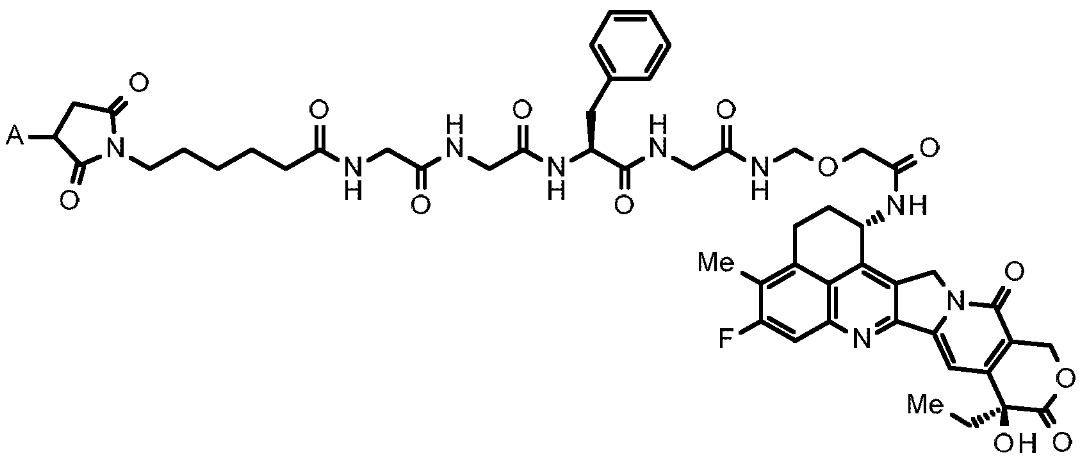

wherein A represents a connecting position to an anti-CDH6 antibody,

is conjugated to the anti-CDH6 antibody via a thioether bond (hereinafter, referred to as the "antibody-drug conjugate (3)") was produced. The DAR of the antibody-drug conjugate (3) is 7 to 8.

Example 11: Antitumor test (8)

**[0221]** Suspended in 100% Matrigel were 786-O cells, a human kidney cancer cell line, purchased from ATCC, and $4 \times 10^6$ cells were subcutaneously transplanted to the right flank of each female nude mouse, and the mice were randomly grouped 19 days after the transplantation (Day 0). The antibody-drug conjugate (3) was intravenously administered to the tail vein of each mouse at a dose of 10 mg/kg on Day 0. Cabozantinib was administered once a day, five times a week, 20 times in total, at a dose of 40 mg/kg. Single administration groups and a combined administration group with the antibody-drug conjugate (3) and cabozantinib, and a solvent administration group as a control group were established.

**[0222]** Results of combined use of the antibody-drug conjugate (3) and cabozantinib are shown in Figure 20. The TGI of single administration of cabozantinib was 67%. The TGI of single administration of the antibody-drug conjugate (3) was 71%. For combined administration of the antibody-drug conjugate (3) and cabozantinib, on the other hand, tumor growth inhibitory effect significantly superior to those of single administration of the antibody-drug conjugate (3) and single administration of cabozantinib was found (P = 0.0024 and P = 0.0004, respectively). The tumor growth inhibition (TGI: 90%) was higher than those of single administration of them, demonstrating strong effect of their combined use. No significant finding such as weight loss was observed in any of the single administration groups and combined administration group.

Example 12: Production of antibody-drug conjugate (4)

**[0223]** In accordance with a production method described in International Publication No. WO 2015/098099 and International Publication No. WO 2017/002776 with use of an anti-TROP2 antibody (an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6), an antibody-drug conjugate in which a drug-linker represented by the following formula:

[Formula 33]

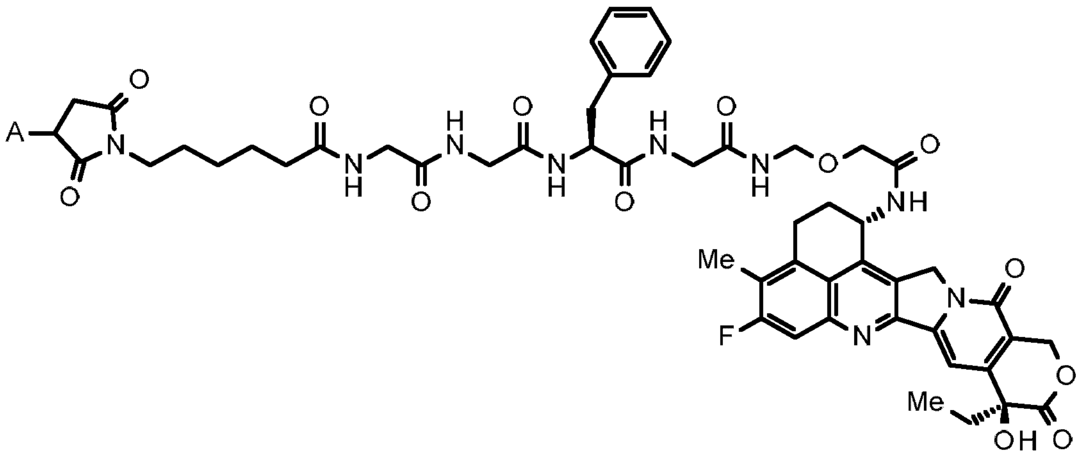

wherein A represents a connecting position to an anti-TROP2 antibody,
is conjugated to the anti-TROP2 antibody via a thioether bond (hereinafter, referred to as the "antibody-drug conjugate (4)") was produced. The DAR of the antibody-drug conjugate (4) is 3.5 or 4.5.

Example 13: Production of Compound (1)

**[0224]** In accordance with a production method described in International Publication No. WO 2014/057687 and International Publication No. WO 2015/115091, a compound represented by the following formula:

[Formula 34]

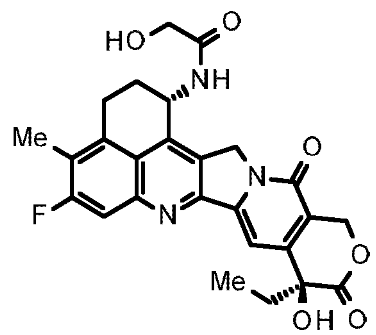

(hereinafter, referred to as the "Compound (1)") was produced.

Example 14: Cell growth inhibition test (1)

**[0225]** The human gastric cancer cell line NCI-N87 purchased from ATCC was used for evaluation. To a 1536-well cell culture plate, erlotinib (hydrochloride), poziotinib, mubritinib, brigatinib, or BIBF1120 prepared with dimethyl sulfoxide (DMSO) to concentrations of 2 mM, 400 μM, 80 μM, 16 μM, 3.2 μM, and 640 nM, or DMSO was added at 25 nL/well. Further, Compound (1) prepared with an RPMI1640 Medium (Thermo Fisher Scientific) containing 10% fetal bovine serum (GE Healthcare) to concentrations of 60 nM, 24 nM, 9.6 nM, 3.8 nM, 1.5 nM, and 0.61 nM, or the antibody-drug

conjugate (4) prepared to concentrations of 32 nM, 11 nM, 3.6 nM, 1.2 nM, 0.40 nM, and 0.13 nM, or the antibody-drug conjugate (1) prepared to concentrations of 8.0 nM, 2.7 nM, 0.89 nM, 0.30 nM, 0.10 nM, and 0.033 nM was added at 2.5 μL/well. Subsequently, NCI-N87 cells suspended with an RPMI1640 Medium containing 10% fetal bovine serum to a concentration of $4 \times 10^4$ cells/mL were seeded at 2.5 μL/well, and cultured at 37°C under 5%$CO_2$ for 6 days.

**[0226]** After culturing, a solution obtained by diluting CellTiter-Glo 2.0 Assay (Promega Corporation) with an equivalent amount of Glo Lysis buffer, 1 × (Promega Corporation) was added at 2 μL/well, and incubation was performed at room temperature for 1 hour, and luminescence intensity was then measured for each well.

**[0227]** Cell growth inhibition rate (%) was calculated by using the following calculation expression.

$$\text{Cell growth inhibition rate (\%)} = 100 \times (T - B) / (C - B) - 100$$

T: Mean luminescence intensity of wells with specimen
B: Mean luminescence intensity of wells with DMSO and medium
C: Mean luminescence intensity of wells with DMSO and cells

**[0228]** Sigmoid fitting with Genedata Screener Analyzer Version 14 (Genedata AG, hereinafter referred to as Screener) was performed for concentration-dependent transition of cell growth inhibition rates under each of combined use conditions.

**[0229]** For effect of combined use, differences between estimated values of additive effect by a Loewe model (Greco WR. et al., Pharmacol. Rev. 1995 Jun; 47 (2): 331-85) and sigmoid-fitted cell growth inhibition rates (%) were matrixized, and a Synergy Score was calculated from the matrix components by using a procedure demonstrated in the literature (Lehar J. et al., Nat Biotechnol. 2009 Jul; 27 (7): 659-66). Synergy Score = 0 indicates additive action, Synergy Score > 0 indicates synergistic action, and Synergy Score < 0 indicates antagonistic action.

**[0230]** Synergy Scores for the combinations are shown in Table 1. In the cell growth inhibition test for the NCI-N87 cell line, Compound (1) exhibited synergistic action when being combined with erlotinib (hydrochloride) or brigatinib, the antibody-drug conjugate (1) exhibited synergistic action when being combined with erlotinib (hydrochloride), poziotinib, brigatinib, or BIBF1120, and the antibody-drug conjugate (4) exhibited synergistic action when being combined with erlotinib (hydrochloride), poziotinib, mubritinib, brigatinib, or BIBF1120.

[Table 1]

| Synergy Scores for each combination in NCI-N87 cell line | | | | | |
|---|---|---|---|---|---|
| | Erlotinib | Poziotinib | Mubritinib | Brigatinib | BIBF1120 |
| Compound (1) | 0.48 | -0.53 | -0.45 | 0.11 | -0.44 |
| Antibody-drug conjugate (1) | 2.38 | 1.10 | -0.06 | 1.55 | 1.73 |
| Antibody-drug conjugate (4) | 2.33 | 1.50 | 1.67 | 0.41 | 1.16 |

Example 15: Cell growth inhibition test (2)

**[0231]** The human breast cancer cell line KPL-4 obtained from Dr. Junichi Kurebayashi, Kawasaki Medical School (British Journal of Cancer. (1999)79 (5/6). 707-717) was used for evaluation. To a 1536-well cell culture plate, erlotinib (hydrochloride), poziotinib, mubritinib, brigatinib, or BIBF1120 prepared with DMSO to concentrations of 2 mM, 400 μM, 80 μM, 16 μM, 3.2 μM, and 640 nM, or DMSO was added at 25 nL/well. Further, Compound (1) prepared with an RPMI1640 Medium containing 10% fetal bovine serum to concentrations of 36 nM, 20 nM, 11 nM, 6.2 nM, 3.4 nM, and 1.9 nM, or the antibody-drug conjugate (4) prepared to concentrations of 40 nM, 13 nM, 4.4 nM, 1.5 nM, 0.49 nM, and 0.16 nM, or the antibody-drug conjugate (1) prepared to concentrations of 3.7 nM, 1.3 nM, 0.48 nM, 0.17 nM, 0.061 nM, and 0.022 nM was added at 2.5 μL/well. Subsequently, KPL-4 cells suspended with an RPMI1640 Medium containing 10% fetal bovine serum to a concentration of $1 \times 10^4$ cells/mL were seeded at 2.5 μL/well, and cultured at 37°C under 5%$CO_2$ for 6 days. After culturing, a solution obtained by diluting CellTiter-Glo 2.0 Assay with an equivalent amount of Glo Lysis buffer, 1 × was added at 2 μL/well, and incubation was performed at room temperature for 1 hour, and luminescence intensity was then measured for each well.

**[0232]** Analysis of cell growth inhibition rates (%) and effect of combined use under each of the conditions was performed in the same manner as in Example 14.

**[0233]** Synergy Scores for the combinations are shown in Table 2. In the cell growth inhibition test for the KPL-4 cell line, Compound (1) exhibited synergistic action when being combined with erlotinib (hydrochloride), mubritinib, or BIBF1120, the antibody-drug conjugate (1) exhibited synergistic action when being combined with erlotinib (hydrochloride), poziotinib, mubritinib, brigatinib, or BIBF1120, and the antibody-drug conjugate (4) exhibited synergistic action when being combined with erlotinib (hydrochloride), mubritinib, brigatinib, or BIBF1120.

[Table 2]

| Synergy Scores for each combination in KPL-4 cell line | | | | | |
|---|---|---|---|---|---|
| | Erlotinib | Poziotinib | Mubritinib | Brigatinib | BIBF1120 |
| Compound (1) | 0.22 | -0.38 | 0.57 | -0.02 | 0.99 |
| Antibody-drug conjugate (1) | 0.77 | 0.03 | 0.28 | 1.58 | 2.26 |
| Antibody-drug conjugate (4) | 3.51 | -0.29 | 1.35 | 1.15 | 1.24 |

Example 16: Cell growth inhibition test (3)

**[0234]** The human lung cancer cell line EBC-1 obtained from Health Science Research Resources Bank (currently known as Japanese Collection of Research Bioresources (JCRB) Cell Bank) was used for evaluation. To a 1536-well cell culture plate, erlotinib (hydrochloride), poziotinib, mubritinib, brigatinib, or BIBF1120 prepared with DMSO to concentrations of 2 mM, 400 μM, 80 μM, 16 μM, 3.2 μM, and 640 nM or DMSO was added at 25 nL/well. Further, Compound (1) prepared with an RPMI1640 Medium containing 10% fetal bovine serum to concentrations of 16 nM, 8.0 nM, 4.0 nM, 2.0 nM, 1.0 nM, and 0.50 nM, or the antibody-drug conjugate (4) prepared to concentrations of 40 nM, 13 nM, 4.4 nM, 1.5 nM, 0.49 nM, and 0.16 nM was added at 2.5 μL/well. Subsequently, EBC-1 cells suspended with an RPMI1640 Medium containing 10% fetal bovine serum to a concentration of $2 \times 10^4$ cells/mL were seeded at 2.5 μL/well, and cultured at 37°C under 5%$CO_2$ for 6 days. After culturing, a solution obtained by diluting CellTiter-Glo 2.0 Assay with an equivalent amount of Glo Lysis buffer, 1 × was added at 2 μL/well, and incubation was performed at room temperature for 1 hour, and luminescence intensity was then measured for each well. Analysis of cell growth inhibition rates (%) and effect of combined use under each of the conditions was performed in the same manner as in Example 14.

**[0235]** Synergy Scores for the combinations are shown in Table 3.

**[0236]** In the cell growth inhibition test for the EBC-1 cell line, Compound (1) exhibited synergistic action when being combined with erlotinib (hydrochloride), poziotinib, brigatinib, or BIBF1120, and the antibody-drug conjugate (4) exhibited synergistic action when being combined with erlotinib (hydrochloride), poziotinib, mubritinib, brigatinib, or BIBF1120.

[Table 3]

| Synergy Scores for each combination in EBC-1 cell line | | | | | |
|---|---|---|---|---|---|
| | Erlotinib | Poziotinib | Mubritinib | Brigatinib | BIBF1120 |
| Compound (1) | 1.88 | 0.45 | -0.60 | 0.35 | 0.50 |
| Antibody-drug conjugate (4) | 6.96 | 1.42 | 0.72 | 2.52 | 6.04 |

Example 17: Cell growth inhibition test (4)

**[0237]** The human breast cancer cell line HCC70 obtained from ATCC was used for evaluation. To a 1536-well cell culture plate, erlotinib (hydrochloride), poziotinib, mubritinib, brigatinib, or BIBF1120 prepared with DMSO to concentrations of 2 mM, 400 μM, 80 μM, 16 μM, 3.2 μM, and 640 nM or DMSO was added at 25 nL/well. Further, Compound (1) prepared with an RPMI1640 Medium containing 10% fetal bovine serum to concentrations of 800 nM, 200 nM, 50 nM, 13 nM, 3.1 nM, and 0.78 nM, or the antibody-drug conjugate (4) prepared to concentrations of 5.6 nM, 2.0 nM, 0.71 nM, 0.26 nM, 0.091 nM, and 0.033 nM was added at 2.5 μL/well. Subsequently, HCC70 cells suspended with an RPMI1640 Medium containing 10% fetal bovine serum to a concentration of $4 \times 10^4$ cells/mL were seeded at 2.5 μL/well, and cultured at 37°C under 5%$CO_2$ for 6 days. After culturing, a solution obtained by diluting CellTiter-Glo 2.0 Assay with an equivalent amount of Glo Lysis buffer, 1 × was added at 2 μL/well, and incubation was performed at room temperature for 1 hour, and luminescence intensity was then measured for each well.

**[0238]** Analysis of cell growth inhibition rates (%) and effect of combined use under each of the conditions was performed in the same manner as in Example 14.

**[0239]** Synergy Scores for the combinations are shown in Table 4.

**[0240]** In the cell growth inhibition test for the HCC70 cell line, Compound (1) exhibited synergistic action when being combined with erlotinib (hydrochloride), poziotinib, mubritinib, brigatinib, or BIBF1120, and the antibody-drug conjugate (4) exhibited synergistic action when being combined with erlotinib (hydrochloride), poziotinib, mubritinib, brigatinib, or BIBF1120.

[Table 4]

| Synergy Scores for each combination in HCC70 cell line | | | | | |
|---|---|---|---|---|---|
| | Erlotinib | Poziotinib | Mubritinib | Brigatinib | BIBF1120 |
| Compound (1) | 0.93 | 0.95 | 3.41 | 4.11 | 1.65 |
| Antibody-drug conjugate (4) | 1.38 | 0.64 | 1.44 | 1.60 | 1.30 |

Example 18: Cell growth inhibition test (5)

**[0241]** The human pancreatic cancer cell line BxPC-3 obtained from ATCC was used for evaluation. To a 1536-well cell culture plate, erlotinib (hydrochloride), poziotinib, mubritinib, brigatinib, or BIBF1120 prepared with DMSO to concentrations of 2 mM, 400 μM, 80 μM, 16 μM, 3.2 μM, and 640 nM or DMSO was added at 25 nL/well. Further, Compound (1) prepared with an RPMI1640 Medium containing 10% fetal bovine serum to concentrations of 80 nM, 32 nM, 13 nM, 5.1 nM, 2.0 nM, and 0.82 nM, or the antibody-drug conjugate (4) prepared to concentrations of 16 nM, 5.3 nM, 1.8 nM, 0.59 nM, 0.20 nM, and 0.066 nM was added at 2.5 μL/well. Subsequently, BxPC-3 cells suspended with an RPMI1640 Medium containing 10% fetal bovine serum to a concentration of $4 \times 10^4$ cells/mL were seeded at 2.5 μL/well, and cultured at 37°C under 5%$CO_2$ for 6 days. After culturing, a solution obtained by diluting CellTiter-Glo 2.0 Assay (Promega Corporation) with an equivalent amount of Glo Lysis buffer, 1 × (Promega Corporation) was added at 2 μL/well, and incubation was performed at room temperature for 1 hour, and luminescence intensity was then measured for each well.
**[0242]** Analysis of cell growth inhibition rates (%) and effect of combined use under each of the conditions was performed in the same manner as in Example 14.
**[0243]** Synergy Scores for the combinations are shown in Table 5.
**[0244]** In the cell growth inhibition test for the BxPC-3 cell line, Compound (1) exhibited synergistic action when being combined with erlotinib (hydrochloride), poziotinib, brigatinib, or BIBF1120, and the antibody-drug conjugate (4) exhibited synergistic action when being combined with erlotinib (hydrochloride), poziotinib, brigatinib, or BIBF1120.

[Table 5]

| Synergy Scores for each combination in BxPC-3 cell line | | | | | |
|---|---|---|---|---|---|
| | Erlotinib | Poziotinib | Mubritinib | Brigatinib | BIBF1120 |
| Compound (1) | 1.29 | 0.37 | -0.40 | 0.93 | 0.28 |
| Antibody-drug conjugate (4) | 1.84 | 0.39 | -0.30 | 1.67 | 1.28 |

Example 19: Antitumor test (9)

**[0245]** MDA-MB-453 cells, a human breast cancer cell line, obtained from ATCC were suspended in Matrigel basement membrane matrix, and $1 \times 10^7$ cells were subcutaneously transplanted to the right flank of each female nude mouse, and the mice were randomly grouped 11 days after the transplantation (Day 0). The antibody-drug conjugate (2) was diluted with ABS buffer, and intravenously administered to the tail vein of each mouse once a week at a dose of 1 mg/kg (a liquid volume of 10 mL/kg) for three weeks (Days 0, 7, 14). Palbociclib was suspended in 0.5% methyl cellulose, and orally administered once a day, five times a week, at a dose of 50 mg/kg (a liquid volume of 10 mL/kg) for three weeks from Day 0. Single administration groups and a combined administration group with the antibody-drug conjugate (2) and palbociclib, and an ABS administration group as a control group were established.
**[0246]** Results of combined use of the antibody-drug conjugate (2) and palbociclib are shown in Figure 21. TGI of single administration of palbociclib was 72%. The TGI of single administration of the antibody-drug conjugate (2) was 54%. For combined administration of the antibody-drug conjugate (2) and palbociclib, on the other hand, tumor growth inhibitory effect significantly superior to those of single administration of the antibody-drug conjugate (2) and single administration of palbociclib was found ($P < 0.0001$ and $P = 0.004$, respectively). The tumor growth inhibition (TGI: 93%) was higher than those of single administration of them, demonstrating strong effect of their combined use. No significant finding such as weight loss was observed in any of the single administration groups and combined administration group.

Example 20: Antitumor test (10)

**[0247]** MDA-MB-453 cells, a human breast cancer cell line, obtained from ATCC were suspended in Matrigel basement membrane matrix, and 1 × $10^7$ cells were subcutaneously transplanted to the right flank of each female nude mouse, and the mice were randomly grouped 7 days after the transplantation (Day 0). The antibody-drug conjugate (2) was diluted with ABS buffer, and intravenously administered to the tail vein of each mouse once a week at a dose of 1 mg/kg (a liquid volume of 10 mL/kg) for three weeks (Days 0, 7, 14). Alpelisib was suspended in 0.5% methyl cellulose, and orally administered once a day, five times a week, at a dose of 25 mg/kg (a liquid volume of 10 mL/kg) for three weeks from Day 0. Single administration groups and a combined administration group with the antibody-drug conjugate (2) and alpelisib, and an ABS administration group as a control group were established.
**[0248]** Results of combined use of the antibody-drug conjugate (2) and alpelisib are shown in Figure 22. TGI of single administration of alpelisib was 56%. The TGI of single administration of the antibody-drug conjugate (2) was 68%. For combined administration of the antibody-drug conjugate (2) and alpelisib, on the other hand, tumor growth inhibitory effect significantly superior to those of single administration of the antibody-drug conjugate (2) and single administration of alpelisib was found (P = 0.01 and P = 0.0001, respectively). The tumor growth inhibition (TGI: 86%) was higher than those of single administration of them, demonstrating strong effect of their combined use. No significant finding such as weight loss was observed in any of the single administration groups and combined administration group.

Free Text of Sequence Listing

**[0249]**

SEQ ID NO: 1 - Amino acid sequence of a heavy chain of the anti-HER2 antibody
SEQ ID NO: 2 - Amino acid sequence of a light chain of the anti-HER2 antibody
SEQ ID NO: 3 - Amino acid sequence of a heavy chain of the anti-HER3 antibody
SEQ ID NO: 4 - Amino acid sequence of a light chain of the anti-HER3 antibody
SEQ ID NO: 5 - Amino acid sequence of a heavy chain of the anti-TROP2 antibody
SEQ ID NO: 6 - Amino acid sequence of a light chain of the anti-TROP2 antibody
SEQ ID NO: 7 - Amino acid sequence of a heavy chain of the anti-B7-H3 antibody
SEQ ID NO: 8 - Amino acid sequence of a light chain of the anti-B7-H3 antibody
SEQ ID NO: 9 - Amino acid sequence of a heavy chain of the anti-CDH6 antibody
SEQ ID NO: 10 - Amino acid sequence of a light chain of the anti-CDH6 antibody

## Claims

1. A pharmaceutical composition wherein an antibody-drug conjugate and a kinase inhibitor are administered in combination, and the antibody-drug conjugate is an antibody-drug conjugate in which a drug-linker represented by the following formula:

[Formula 1]

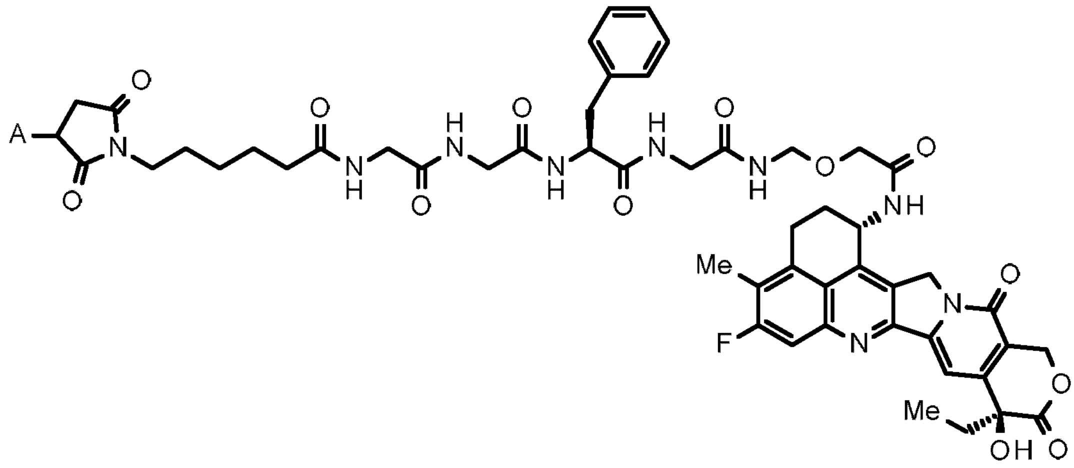

wherein A represents a connecting position to an antibody, is conjugated to the antibody via a thioether bond, and the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

2. The pharmaceutical composition according to claim 1, wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

3. The pharmaceutical composition according to claim 2, wherein the kinase inhibitor is a CDK4/6 inhibitor.

4. The pharmaceutical composition according to claim 3, wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

5. The pharmaceutical composition according to claim 2, wherein the kinase inhibitor is an mTOR inhibitor.

6. The pharmaceutical composition according to claim 5, wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

7. The pharmaceutical composition according to claim 2, wherein the kinase inhibitor is a PI3K inhibitor.

8. The pharmaceutical composition according to claim 7, wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

9. The pharmaceutical composition according to claim 2, wherein the kinase inhibitor is an RAF inhibitor.

10. The pharmaceutical composition according to claim 9, wherein the RAF inhibitor is regorafenib, sorafenib, vemurafenib, dabrafenib, encorafenib, RAF265, GDC-5573, LY3009120, or RO5126766, or a pharmacologically acceptable salt thereof.

11. The pharmaceutical composition according to claim 2, wherein the kinase inhibitor is a VEGFR inhibitor.

**12.** The pharmaceutical composition according to claim 11, wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

**13.** The pharmaceutical composition according to claim 2, wherein the kinase inhibitor is a KIT inhibitor.

**14.** The pharmaceutical composition according to claim 13, wherein the KIT inhibitor is regorafenib, sorafenib, imatinib, ilorasertib, sunitinib, pazopanib, lenvatinib, or dasatinib, or a pharmacologically acceptable salt thereof.

**15.** The pharmaceutical composition according to claim 2, wherein the kinase inhibitor is an RET inhibitor.

**16.** The pharmaceutical composition according to claim 15, wherein the RET inhibitor is regorafenib, sorafenib, vandetanib, lenvatinib, or sunitinib, or a pharmacologically acceptable salt thereof.

**17.** The pharmaceutical composition according to claim 2, wherein the kinase inhibitor is a PDGFR inhibitor.

**18.** The pharmaceutical composition according to claim 17, wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.

**19.** The pharmaceutical composition according to claim 2, wherein the kinase inhibitor is an FGFR inhibitor.

**20.** The pharmaceutical composition according to claim 19, wherein the FGFR inhibitor is regorafenib, sorafenib, lenvatinib, nintedanib, axitinib, or pazopanib, or a pharmacologically acceptable salt thereof.

**21.** The pharmaceutical composition according to claim 2, wherein the kinase inhibitor is an FLT3 inhibitor.

**22.** The pharmaceutical composition according to claim 21, wherein the FLT3 inhibitor is gilteritinib, quizartinib, midostaurin, sorafenib, ilorasertib, ENMD-2076, or sunitinib, or a pharmacologically acceptable salt thereof.

**23.** The pharmaceutical composition according to claim 2, wherein the kinase inhibitor is an ALK inhibitor.

**24.** The pharmaceutical composition according to claim 23, wherein the ALK inhibitor is brigatinib, crizotinib, ceritinib, alectinib, or lorlatinib, or a pharmacologically acceptable salt thereof.

**25.** The pharmaceutical composition according to claim 2, wherein the kinase inhibitor is a CSF-1R inhibitor.

**26.** The pharmaceutical composition according to claim 25, wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.

**27.** The pharmaceutical composition according to claim 2, wherein the kinase inhibitor is an EGFR inhibitor.

**28.** The pharmaceutical composition according to claim 27, wherein the EGFR inhibitor is gefitinib, erlotinib, afatinib, osimertinib, dacomitinib, lapatinib, neratinib, pyrotinib, or poziotinib, or a pharmacologically acceptable salt thereof.

**29.** The pharmaceutical composition according to claim 2, wherein the kinase inhibitor is an HER2 inhibitor.

**30.** The pharmaceutical composition according to claim 29, wherein the HER2 inhibitor is tucatinib, neratinib, mubritinib, lapatinib, pyrotinib, or poziotinib, or a pharmacologically acceptable salt thereof.

**31.** The pharmaceutical composition according to any one of claims 1 to 30, wherein the antibody in the antibody-drug conjugate is an anti-HER2 antibody, an anti-HER3 antibody, an anti-TROP2 antibody, an anti-B7-H3 antibody, or an anti-CDH6 antibody.

**32.** The pharmaceutical composition according to claim 31, wherein the antibody in the antibody-drug conjugate is an anti-HER2 antibody.

**33.** The pharmaceutical composition according to claim 32, wherein the anti-HER2 antibody is an antibody comprising

a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

**34.** The pharmaceutical composition according to claim 32, wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

**35.** The pharmaceutical composition according to claim 32, wherein the anti-HER2 antibody is an antibody comprising a heavy chain consisting of an amino acid sequence represented by SEQ ID NO: 1 and a light chain consisting of an amino acid sequence represented by SEQ ID NO: 2.

**36.** The pharmaceutical composition according to claim 32, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**37.** The pharmaceutical composition according to any one of claims 32 to 36, wherein the average number of units of the drug-linker conjugated per antibody molecule in the antibody-drug conjugate is in the range of from 7 to 8.

**38.** The pharmaceutical composition according to claim 31, wherein the antibody in the antibody-drug conjugate is an anti-HER3 antibody.

**39.** The pharmaceutical composition according to claim 38, wherein the anti-HER3 antibody is an antibody comprising a heavy chain consisting of the amino acid sequence represented by SEQ ID NO: 3 and a light chain consisting of the amino acid sequence represented by SEQ ID NO: 4.

**40.** The pharmaceutical composition according to claim 39, wherein the anti-HER3 antibody lacks a lysine residue at the carboxyl terminus of the heavy chain.

**41.** The pharmaceutical composition according to any one of claims 38 to 40, wherein the average number of units of the drug-linker conjugated per antibody molecule in the antibody-drug conjugate is in the range of from 7 to 8.

**42.** The pharmaceutical composition according to claim 31, wherein the antibody in the antibody-drug conjugate is an anti-TROP2 antibody.

**43.** The pharmaceutical composition according to claim 42, wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

**44.** The pharmaceutical composition according to claim 43, wherein the anti-TROP2 antibody lacks a lysine residue at the carboxyl terminus of the heavy chain.

**45.** The pharmaceutical composition according to any one of claims 42 to 44, wherein the average number of units of the drug-linker conjugated per antibody molecule in the antibody-drug conjugate is in the range of from 3.5 to 4.5.

**46.** The pharmaceutical composition according to claim 31, wherein the antibody in the antibody-drug conjugate is an anti-B7-H3 antibody.

**47.** The pharmaceutical composition according to claim 46, wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**48.** The pharmaceutical composition according to claim 47, wherein the anti-B7-H3 antibody lacks a lysine residue at

the carboxyl terminus of the heavy chain.

**49.** The pharmaceutical composition according to any one of claims 46 to 48, wherein the average number of units of the drug-linker conjugated per antibody molecule in the antibody-drug conjugate is in the range of from 3.5 to 4.5.

**50.** The pharmaceutical composition according to claim 31, wherein the antibody in the antibody-drug conjugate is an anti-CDH6 antibody.

**51.** The pharmaceutical composition according to claim 50, wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

**52.** The pharmaceutical composition according to claim 51, wherein the anti-CDH6 antibody lacks a lysine residue at the carboxyl terminus of the heavy chain.

**53.** The pharmaceutical composition according to any one of claims 50 to 52, wherein the average number of units of the drug-linker conjugated per antibody molecule in the antibody-drug conjugate is in the range of from 7 to 8.

**54.** The pharmaceutical composition according to any one of claims 1 to 53, wherein the antibody-drug conjugate and the kinase inhibitor are separately contained as active components in different formulations, and are administered simultaneously or at different times.

**55.** The pharmaceutical composition according to any one of claims 1 to 54, wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

**56.** The pharmaceutical composition according to claim 55, wherein the pharmaceutical composition is for use in treating breast cancer.

**57.** The pharmaceutical composition according to claim 55, wherein the pharmaceutical composition is for use in treating colorectal cancer.

**58.** The pharmaceutical composition according to claim 55, wherein the pharmaceutical composition is for use in treating gastric cancer.

**59.** The pharmaceutical composition according to claim 55, wherein the pharmaceutical composition is for use in treating lung cancer.

**60.** The pharmaceutical composition according to claim 55, wherein the pharmaceutical composition is for use in treating pancreatic cancer.

**61.** The pharmaceutical composition according to claim 55, wherein the pharmaceutical composition is for use in treating kidney cancer.

**62.** The pharmaceutical composition according to claim 55, wherein the pharmaceutical composition is for use in treating ovarian cancer.

**63.** A pharmaceutical composition wherein an antibody-drug conjugate and a kinase inhibitor are administered in combination, and the antibody-drug conjugate is an antibody-drug conjugate represented by the following formula:

[Formula 2]

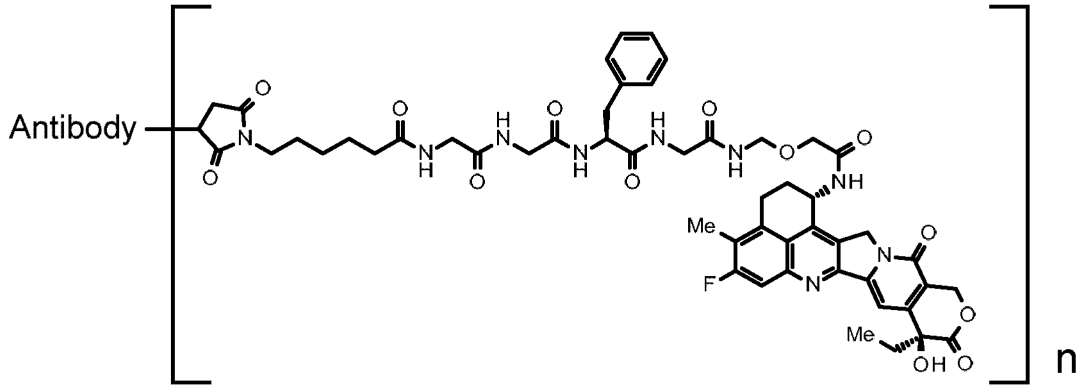

wherein a drug-linker is conjugated to an antibody via a thioether bond, and n indicates the average number of units of the drug-linker conjugated per antibody molecule, and
the kinase inhibitor is at least one selected from the group consisting of a CDK4/6 inhibitor, an mTOR inhibitor, a PI3K inhibitor, an AKT inhibitor, an ERK inhibitor, an MEK inhibitor, an RAF inhibitor, a CDK1 inhibitor, a CDK2 inhibitor, a CHK1 inhibitor, a WEE1 inhibitor, a PLK1 inhibitor, an Aurora kinase inhibitor, a Bcr-Abl inhibitor, an Src inhibitor, an EPH inhibitor, a VEGFR inhibitor, a KIT inhibitor, an RET inhibitor, a PDGFR inhibitor, an FGFR inhibitor, a BTK inhibitor, an FLT3 inhibitor, an ALK inhibitor, a JAK inhibitor, an MET inhibitor, a CSF-1R inhibitor, an NTRK inhibitor, an EGFR inhibitor, and an HER2 inhibitor.

**64.** The pharmaceutical composition according to claim 63, wherein the kinase inhibitor is at least one selected from the group consisting of a CDK4/6 inhibitor, an mTOR inhibitor, a PI3K inhibitor, an RAF inhibitor, a VEGFR inhibitor, a KIT inhibitor, an RET inhibitor, a PDGFR inhibitor, an FGFR inhibitor, an FLT3 inhibitor, an ALK inhibitor, a CSF-1R inhibitor, an EGFR inhibitor, and an HER2 inhibitor.

**65.** The pharmaceutical composition according to claim 64, wherein the kinase inhibitor is a CDK4/6 inhibitor.

**66.** The pharmaceutical composition according to claim 65, wherein the CDK4/6 inhibitor is abemaciclib, palbociclib, ribociclib, trilaciclib, G1T38, PF-06873600, TP-1287, FN-1501, or KRX-0601, or a pharmacologically acceptable salt thereof.

**67.** The pharmaceutical composition according to claim 64, wherein the kinase inhibitor is an mTOR inhibitor.

**68.** The pharmaceutical composition according to claim 67, wherein the mTOR inhibitor is everolimus, sirolimus, temsirolimus, TAK-228, CC-223, AZD8055, dactolisib, apitolisib, gedatolisib, LY3023414, PF-04691502, NVP-BGT226, or PQR309, or a pharmacologically acceptable salt thereof.

**69.** The pharmaceutical composition according to claim 64, wherein the kinase inhibitor is a PI3K inhibitor.

**70.** The pharmaceutical composition according to claim 69, wherein the PI3K inhibitor is taselisib, alpelisib, TAK-117, GSK2636771, AZD8186, IPI-549, idelalisib, duvelisib, AMG319, buparlisib, pictilisib, pilaralisib, copanlisib, sonolisib, CH5132799, ZSTK474, GDC-0077, dactolisib, apitolisib, gedatolisib, LY3023414, PF-04691502, NVP-BGT226, PQR309, KRX-0601, or rigosertib, or a pharmacologically acceptable salt thereof.

**71.** The pharmaceutical composition according to claim 64, wherein the kinase inhibitor is an RAF inhibitor.

**72.** The pharmaceutical composition according to claim 71, wherein the RAF inhibitor is regorafenib, sorafenib, vemurafenib, dabrafenib, encorafenib, RAF265, GDC-5573, LY3009120, or RO5126766, or a pharmacologically acceptable salt thereof.

**73.** The pharmaceutical composition according to claim 64, wherein the kinase inhibitor is a VEGFR inhibitor.

**74.** The pharmaceutical composition according to claim 73, wherein the VEGFR inhibitor is regorafenib, sorafenib, vandetanib, sunitinib, axitinib, pazopanib, lenvatinib, nintedanib, cabozantinib, tivozanib, brivanib, linifanib, lucitanib, ilorasertib, or ENMD-2076, or a pharmacologically acceptable salt thereof.

**75.** The pharmaceutical composition according to claim 64, wherein the kinase inhibitor is a KIT inhibitor.

**76.** The pharmaceutical composition according to claim 75, wherein the KIT inhibitor is regorafenib, sorafenib, imatinib, ilorasertib, sunitinib, pazopanib, lenvatinib, or dasatinib, or a pharmacologically acceptable salt thereof.

**77.** The pharmaceutical composition according to claim 64, wherein the kinase inhibitor is an RET inhibitor.

**78.** The pharmaceutical composition according to claim 77, wherein the RET inhibitor is regorafenib, sorafenib, vandetanib, lenvatinib, or sunitinib, or a pharmacologically acceptable salt thereof.

**79.** The pharmaceutical composition according to claim 64, wherein the kinase inhibitor is a PDGFR inhibitor.

**80.** The pharmaceutical composition according to claim 79, wherein the PDGFR inhibitor is regorafenib, sorafenib, sunitinib, axitinib, pazopanib, lenvatinib, nintedanib, ilorasertib, imatinib, nilotinib, or dasatinib, or a pharmacologically acceptable salt thereof.

**81.** The pharmaceutical composition according to claim 64, wherein the kinase inhibitor is an FGFR inhibitor.

**82.** The pharmaceutical composition according to claim 81, wherein the FGFR inhibitor is regorafenib, sorafenib, lenvatinib, nintedanib, axitinib, or pazopanib, or a pharmacologically acceptable salt thereof.

**83.** The pharmaceutical composition according to claim 64, wherein the kinase inhibitor is an FLT3 inhibitor.

**84.** The pharmaceutical composition according to claim 83, wherein the FLT3 inhibitor is gilteritinib, quizartinib, midostaurin, sorafenib, ilorasertib, ENMD-2076, or sunitinib, or a pharmacologically acceptable salt thereof.

**85.** The pharmaceutical composition according to claim 64, wherein the kinase inhibitor is an ALK inhibitor.

**86.** The pharmaceutical composition according to claim 85, wherein the ALK inhibitor is brigatinib, crizotinib, ceritinib, alectinib, or lorlatinib, or a pharmacologically acceptable salt thereof.

**87.** The pharmaceutical composition according to claim 64, wherein the kinase inhibitor is a CSF-1R inhibitor.

**88.** The pharmaceutical composition according to claim 87, wherein the CSF-1R inhibitor is pexidartinib, BLZ-945, JNJ-40346527, JNJ-28312141, ilorasertib, imatinib, sunitinib, or axitinib, or a pharmacologically acceptable salt thereof.

**89.** The pharmaceutical composition according to claim 64, wherein the kinase inhibitor is an EGFR inhibitor.

**90.** The pharmaceutical composition according to claim 89, wherein the EGFR inhibitor is gefitinib, erlotinib, afatinib, osimertinib, dacomitinib, lapatinib, neratinib, pyrotinib, or poziotinib, or a pharmacologically acceptable salt thereof.

**91.** The pharmaceutical composition according to claim 64, wherein the kinase inhibitor is an HER2 inhibitor.

**92.** The pharmaceutical composition according to claim 91, wherein the HER2 inhibitor is tucatinib, neratinib, mubritinib, lapatinib, pyrotinib, or poziotinib, or a pharmacologically acceptable salt thereof.

**93.** The pharmaceutical composition according to any one of claims 63 to 92, wherein the antibody in the antibody-drug conjugate is an anti-HER2 antibody, an anti-HER3 antibody, an anti-TROP2 antibody, an anti-B7-H3 antibody, or an anti-CDH6 antibody.

**94.** The pharmaceutical composition according to claim 93, wherein the antibody in the antibody-drug conjugate is an anti-HER2 antibody.

**95.** The pharmaceutical composition according to claim 94, wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid

residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

**96.** The pharmaceutical composition according to claim 94, wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

**97.** The pharmaceutical composition according to claim 94, wherein the anti-HER2 antibody is an antibody comprising a heavy chain consisting of an amino acid sequence represented by SEQ ID NO: 1 and a light chain consisting of an amino acid sequence represented by SEQ ID NO: 2.

**98.** The pharmaceutical composition according to claim 94, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**99.** The pharmaceutical composition according to any one of claims 94 to 98, wherein the average number of units of the drug-linker conjugated per antibody molecule in the antibody-drug conjugate is in the range of from 7 to 8.

**100.** The pharmaceutical composition according to claim 93, wherein the antibody in the antibody-drug conjugate is an anti-HER3 antibody.

**101.** The pharmaceutical composition according to claim 100, wherein the anti-HER3 antibody is an antibody comprising a heavy chain consisting of the amino acid sequence represented by SEQ ID NO: 3 and a light chain consisting of the amino acid sequence represented by SEQ ID NO: 4.

**102.** The pharmaceutical composition according to claim 101, wherein the anti-HER3 antibody lacks a lysine residue at the carboxyl terminus of the heavy chain.

**103.** The pharmaceutical composition according to any one of claims 100 to 102, wherein the average number of units of the drug-linker conjugated per antibody molecule in the antibody-drug conjugate is in the range of from 7 to 8.

**104.** The pharmaceutical composition according to claim 93, wherein the antibody in the antibody-drug conjugate is an anti-TROP2 antibody.

**105.** The pharmaceutical composition according to claim 104, wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

**106.** The pharmaceutical composition according to claim 105, wherein the anti-TROP2 antibody lacks a lysine residue at the carboxyl terminus of the heavy chain.

**107.** The pharmaceutical composition according to any one of claims 104 to 106, wherein the average number of units of the drug-linker conjugated per antibody molecule in the antibody-drug conjugate is in the range of from 3.5 to 4.5.

**108.** The pharmaceutical composition according to claim 93, wherein the antibody in the antibody-drug conjugate is an anti-B7-H3 antibody.

**109.** The pharmaceutical composition according to claim 108, wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**110.** The pharmaceutical composition according to claim 109, wherein the anti-B7-H3 antibody lacks a lysine residue at the carboxyl terminus of the heavy chain.

**111.** The pharmaceutical composition according to any one of claims 108 to 110, wherein the average number of units of the drug-linker conjugated per antibody molecule in the antibody-drug conjugate is in the range of from 3.5 to 4.5.

**112.** The pharmaceutical composition according to claim 93, wherein the antibody in the antibody-drug conjugate is an anti-CDH6 antibody.

**113.** The pharmaceutical composition according to claim 112, wherein the anti-CDH6 antibody is an antibody comprising a heavy chain consisting of an amino acid sequence consisting of amino acid residues 20 to 471 of SEQ ID NO: 9 and a light chain consisting of an amino acid sequence consisting of amino acid residues 21 to 233 of SEQ ID NO: 10.

**114.** The pharmaceutical composition according to claim 113, wherein the anti-CDH6 antibody lacks a lysine residue at the carboxyl terminus of the heavy chain.

**115.** The pharmaceutical composition according to any one of claims 112 to 114, wherein the average number of units of the drug-linker conjugated per antibody molecule in the antibody-drug conjugate is in the range of from 7 to 8.

**116.** The pharmaceutical composition according to any one of claims 63 to 115, wherein the antibody-drug conjugate and the kinase inhibitor are separately contained as active components in different formulations, and are administered simultaneously or at different times.

**117.** The pharmaceutical composition according to any one of claims 63 to 116, wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, uterine carcinosarcoma, urothelial cancer, prostate cancer, bladder cancer, gastrointestinal stromal tumor, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, osteosarcoma, sarcoma, and melanoma.

**118.** The pharmaceutical composition according to claim 117, wherein the pharmaceutical composition is for use in treating breast cancer.

**119.** The pharmaceutical composition according to claim 117, wherein the pharmaceutical composition is for use in treating colorectal cancer.

**120.** The pharmaceutical composition according to claim 117, wherein the pharmaceutical composition is for use in treating gastric cancer.

**121.** The pharmaceutical composition according to claim 117, wherein the pharmaceutical composition is for use in treating lung cancer.

**122.** The pharmaceutical composition according to claim 117, wherein the pharmaceutical composition is for use in treating pancreatic cancer.

**123.** The pharmaceutical composition according to claim 117, wherein the pharmaceutical composition is for use in treating kidney cancer.

**124.** The pharmaceutical composition according to claim 117, wherein the pharmaceutical composition is for use in treating ovarian cancer.

[Figure 1]

SEQ ID NO: 1 - Amino acid sequence of a heavy chain of the anti-HER2 antibody

EVQLVESGGGLVQPGGSLRLSCAASGFNIKDTYIHWVR
QAPGKGLEWVARIYPTNGYTRYADSVKGRFTISADTSK
NTAYLQMNSLRAEDTAVYYCSRWGGDGFYAMDYWGQGT
LVTVSSASTKGPSVFPLAPSSKSTSGGTAALGCLVKDY
FPEPVTVSWNSGALTSGVHTFPAVLQSSGLYSLSSVVT
VPSSSLGTQTYICNVNHKPSNTKVDKKVEPKSCDKTHT
CPPCPAPELLGGPSVFLFPPKPKDTLMISRTPEVTCVV
VDVSHEDPEVKFNWYVDGVEVHNAKTKPREEQYNSTYR
VVSVLTVLHQDWLNGKEYKCKVSNKALPAPIEKTISKA
KGQPREPQVYTLPPSREEMTKNQVSLTCLVKGFYPSDI
AVEWESNGQPENNYKTTPPVLDSDGSFFLYSKLTVDKS
RWQQGNVFSCSVMHEALHNHYTQKSLSLSPGK

[Figure 2]

SEQ ID NO: 2 - Amino acid sequence of a light chain of the anti-HER2 antibody

DIQMTQSPSSLSASVGDRVTITCRASQDVNTAVAWYQQ
KPGKAPKLLIYSASFLYSGVPSRFSGSRSGTDFTLTIS
SLQPEDFATYYCQQHYTTPPTFGQGTKVEIKRTVAAPS
VFIFPPSDEQLKSGTASVVCLLNNFYPREAKVQWKVDN
ALQSGNSQESVTEQDSKDSTYSLSSTLTLSKADYEKHK
VYACEVTHQGLSSPVTKSFNRGEC

[Figure 3]

SEQ ID NO: 3 - Amino acid sequence of a heavy chain of the anti-HER3 antibody

QVQLQQWGAGLLKPSETLSLTCAVYGGSFSGYYWSWIR
QPPGKGLEWIGEINHSGSTNYNPSLKSRVTISVETSKN
QFSLKLSSVTAADTAVYYCARDKWTWYFDLWGRGTLVT
VSSASTKGPSVFPLAPSSKSTSGGTAALGCLVKDYFPE
PVTVSWNSGALTSGVHTFPAVLQSSGLYSLSSVVTVPS
SSLGTQTYICNVNHKPSNTKVDKRVEPKSCDKTHTCPP
CPAPELLGGPSVFLFPPKPKDTLMISRTPEVTCVVVDV
SHEDPEVKFNWYVDGVEVHNAKTKPREEQYNSTYRVVS
VLTVLHQDWLNGKEYKCKVSNKALPAPIEKTISKAKGQ
PREPQVYTLPPSREEMTKNQVSLTCLVKGFYPSDIAVE
WESNGQPENNYKTTPPVLDSDGSFFLYSKLTVDKSRWQ
QGNVFSCSVMHEALHNHYTQKSLSLSPGK

[Figure 4]

SEQ ID NO: 4 - Amino acid sequence of a light chain of the anti-HER3 antibody

DIEMTQSPDSLAVSLGERATINCRSSQSVLYSSSNRNY
LAWYQQNPGQPPKLLIYWASTRESGVPDRFSGSGSGTD
FTLTISSLQAEDVAVYYCQQYYSTPRTFGQGTKVEIKR
TVAAPSVFIFPPSDEQLKSGTASVVCLLNNFYPREAKV
QWKVDNALQSGNSQESVTEQDSKDSTYSLSSTLTLSKA
DYEKHKVYACEVTHQGLSSPVTKSFNRGEC

[Figure 5]

SEQ ID NO: 5 - Amino acid sequence of a heavy chain of the anti-TROP2 antibody

MKHLWFFLLLVAAPRWVLSQVQLVQSGAEVKKPGASVK
VSCKASGYTFTTAGMQWVRQAPGQGLEWMGWINTHSGV
PKYAEDFKGRVTISADTSTSTAYLQLSSLKSEDTAVYY
CARSGFGSSYWYFDVWGQGTLVTVSSASTKGPSVFPLA
PSSKSTSGGTAALGCLVKDYFPEPVTVSWNSGALTSGV
HTFPAVLQSSGLYSLSSVVTVPSSSLGTQTYICNVNHK
PSNTKVDKRVEPKSCDKTHTCPPCPAPELLGGPSVFLF
PPKPKDTLMISRTPEVTCVVVDVSHEDPEVKFNWYVDG
VEVHNAKTKPREEQYNSTYRVVSVLTVLHQDWLNGKEY
KCKVSNKALPAPIEKTISKAKGQPREPQVYTLPPSREE
MTKNQVSLTCLVKGFYPSDIAVEWESNGQPENNYKTTP
PVLDSDGSFFLYSKLTVDKSRWQQGNVFSCSVMHEALH
NHYTQKSLSLSPGK

Signal sequence (1-19), Variable region (20-140), Constant region (141-470)

[Figure 6]

SEQ ID NO: 6 - Amino acid sequence of a light chain of the anti-TROP2 antibody

MVLQTQVFISLLLWISGAYGDIQMTQSPSSLSASVGDR
VTITCKASQDVSTAVAWYQQKPGKAPKLLIYSASYRYT
GVPSRFSGSGSGTDFTLTISSLQPEDFAVYYCQQHYIT
PLTFGQGTKLEIKRTVAAPSVFIFPPSDEQLKSGTASV
VCLLNNFYPREAKVQWKVDNALQSGNSQESVTEQDSKD
STYSLSSTLTLSKADYEKHKVYACEVTHQGLSSPVTKS
FNRGEC

Signal sequence (1-20), Variable region (21-129), Constant region (130-234)

[Figure 7]

SEQ ID NO: 7 - Amino acid sequence of a heavy chain of the anti-B7-H3 antibody

M K H L W F F L L L V A A P R W V L S Q V Q L V Q S G A E V K K P G S S V K
V S C K A S G Y T F T N Y V M H W V R Q A P G Q G L E W M G Y I N P Y N D D
V K Y N E K F K G R V T I T A D E S T S T A Y M E L S S L R S E D T A V Y Y
C A R W G Y Y G S P L Y Y F D Y W G Q G T L V T V S S A S T K G P S V F P L
A P S S K S T S G G T A A L G C L V K D Y F P E P V T V S W N S G A L T S G
V H T F P A V L Q S S G L Y S L S S V V T V P S S S L G T Q T Y I C N V N H
K P S N T K V D K R V E P K S C D K T H T C P P C P A P E L L G G P S V F L
F P P K P K D T L M I S R T P E V T C V V V D V S H E D P E V K F N W Y V D
G V E V H N A K T K P R E E Q Y N S T Y R V V S V L T V L H Q D W L N G K E
Y K C K V S N K A L P A P I E K T I S K A K G Q P R E P Q V Y T L P P S R E
E M T K N Q V S L T C L V K G F Y P S D I A V E W E S N G Q P E N N Y K T T
P P V L D S D G S F F L Y S K L T V D K S R W Q Q G N V F S C S V M H E A L
H N H Y T Q K S L S L S P G K

Signal sequence (1-19), Variable region (20-141), Constant region (142-471)

[Figure 8]

SEQ ID NO: 8 - Amino acid sequence of a light chain of the anti-B7-H3 antibody

M V L Q T Q V F I S L L L W I S G A Y G E I V L T Q S P A T L S L S P G E R
A T L S C R A S S R L I Y M H W Y Q Q K P G Q A P R P L I Y A T S N L A S G
I P A R F S G S G S G T D F T L T I S S L E P E D F A V Y Y C Q Q W N S N P
P T F G Q G T K V E I K R T V A A P S V F I F P P S D E Q L K S G T A S V V
C L L N N F Y P R E A K V Q W K V D N A L Q S G N S Q E S V T E Q D S K D S
T Y S L S S T L T L S K A D Y E K H K V Y A C E V T H Q G L S S P V T K S F
N R G E C

Signal sequence (1-20), Variable region (21-128), Constant region (129-233)

[Figure 9]

SEQ ID NO: 9 - Amino acid sequence of a heavy chain of the anti-CDH6 antibody

MKHLWFFLLLVAAPRWVLSEVQLVQSGAEVKKPGASVK
VSCKASGYTFTRNFMHWVRQAPGQGLEWMGWIYPGDGE
TEYAQKFQGRVTITADTSTSTAYMELSSLRSEDTAVYY
CARGVYGGFAGGYFDFWGQGTLVTVSSASTKGPSVFPL
APSSKSTSGGTAALGCLVKDYFPEPVTVSWNSGALTSG
VHTFPAVLQSSGLYSLSSVVTVPSSSLGTQTYICNVNH
KPSNTKVDKRVEPKSCDKTHTCPPCPAPELLGGPSVFL
FPPKPKDTLMISRTPEVTCVVVDVSHEDPEVKFNWYVD
GVEVHNAKTKPREEQYNSTYRVVSVLTVLHQDWLNGKE
YKCKVSNKALPAPIEKTISKAKGQPREPQVYTLPPSRE
EMTKNQVSLTCLVKGFYPSDIAVEWESNGQPENNYKTT
PPVLDSDGSFFLYSKLTVDKSRWQQGNVFSCSVMHEAL
HNHYTQKSLSLSPGK

Signal sequence (1-19), Variable region (20-141), Constant region (142-471)

[Figure 10]

SEQ ID NO: 10 - Amino acid sequence of a light chain of the anti-CDH6 antibody

MVLQTQVFISLLLWISGAYGDIQMTQSPSSLSASVGDR
VTITCKASQNIYKNLAWYQQKPGKAPKLLIYDANTLQT
GVPSRFSGSGSGSDFTLTISSLQPEDFATYFCQQYYSG
WAFGQGTKVEIKRTVAAPSVFIFPPSDEQLKSGTASVV
CLLNNFYPREAKVQWKVDNALQSGNSQESVTEQDSKDS
TYSLSSTLTLSKADYEKHKVYACEVTHQGLSSPVTKSF
NRGEC

Signal sequence (1-20), Variable region (21-128), Constant region (129-233)

[Figure 11]

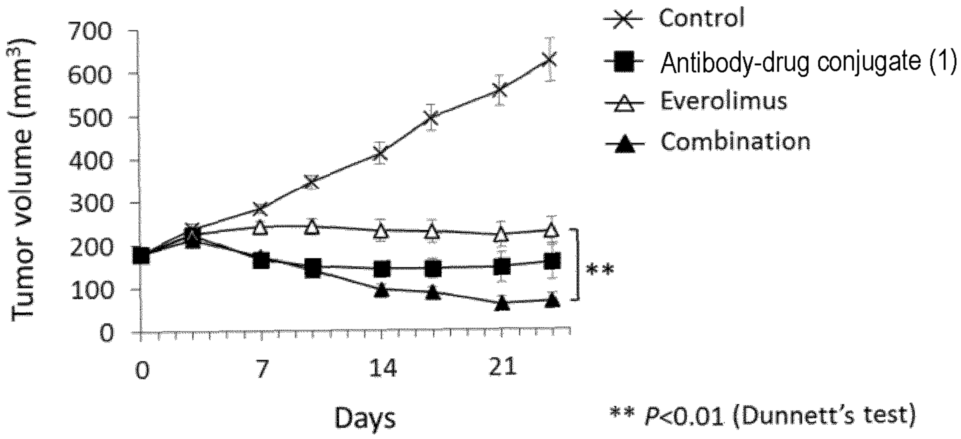


[Figure 12]

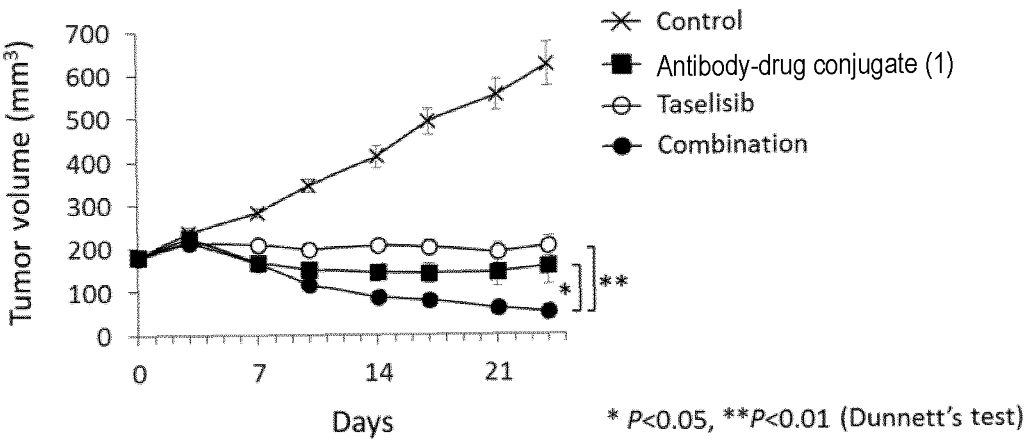


[Figure 13]

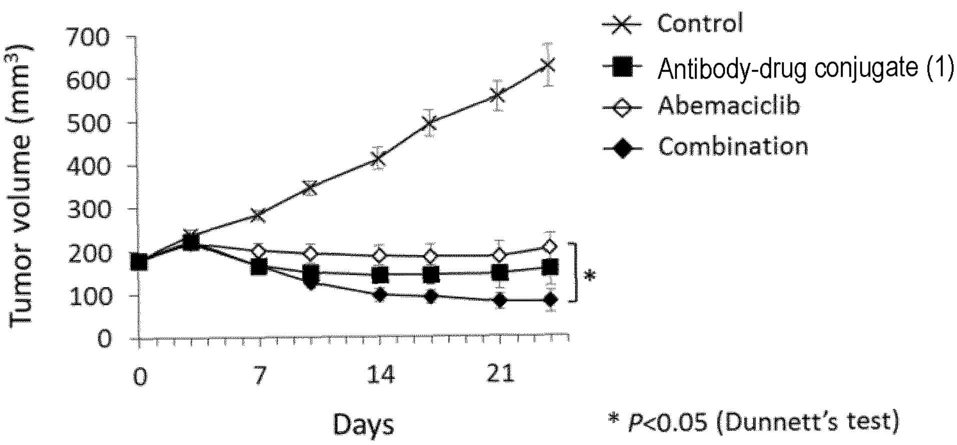

[Figure 14]

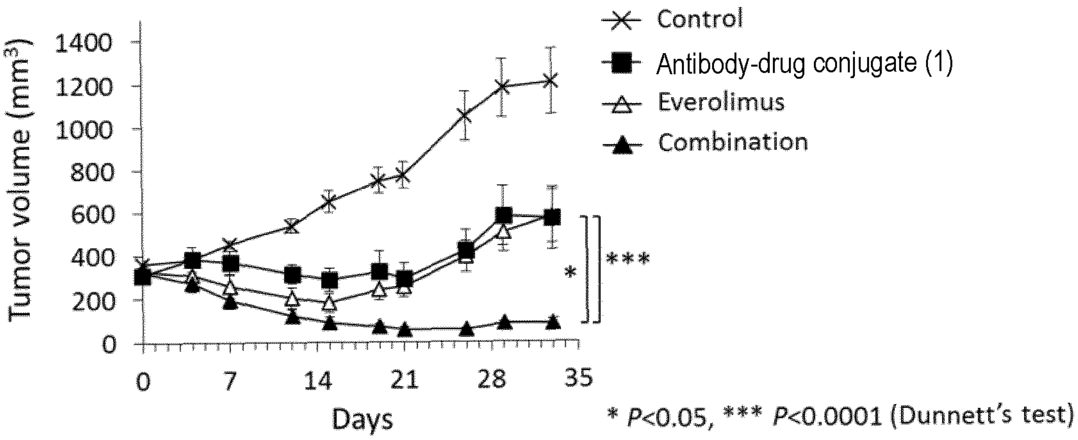


[Figure 15]

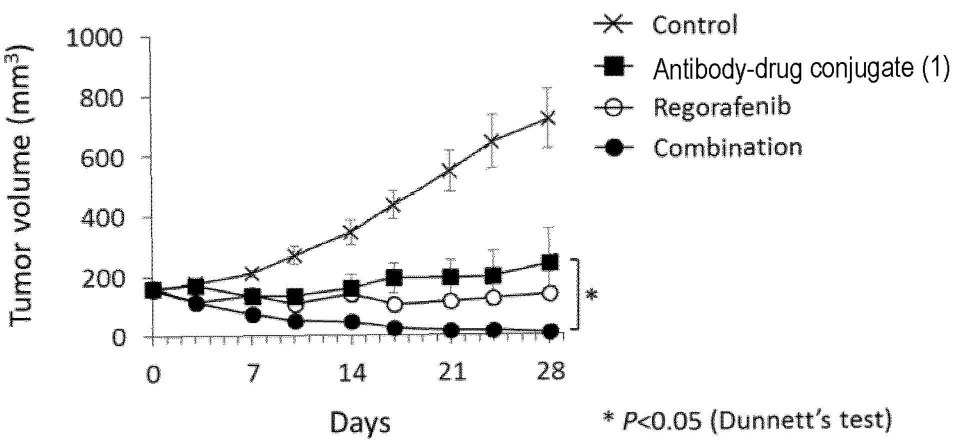


[Figure 16]

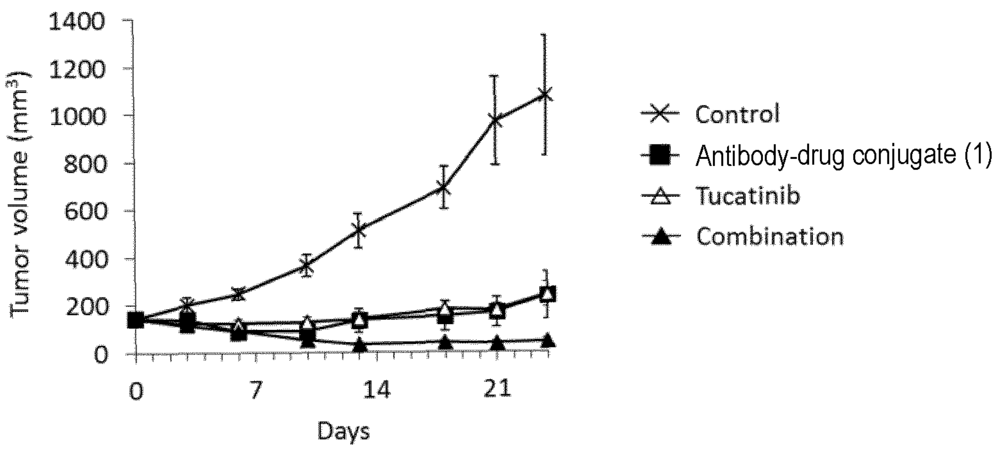

[Figure 17]

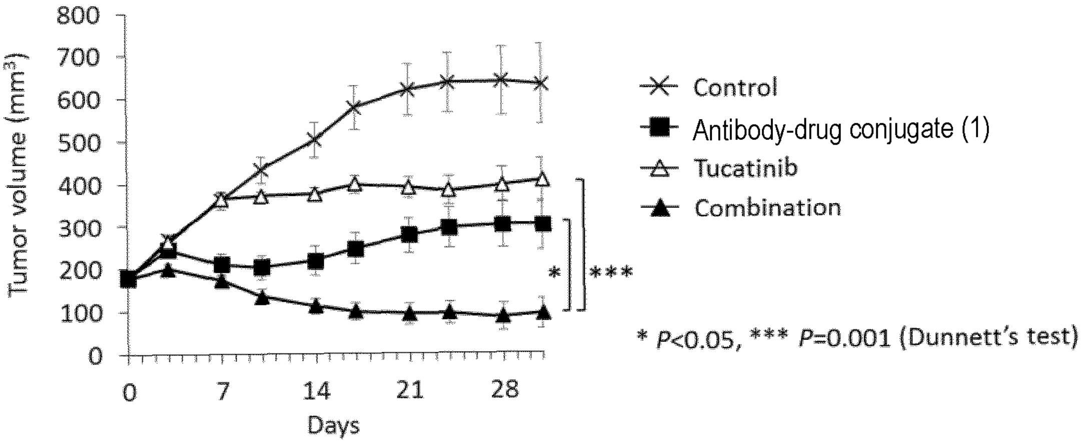


[Figure 18]

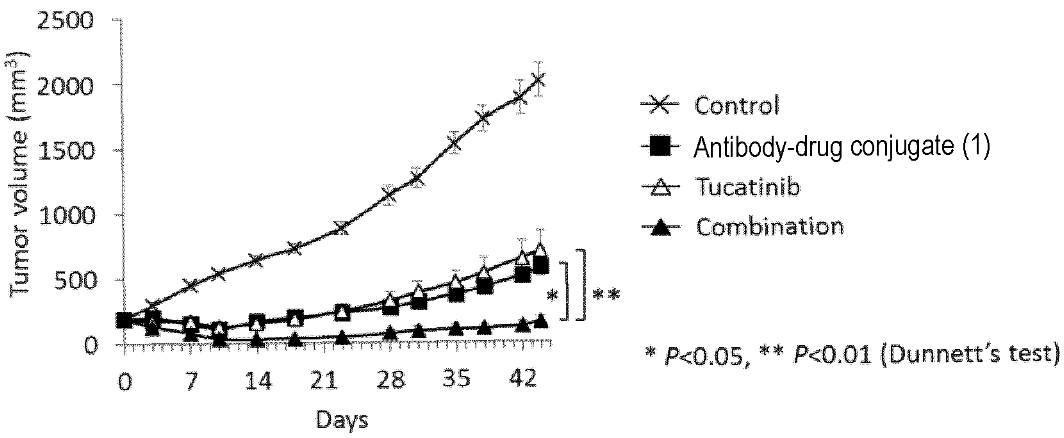


[Figure 19]

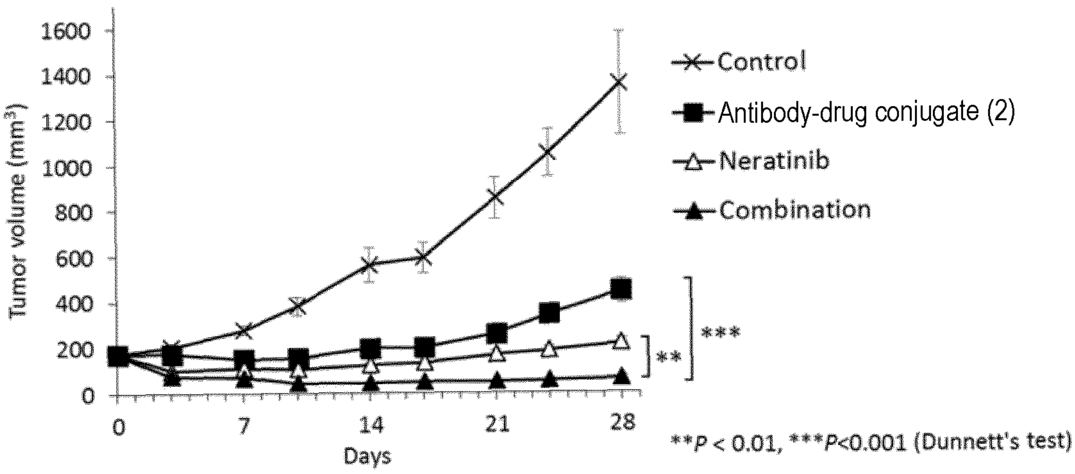

[Figure 20]

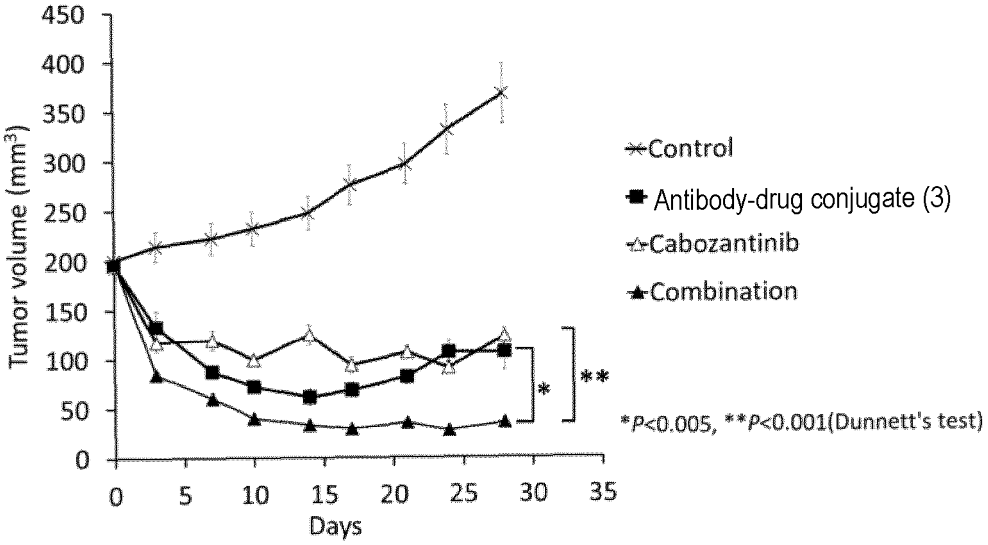


[Figure 21]

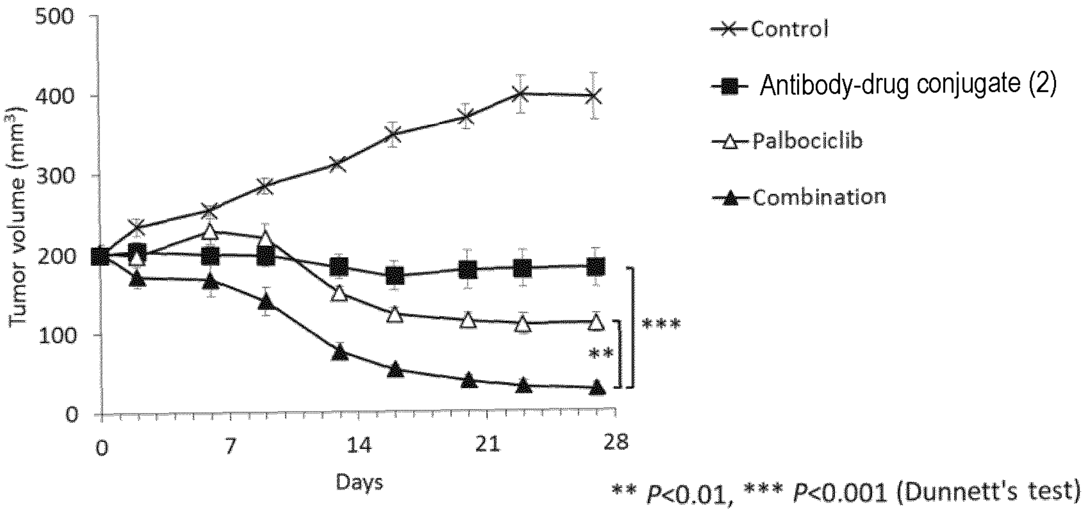

[Figure 22]

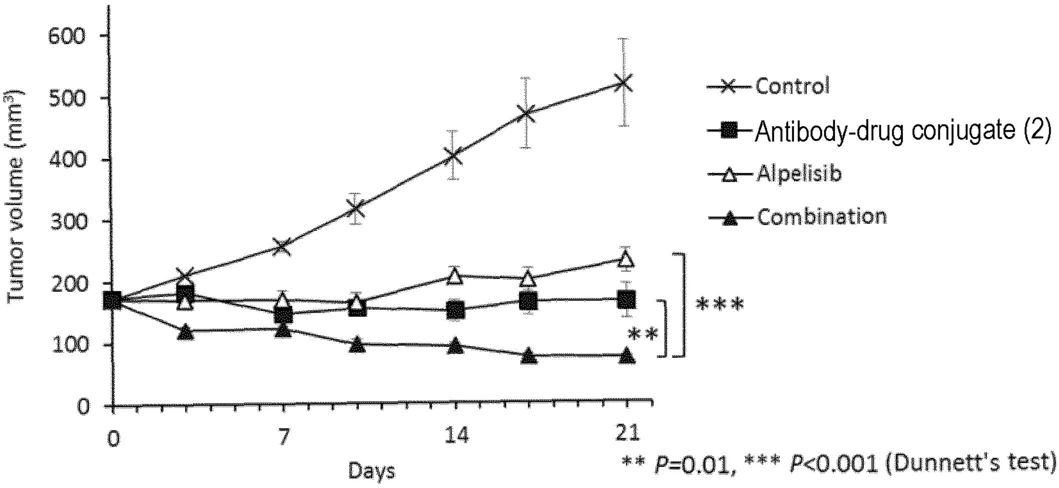
600
500
400
300
200
100
0
Tumor volume (mm³)
0
7
14
21
Days
Control
Antibody-drug conjugate (2)
Alpelisib
Combination
***
**
** P=0.01, *** P<0.001 (Dunnett's test)

## INTERNATIONAL SEARCH REPORT

International application No.
PCT/JP2019/050017

A. CLASSIFICATION OF SUBJECT MATTER
Int.Cl. See extra sheet
According to International Patent Classification (IPC) or to both national classification and IPC

B. FIELDS SEARCHED
Minimum documentation searched (classification system followed by classification symbols)
Int.Cl. A61K47/68; A61K31/422; A61K31/436; A6'1K31/44; A61K31/4439; A61K31/47; A61K31/4709; A61K31/4745; A61K31/506; A61K31/517; A61K31/519; A61K31/553; A61K39/395; A61K45/00; A61P35/00; A61P35/02; A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN); UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X<br><br><br><br><br>Y | JP 2017-503784 A (DAIICHI SANKYO COMPANY, LIMITED) 02.02.2017 (2017-02-02) claims, examples, paragraphs [0190]-[198] | 1-2, 27-28, 31, 38-41, 54-64, 89-90, 93, 100-103, 116-124<br>1-124 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 March 2020 (10.03.2020) | 17 March 2020 (17.03.2020) |
| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |

Form PCT/ISA/210 (second sheet) (January 2015)

INTERNATIONAL SEARCH REPORT

International application No. PCT/JP2019/050017

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2018/159582 A1 (KINDAI UNIVERSITY) 07.09.2018 (2018-09-07) claims, examples | 1-2, 27-28, 31, 38-41, 54-64, 89-90, 93, 100-103, 116-124<br>1-124 |
| Y | WO 2015/115091 A1 (DAIICHI SANKYO COMPANY, LIMITED) 06.08.2015 (2015-08-06) claims, examples, paragraph [0165] | 1-124 |
| Y | WO 2015/098099 A1 (DAIICHI SANKYO COMPANY, LIMITED) 02.07.2015 (2015-07-02) claims, examples, paragraph [0160] | 1-124 |
| Y | WO 2018/212136 A1 (DAIICHI SANKYO COMPANY, LIMITED) 22.11.2018 (2018-11-22) claims, examples, paragraph [0217] | 1-124 |
| Y | WO 2014/057687 A1 (DAIICHI SANKYO COMPANY, LIMITED) 17.04.2014 (2014-04-17) claims, examples, paragraph [0214] | 1-124 |
| Y | ZHANG, J. et al., "The CDK4/6 inhibitor palbociclib synergizes with irinotecan to promote colorectal cancer cell death under hypoxia.", Cell Cycle, 2017, 16(12), pp. 1193-1200, title, abstract, fig. 4 | 1-124 |
| Y | JP 2008-524225 A (NOVARTIS AG.) 10.07.2008 (2008-07-10) claims, examples | 1-124 |
| Y | YOSHIOKA, S. et al., "The PI3K-Akt pathway in SN-38-induced apoptosis in human gastric cancer cell lines.", Yonago Acta Medica, 2005, 48, pp. 7-16, abstract, page 11, paragraph [0001] to page 12, left column, paragraph [0002] | 1-124 |
| Y | SAMALIN, E. et al., "Sorafenib and irinotecan (NEXIRI) as second-or later-line treatment for patients with metastatic colorectal cancer and KRAS-mutated tumours:a multicentre Phase I/II trial.", British Journal of Cancer, 110, 2014, pp. 1148-1154, abstract, page 1149, left column, paragraph [0003] | 1-124 |
| Y | CANU, B. et al., "Irinotecan synergistically enhances the antiproliferative and proapoptotic effects of axitinib in vitro and improves its anticancer activity in vivo.", Neoplasia, 2011, 13(3), pp. 217-229, abstract | 1-124 |

Form PCT/ISA/210 (continuation of second sheet) (January 2015)

INTERNATIONAL SEARCH REPORT

International application No. PCT/JP2019/050017

C (Continuation). DOCUMENTS CONSIDERED TO BE RELEVANT

| Category* | Citation of document, with indication, where appropriate, of the relevant passages | Relevant to claim No. |
|---|---|---|
| Y | KIM, JL. et al., "Imatinib-induced apoptosis of gastric cancer cells is mediated by endoplasmic reticulum stress.", Oncology Reports, 19 December 2018, 2019, 41, pp. 1616-1626, doi:10.3892/or.2018.6945, abstract | 1-124 |
| Y | YASHIRO, M. et al., "An EGFR inhibitor enhances the efficacy of SN38, an active matabolite of irinotecan, in SN38-ref_ractory gastric carcinoma cells.", British Journal of Cancer, 2011, 105, pp. 1522-1532, abstract | 1-124 |
| Y | PELEG, R. et al., "Modification of topo1somerases in mammospheres derived from breast cancer cell line: clinical implications for combined treatments with tyrosine kinase inhibitors.", BMC Cancer, 2014, 14, 910, pp. 1-17, abstract | 1-124 |

Form PCT/ISA/210 (continuation of second sheet) (January 2015)

**INTERNATIONAL SEARCH REPORT**
Information on patent family members

International application No.
PCT/JP2019/050017

| Patent Documents referred in the Report | Publication Date | Patent Family | Publication Date |
|---|---|---|---|
| JP 2017-503784 A | 02 Feb. 2017 | US 2017/0021031 A1<br>claims, examples,<br>paragraphs [0443]-[0474]<br>US 2019/0151328 A1<br>WO 2015/155998 A1<br>EP 3129063 A1<br>CN 106163559 A<br>KR 10-2016-0144396 A<br>JP 2017-197515 A<br>JP 2017-222638 A<br>JP 2019-135248 A | |
| WO 2018/159582 A1 | 07 Sep. 2018 | CN 110475569 A<br>claims, examples<br>KR 10-2019-0120764 A | |
| WO 2015/115091 A1 | 06 Aug. 2015 | US 2016/0333112 A1<br>claims, examples,<br>paragraph [0384]<br>US 2019/0077880 A1<br>EP 3101032 A1<br>EP 3466976 A1<br>CN 105829346 A<br>KR 10-2016-0113099 A<br>KR 10-2018-0122038 A<br>CN 110464847 A<br>JP 2017-36285 A<br>JP 2017-95457 A<br>JP 2017-105789 A<br>JP 2019-112403 A | |
| WO 2015/098099 A1 | 02 Jul. 2015 | US 2016/0297890 A1<br>claims, examples,<br>paragraph [0387]<br>US 2018/0094073 A1<br>US 2019/0144559 A1<br>EP 3088419 A1<br>EP 3424955 A1<br>CN 105849126 A<br>KR 10-2019-0006087 A<br>JP 2017-197523 A<br>JP 2019-69951 A | |
| WO 2018/212136 A1 | 22 Nov. 2018 | (Family: none) | |

Form PCT/ISA/210 (patent family annex) (January 2015)

**INTERNATIONAL SEARCH REPORT**
Information on patent family members

International application No.
PCT/JP2019/050017

| Patent Documents referred in the Report | Publication Date | Patent Family | Publication Date |
|---|---|---|---|
| WO 2014/057687 A1 | 17 Apr. 2014 | US 2015/0297748 A1<br>claims, examples, paragraph [0450]<br>US 2016/0279259 A1<br>US 2019/0008981 A1<br>EP 2907824 A1<br>EP 3342785 A1<br>KR 10-2015-0067149 A<br>CN 104755494 A<br>KR 10-2018-0030734 A<br>CN 109081871 A<br>JP 2017-36274 A<br>JP 2016-196484 A<br>JP 2018-8982 A<br>JP 2018-188455 A | |
| JP 2008-524225 A | 10 Jul. 2008 | US 2010/0028338 A1<br>claims, examples<br>WO 2006/065780 A2<br>EP 1827437 A2<br>CN 101080227 A<br>KR 10-2007-0091286 A | |

Form PCT/ISA/210 (patent family annex) (January 2015)

**INTERNATIONAL SEARCH REPORT**

International application No.
PCT/JP2019/050017

<page 2, A. CLASSIFICATION OF SUBJECT MATTER>

A61K 47/68(2017.01)i; A61K 31/422(2006.01)i; A61K 31/436(2006.01)i; A61K 31/44(2006.01)I; A61K 31/4439(2006.01)i; A61K 31/47(2006.01)i; A61K 31/4709(2006.01)i; A61K 31/4745(2006.01)i; A61K 31/506(2006.01)i; A61K 31/517(2006.01)i; A61K 31/519(2006.01)i; A61K 31/553(2006.01)i; A61K 39/395(2006.01)i; A61K 45/00(2006.01)i; A61P 35/00(2006.01)i; A61P 35/02(2006.01)i; A61P 43/00(2006.01)i

FI: A61K47/68 ZNA; A61K31/422; A61K31/436; A61K31/44; A61K31/4439; A61K31/47; A61K31/4709; A61K3i/4745; A61K31/506; A61K31/517; A61K31/519; A61K31/553; A61K39/395 C; A61K39/395 E; A61K39/395 L; A61K39/395 T; A61K45/00; A61P35/00; A61P35/02; A61P43/00 111; A61P43/00 121

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014057687 A **[0007] [0049] [0053] [0058] [0074] [0224]**
- WO 2014061277 A **[0007]**
- WO 2015098099 A **[0007] [0047] [0053] [0058] [0070] [0223]**
- WO 2015115091 A **[0007] [0053] [0058] [0062] [0199] [0224]**
- WO 2015146132 A **[0007]**
- WO 2015155976 A **[0007]**
- WO 2015155998 A **[0007] [0053] [0058] [0066] [0217]**
- WO 2018212136 A **[0007] [0051] [0058] [0078] [0220]**
- WO 9007861 A **[0035]**
- US 5821337 A **[0035] [0043]**
- WO 9954342 A **[0038]**
- WO 0061739 A **[0038]**
- WO 0231140 A **[0038]**
- WO 2007133855 A **[0038]**
- WO 2013120066 A **[0038]**
- WO 0100245 A **[0043]**
- WO 2007077028 A **[0045]**
- WO 2008100624 A **[0045]**
- WO 2019044947 A **[0053]**
- WO 2017002776 A **[0058] [0070] [0074] [0223]**
- US 7855211 B **[0082]**
- US 6936612 B **[0082]**
- US 9193732 B **[0082]**
- WO 2012061156 A **[0082]**
- WO 2018033815 A **[0082]**
- WO 2016187316 A **[0082]**
- WO 1989007105 A **[0082]**
- US 5665772 A **[0084]**
- US 5989591 A **[0084]**
- US 5362718 A **[0084]**
- WO 2008070740 A **[0084]**
- WO 2009143313 A **[0084]**
- WO 2014140073 A **[0086]**
- WO 2010029082 A **[0086]**
- US 9469643 B **[0086]**
- US 8193182 B **[0086]**
- WO 2007084786 A **[0086]**
- WO 2007129161 A **[0086]**
- WO 2007044729 A **[0086]**
- US 7511041 B **[0086]**
- WO 2003024183 A **[0086]**
- CH 5132799 **[0086]**
- WO 2017001645 A **[0086]**
- WO 2006010152 A **[0086]**
- WO 2008006040 A **[0089]**
- WO 2008098104 A **[0089]**
- WO 2014124230 A **[0091]**
- WO 2016106029 A **[0091]**
- US 7378423 B **[0092]**
- US 7777050 B **[0092]**
- WO 2005023251 A **[0092]**
- WO 2007014011 A **[0092]**
- WO 2006045514 A **[0092]**
- US 7803839 B **[0092]**
- US 7351834 B **[0094]**
- US 7235576 B **[0094]**
- US 7504509 B **[0094]**
- US 7994185 B **[0094]**
- US 8501758 B **[0094]**
- WO 2013100632 A **[0094]**
- WO 2005077954 A **[0097]**
- WO 1997020842 A **[0097]**
- WO 2001053293 A **[0097]**
- WO 2005037800 A **[0097]**
- WO 2007148158 A **[0097]**
- WO 2012049153 A **[0102]**
- WO 2005111039 A **[0103]**
- WO 2010065825 A **[0103]**
- US 6894051 B **[0105]**
- US 6596746 B **[0105]**
- US 6002008 A **[0105]**
- US 7169791 B **[0105]**
- US 8114874 B **[0105]**
- US 7173038 B **[0110]**
- US 6573293 B **[0110]**
- US 6534524 B **[0110]**
- US 7105530 B **[0110]**
- US 7253286 B **[0110]**
- US 6762180 B **[0110]**
- US 7579473 B **[0110]**
- WO 2002088110 A **[0110]**
- WO 2004009784 A **[0110]**
- WO 2014022975 A **[0110]**
- WO 2008112408 A **[0110]**
- US 7514444 B **[0117] [0118]**
- US 9290504 B **[0117]**
- WO 2011152351 A **[0117]**
- WO 2003037347 A **[0118]**
- US 9012462 B **[0120]**
- US 7858643 B **[0120]**
- US 7153964 B **[0120]**
- US 9126931 B **[0120]**
- US 8680111 B **[0120]**

- US 7598257 B **[0122]**
- US 7265221 B **[0122]**
- US 8158616 B **[0122]**
- WO 2007058627 A **[0122]**
- WO 2008064157 A **[0124]**
- WO 2009006959 A **[0124]**
- WO 2007121484 A **[0125]**
- WO 2009052237 A **[0125]**
- WO 2009013126 A **[0127]**
- WO 2015089139 A **[0127]**
- WO 1996033980 A **[0128]**
- WO 1996030347 A **[0128]**
- WO 2002050043 A **[0128]**
- WO 2013014448 A **[0128]**
- US 7772243 B **[0128]**
- US 713485 A **[0128]**
- WO 2011029265 A **[0128]**
- WO 2014116070 A **[0128]**
- WO 2013056183 A **[0129]**
- WO 2001077107 A **[0129]**
- JP 2018240049 A **[0250]**
- JP 2019085338 A **[0250]**


**Non-patent literature cited in the description**


- **OTTO T. et al.** *Nat. Rev. Cancer,* 2017, vol. 17 (2), 93-115 **[0008]**
- **ZHANG YJ. et al.** *Drug Discov. Today,* 2011, vol. 16 (7-8), 325-331 **[0008]**
- **ZAYTSEVA YY. et al.** *Cancer Lett,* 2012, vol. 319 (1), 1-7 **[0008]**
- **JANKU F. et al.** *Nat. Rev. Clin. Oncol.,* 2018, vol. 15 (5), 273-291 **[0008]**
- **BERGHOLZ JS. et al.** *J. Clin. Oncol.,* 2018, vol. 36 (13), 1339-1342 **[0008]**
- **ZHAO Y. et al.** *Nat. Rev. Clin. Oncol.,* 2014, vol. 11 (7), 385-400 **[0008]**
- **CAUNT CJ. et al.** *Nat. Rev. Cancer,* 2015, vol. 15 (10), 577-592 **[0008]**
- **RYAN MB. et al.** *Nat. Rev. Clin. Oncol.,* 2018, vol. 15 (11), 709-720 **[0008]**
- **FERQUSON FM. et al.** *Nat. Rev. Drug Discov.,* 2018, vol. 17 (5), 353-377 **[0008]**
- **KONIG H. et al.** *Current Cancer Drug Targets,* 2015, vol. 15, 803-821 **[0008]**
- **KHEDER ES. et al.** *Clinical Cancer Research,* 2018, vol. 24 (23), 5807-5814 **[0008]**
- **DUCRY, L. et al.** *Bioconjugate Chem,* 2010, vol. 21, 5-13 **[0008]**
- **ALLEY, S. C. et al.** *Current Opinion in Chemical Biology,* 2010, vol. 14, 529-537 **[0008]**
- **DAMLE N. K.** *Expert Opin. Biol. Ther.,* 2004, vol. 4, 1445-1452 **[0008]**
- **SENTER P. D. et al.** *Nature Biotechnology,* 2012, vol. 30, 631-637 **[0008]**
- **BURRIS HA. et al.** *J. Clin. Oncol.,* 2011, vol. 29 (4), 398-405 **[0008]**
- **OGITANI Y. et al.** *Clinical Cancer Research,* 2016, vol. 22 (20), 5097-5108 **[0008]**
- **OGITANI Y. et al.** *Cancer Science,* 2016, vol. 107, 1039-1046 **[0008] [0022]**
- **DOI T et al.** *Lancet Oncol,* 2017, vol. 18, 1512-22 **[0008]**
- **TAKEGAWA N et al.** *Int. J. Cancer,* 2017, vol. 141, 1682-1689 **[0008]**
- **YONESAKA K et al.** *Int. Oncogene,* 2017, vol. 141, 1682-1689 **[0008]**
- **OGITANI Y. et al.** *Clinical Cancer Research,* 29 March 2016, vol. 22 (20), 5097-5108 **[0021]**
- *Cell Death and Differentiation,* 2008, vol. 15, 751-761 **[0027]**
- *Molecular Biology of the Cell,* December 2004, vol. 15, 5268-5282 **[0027]**
- *Bio Techniques,* January 2000, vol. 28, 162-165 **[0027]**
- **KOHLER ; MILSTEIN.** *Nature,* 1975, vol. 256, 495-497 **[0031]**
- Monoclonal Antibodies. Plenum Press, 1980, 365-367 **[0031]**
- *Proc. Natl. Acad. Sci. USA,* 1984, vol. 81, 6851-6855 **[0034]**
- *Nature,* 1986, vol. 321, 522-525 **[0035]**
- **TOMIZUKA, K. et al.** *Nature Genetics,* 1997, vol. 16, 133-143 **[0036]**
- **KUROIWA, Y.** *Nucl. Acids Res.,* 1998, vol. 26, 3447-3448 **[0036]**
- **YOSHIDA, H.** Animal Cell Technology:Basic and Applied Aspects. Kluwer Academic Publishers, 1999, vol. 10, 69-73 **[0036]**
- **TOMIZUKA, K.** *Proc. Natl. Acad. Sci. USA,* 2000, vol. 97, 722-727 **[0036]**
- **WORMSTONE, I. M.** *Investigative Ophthalmology & Visual Science,* 2002, vol. 43 (7), 2301-2308 **[0036]**
- **CARMEN, S.** *Briefings in Functional Genomics and Proteomics,* 2002, vol. 1 (2), 189-203 **[0036]**
- **SIRIWARDENA, D.** *Ophthalmology,* 2002, vol. 109 (3), 427-431 **[0036]**
- *Journal of Chromatography A,* 1995, vol. 705, 129-134 **[0039]**
- *Analytical Biochemistry,* 2007, vol. 360, 75-83 **[0039]**
- **HERMANSON, G. T.** Bioconjugate Techniques. Academic Press, 1996, 56-136, 456-493 **[0055]**
- *J Med Chem,* 2018, vol. 61 (4), 1499-1518 **[0082]**
- *J Med Chem,* 2015, vol. 58 (13), 5323-5333 **[0084]**
- *Cancer Res,* 2010, vol. 70 (1), 288-98 **[0084]**
- *Invest New Drugs,* 2015, vol. 33 (2), 463-71 **[0084]**
- *Mol Cancer Ther,* 2016, vol. 15 (10), 2344-2356 **[0084]**
- *Mol Cancer Ther,* 2011, vol. 10 (11), 2189-99 **[0084]**
- *Ann Oncol,* 2012, vol. 23 (9), 2399-408 **[0084]**

- *J Med Chem,* 2017, vol. 60 (17), 7524-7538 **[0084]**
- *Org Process Res Dev,* 2017, vol. 21 (4), 669-673 **[0086]**
- *Clin Cancer Res,* 2017, vol. 23 (19), 5981-92 **[0086]**
- *J Med Chem,* 2015, vol. 58 (2), 943-962 **[0086]**
- *ACS Med Chem Lett,* 2016, vol. 7 (9), 862-867 **[0086]**
- *J Med Chem,* 2015, vol. 58 (1), 480-511 **[0086]**
- *Bioorg Med Chem Lett,* 2011, vol. 21 (6), 1767-1772 **[0086]**
- *J Natl Cancer Inst,* 2006, vol. 98 (8), 545-56 **[0086]**
- *Mol Cancer Ther,* 2010, vol. 9 (7), 1956-67 **[0089]**
- *Int J Cancer,* 2017, vol. 140 (2), 449-454 **[0089]**
- *Mol Cancer Ther,* 2012, vol. 11 (4), 873-87 **[0089]**
- *Cancer Res,* 2014, vol. 74 (16), 4458-69 **[0089]**
- *Oncotarget,* 2014, vol. 5 (24), 12728-37 **[0091]**
- *Mol Cancer Ther,* 2017, vol. 16 (11), 2351-2363 **[0091]**
- *J Med Chem,* 2016, vol. 59 (12), 5650-5660 **[0091]**
- *ACS Med Chem Lett,* 2018, vol. 9 (7), 761-767 **[0091]**
- *J Pharmacol Exp Ther,* 2009, vol. 331 (2), 485-95 **[0092]**
- *Clin Cancer Res,* 2010, vol. 16 (8), 2450-7 **[0092]**
- *ACS Med Chem Lett,* 2014, vol. 5 (4), 309-14 **[0092]**
- *Bioorg Med Chem Lett,* 2014, vol. 24 (19), 4714-4723 **[0092]**
- *ACS Med Chem Lett,* 2015, vol. 6 (9), 961-5 **[0094]**
- *J Med Chem,* 2015, vol. 58 (10), 4165-4179 **[0094]**
- *J Med Chem,* 2009, vol. 52 (16), 5152-63 **[0097]**
- *J Med Chem,* 2008, vol. 51 (16), 4986-4999 **[0097]**
- *Bioorg Chem,* 2010, vol. 18 (5), 1844-53 **[0097]**
- *Bioorg Med Chem Lett,* 2011, vol. 21 (1), 471-4 **[0100]**
- *Mol Cancer Ther,* 2015, vol. 14 (9), 2004-13 **[0100]**
- *Invest New Drugs,* 2013, vol. 31 (1), 136-44 **[0100]**
- *Mol Cancer Ther,* 2007, vol. 6 (1), 147-53 **[0100]**
- *Org Process Res Dev,* 2015, vol. 19 (6), 661-672 **[0100]**
- *Oncotarget,* 2016, vol. 7 (3), 2329-42 **[0100]**
- *Mol Cancer Ther,* 2009, vol. 8 (11), 2992-3000 **[0101]**
- *Clin Cancer Res,* 2011, vol. 17 (4), 849-60 **[0103]**
- *Cancer Res,* 2010, vol. 70 (23), 9846-54 **[0103]**
- *J Med Chem,* 2009, vol. 52 (23), 7808-7816 **[0118]**
- *Clin Cancer Res,* 2014, vol. 20 (12), 3146-58 **[0125]**
- *J Med Chem,* 2011, vol. 54 (22), 7860-7883 **[0125]**
- *J Med Chem,* 2005, vol. 48 (4), 1107-1131 **[0128]**
- *British Journal of Cancer,* 1999, vol. 79 (5-6), 707-717 **[0211] [0218] [0231]**
- **GRECO WR et al.** *Pharmacol. Rev.,* June 1995, vol. 47 (2), 331-85 **[0229]**
- **LEHAR J et al.** *Nat Biotechnol,* July 2009, vol. 27 (7), 659-66 **[0229]**